# EUROPEAN PATENT SPECIFICATION

(11) **EP 0 717 747 B1**
(45) Date of publication and mention of the grant of the patent: **04.06.2003**
(21) Application number: 94929183.5
(22) Date of filing: 09.09.1994
(51) Int. Cl.: C12N 15/57, C12N 9/52, A61K 38/48, C07K 1/22

(54) **PORPHYROMONAS GINGIVALIS ARGININE-SPECIFIC PROTEINASE CODING SEQUENCES**
PORPHYROMONAS GINGIVALIS ARGININ-SPEZIFISCHE PROTEINASE KODIERENDE SEQUENZEN
SEQUENCES DE CODAGE D'UNE PROTEINASE SPECIFIQUE DE L'ARGININE DE LA PORPHYROMONAS GINGIVALIS

(30) Priority: 10.09.1993 US 119361; 21.10.1993 US 141324; 24.06.1994 US 265441
(43) Date of publication of application: 26.06.1996
(73) Proprietor: THE UNIVERSITY OF GEORGIA RESEARCH FOUNDATION, INC., Athens, Georgia 30602 (US)
(72) Inventor: TRAVIS, James, Athens, GA 30605 (US); POTEMPA, Jan Stanislaw, Apartment 10A, Athens, GA 30605 (US); BARR, Philip, J., Berkeley, CA 94705 (US); PAVLOFF, Nadine, Novato, CA 94947 (US)
(74) Representative: Silveston, Judith
(86) International application number: US9410283
(87) International publication number: WO95007286

(56) References cited:
- JOURNAL OF BIOLOGICAL CHEMISTRY, Vol. 267, No. 26, issued 15 September 1992, CHEN et al., "Purification and Characterization of a 50-kD Cysteine Proteinase (Gingipain) from Porphyromonas gingivalis", pages 18896-18901.
- FASEB JOURNAL ABSTRACT, Vol. 5, No. 4, issued 1991, POTEMPA et al., "Purification and Characterization of a 50 kDa Cysteine Proteinase of Porphyromonas gingivalis", page A829, abstract no. 2667.
- SCIENCE, Vol. 239, issued 11 March 1988, LEE et al., "Generation of cDNA Probes Directed by Amino Acid Sequence: Cloning of Urate Oxidase", pages 1288-1291.

## Description

This invention was made, at least in part, with funding from the National Institutes of Health (Grant Nos. DE 09761, HL 26148 and HL 37090). Accordingly, the United States Government may have certain rights in this invention.

### FIELD OF THE INVENTION

The field of this invention is bacterial proteases, more particularly those of Porphyromonas gingivalis, most particularly the arginine-specific protease termed Arg-gingipain herein and the nucleotide sequences encoding same.

### BACKGROUND OF THE INVENTION

Porphyromonas gingivalis (formerly Bacteroides gingivalis) is an obligately anaerobic bacterium which is implicated in periodontal disease. P. gingivalis produces proteolytic enzymes in relatively large quantities; these proteinases are recognized as important virulence factors. A number of physiologically significant proteins, including collagen, fibronectin, immunoglobulins, complement factors C3, C4, C5, and B, lysozyme, iron-binding proteins, plasma proteinase inhibitors, fibrin and fibrinogen, and key factors of the plasma coagulation cascade system, are hydrolyzed by proteinases from this microorganism. Such broad proteolytic activity may play a major role in the evasion of host defense mechanisms and the destruction of gingival connective tissue associated with progressive periodontitis (Saglie et al. (1988) *J. Periodontal.* 59, 259-265).

There are conflicting data as to the number and types of proteinases produced by P. gingivalis. In the past, proteolytic activities of P. gingivalis were classified into two groups; those enzymes which specifically degraded collagen and the general "trypsin-like" proteinases which appeared to be responsible for other proteolytic activity. Trypsin (and trypsin-like proteases) cleaves after arginine or lysine in the substrates (See, e.g. Lehninger A. L. (1982), Principles of Biochemistry. Worth Publishing, Inc., New York). Although many attempts have been made to separate one of these trypsin-like proteinases, Chen et al. (1992) *J. Biol. Chem.* 267, 18896-18901 reported the first rigorous purification and biochemical and enzymological characterization for an Arginine-specific P. gingivalis protease.

This application reports the purification of 50 kDa and high molecular weight trypsin-like, thiol-activated proteinases of P. gingivalis and nucleotide sequences encoding same.

### SUMMARY OF THE INVENTION

An object of the present invention is to provide a nucleotide sequence encoding a low molecular weight Arg-gingipain, termed Arg-gingipain-1 (or gingipain-1), herein, said gingipain-1 having an apparent molecular mass of 50 kDa as estimated by sodium dodecyl sulfate polyacrylamide gel electrophoresis and an apparent molecular mass of 44 kDa as estimated by gel filtration chromatography, said gingipain-1 having amidolytic and proteolytic activity for cleavage after arginine residues and having no amidolytic and/or proteolytic activity for cleavage after lysine residues, wherein the amidolytic and/or proteolytic activity is inhibited by cysteine protease group-specific inhibitors including iodoacetamide, iodoacetic acid, N-ethylmaleimide, leupeptin, antipain, transepoxysuccinyl-L-leucylamido-(4-guanidine)butane, TLCK, TPCK, p-aminobenzamidine, N-chlorosuccinamide, and chelating agents including EDTA and EGTA, wherein the amidolytic and/or proteolytic activity of said gingipain-1 is not sensitive to inhibition by human cystatin C, α2-macroglobulin, α1-proteinase inhibitor, antithrombin III, α2-antiplasmin, serine protease group-specific inhibitors including diisopropylfluorophosphate, phenylmethyl sulfonylfluoride and 3,4-diisochlorocoumarin, and wherein the amidolytic and/or proteolytic activities of gingipain-1 are stabilized by Ca²⁺ and wherein the amidolytic and/or proteolytic activities of said gingipain-1 are stimulated by glycine-containing peptides and glycine analogues. In a specifically exemplified gingipain-1 protein, the protein is characterized by an N-terminal amino acid sequence as given in SEQ ID NO:1 Tyr-Thr-Pro-Val-Glu-Glu-Lys-Gln-Asn-Gly-Arg-Met-Ile-Val-Ile-Val-Ala-Lys-Lys-Tyr-Glu-Gly-Asp-Ile-Lys-Asp-Phe-Val-Asp-Trp-Lys-Asn-Gln-Arg-Gly-Leu-Thr-Lys-Xaa-Val-Lys-Xaa-Ala) and by a C-terminal amino acid sequence as given in SEQ ID NO:6 (Glu-Leu-Leu-Arg).

A further object of this invention is a nucleotide sequence encoding a high molecular weight form of Arg-gingipain, termed Arg-gingipain-2 herein, which comprises a proteolytic component essentially as described hereinabove and at least one hemagglutinin component.

As specifically exemplified, the encoded Arg-gingipain-hemagglutinin complex is transcribed as a prepolyprotein, with the amino acid sequence as given in SEQ ID NO:11 from amino acid 1-1704. The encoded mature high molecular weight Arg-gingipain protein has a protease component having a complete deduced amino acid sequence as given in SEQ ID NO:11 from amino acid 228 through amino acid 719. An alternative protease component amino acid sequence is given in SEQ ID NO:4, amino acids 1-510. Arg-gingipain-2 further comprises at least one hemagglutinin component. The hemagglutinin components which are found associated with the 50 kDA Arg-specific proteolytic component are 44 kDa, 27 kDa and 17 kDa, and have amino acid sequences as given in SEQ ID No:11, from 720 to 1091, from 1092 to 1429 and from 1430 to 1704, respectively.

It is an additional object of the invention to provide nucleic acid molecules for the recombinant production of an Arg-gingipain. Substantially pure recombinant Arg-gingipain-1 protein can be prepared after expression of the nucleotide sequences encoding Arg-gingipain in a heterologous host cell using the methods disclosed herein. Said substantially pure Arg-gingipain-1 exhibits amidolytic and/or proteolytic activity with specificity for cleavage after arginine, but exhibits no amidolytic and/or proteolytic activity with specificity for cleavage after lysine residues. The purification method exemplified herein comprises the steps of precipitating extracellular protein from cell-free culture supernatant of Porphyromonas gingivalis with ammonium sulfate (90% w/v saturation), fractionating the precipitated proteins by gel filtration, further fractionating by anion exchange chromatography those proteins in the fractions from gel filtration with the highest specific activity for amidolytic activity as measured with Benzoyl-L-arginyl-p-nitroanilide and collecting those proteins which were not bound to the anion exchange column, and fractionating those proteins by FPLC over a cation exchange column (MonoS HR5/5, Pharmacia, Piscataway, NJ) and finally separating gingipain-1 from lysine-specific proteolytic/amidolytic proteins) by affinity chromatography over L-arginyl-agarose. Preferably the P. gingivalis used is strain H66, and preferably the culture is grown to early stationary phase. Arg-gingipain-1 can also be purified from cells using appropriate modifications of the foregoing procedures (cells must be disrupted, e.g., by lysis in a French pressure cell). Preferably the gel filtration step is carried out using Sephadex G-150, the anion exchange chromatography step is carried out using diethylaminoethyl (DEAE)-cellulose, the FPLC step is carried out using Mono S, and the affinity chromatography is carried out using L-arginyl-Sepharose 4B.

It is a further object of this invention to provide recombinant polynucleotides (e.g., a recombinant DNA molecule) comprising a nucleotide sequence encoding an Arg-gingipain protein, preferably having an amino acid sequence as given in SEQ ID NO:11 from amino acid 228 through amino acid 719 or having an amino acid sequence as given in SEQ ID NO:4, amino acids 1 through 510. As specifically exemplified herein, the nucleotide sequence encoding a mature Arg-gingipain protease is given in SEQ ID NO:10, nucleotides 1630 through 3105, or SEQ ID NO:3 from nucleotides 1630 through 3105. The skilled artisan will understand that the amino acid sequence of the exemplified gingipain protein can be used to identify and isolate additional, nonexemplified nucleotide sequences which will encode a functional protein of the same amino acid sequence as given in SEQ ID NO:4 from amino acid 1 through amino acid 510 or an amino acid sequence of greater than 90% identity and having equivalent biological activity. The skilled artisan understands that it may be desirable to express the Arg-gingipain as a secreted protein; if so, he knows how to modify the exemplified coding sequence for the "mature" gingipain-2 by adding a nucleotide sequence encoding a signal peptide appropriate to the host in which the sequence is expressed. When it is desired that the sequence encoding an Arg-gingipain protein be expressed, then the skilled artisan will operably link transcription and translational control regulatory sequences to the coding sequences, with the choice of the regulatory sequences being determined by the host in which the coding sequence is to be expressed. With respect to a recombinant DNA molecule carrying an Arg-gingipain coding sequence, the skilled artisan will choose a vector (such as a plasmid or a viral vector) which can be introduced into and which can replicate in the host cell. The host cell can be a bacterium, preferably Escherichia coli, or a yeast or mammalian cell.

Also provided is a specific exemplification of a nucleotide sequence encoding an Arg-gingipain, including low molecular weight gingipain-1 protease component and the protease component of high molecular weight gingipain and its associated hemagglutinin components. These components are processed from a prepolyprotein. As specifically exemplified, the coding sequence, from nucleotide 949 to nucleotide 6063 in SEQ ID NO:10, including the stop codon, encodes a prepolyprotein having an amino acid sequence as given in SEQ ID NO:11. The prepolyprotein is encoded by a nucleotide sequence as given in SEQ ID NO:10 from nucleotide 949 to 6063. The mature protease molecule is encoded at nucleotides 1630 through 3105 in SEQ ID NO:10. The mature Arg-specific proteolytic component has an amino acid sequence as given in SEQ ID NO:11 from 228-719, and the hemagglutinin component has an amino acid sequence as in SEQ ID No:11 from 720-1091, from 1092 to 1429 or from 1430 to 1704.

In another embodiment, recombinant polynucleotides which encode an Arg-gingipain, including, e.g., protein fusions or deletions, as well as expression systems are provided. Expression systems are defined as polynucleotides which, when transformed into an appropriate host cell, can express a proteinase. The recombinant polynucleotides possess a nucleotide sequence which is substantially similar to a natural Arggingipain-encoding polynucleotide or a fragment thereof.

The polynucleotides include RNA, cDNA, genomic DNA, synthetic forms, and mixed polymers, both sense and antisense strands, and may be chemically or biochemically modified or contain non-natural or derivatized nucleotide bases. DNA is preferred. Recombinant polynucleotides comprising sequences otherwise not naturally occurring are also provided by this invention, as are alterations of a wild type proteinase sequence, including but not limited to deletion, insertion, substitution of one or more nucleotides or by fusion to other polynucleotide sequences.

The present invention also provides for fusion polypeptides comprising an Arg-gingipain. Homologous polypeptides may be fusions between two or more proteinase sequences or between the sequences of a proteinase and a related protein. Likewise, heterologous fusions may be constructed which would exhibit a combination of properties or activities of the proteins from which they are derived. Fusion partners include but are not limited to immunoglobulins, ubiquitin bacterial β-galactosidase, trpE, protein A, β-lactamase, alpha amylase, alcohol dehydrogenase and yeast alpha mating factor, (Godowski et al. (1988) Science, 241, 812-816). Fusion proteins will typically be made by recombinant methods but may be chemically synthesized.

Compositions and immunogenic preparations including but not limited to vaccines, comprising recombinant Arg-gingipain derived from P. gingivalis and a suitable carrier therefor are provided. Such vaccines are useful, for example, in immunizing an animal, including humans, against inflammatory response and tissue damage caused by P. gingivalis in periodontal disease. The vaccine preparations comprise an immunogenic amount of a proteinase or an immunogenic fragment or subunit thereof. Such vaccines may comprise one or more Arg-gingipain proteinases, or an Arg-gingipain in combination with another protein or other immunogen. By "immunogenic amount" is meant an amount capable of eliciting the production of antibodies directed against one or more Arg-gingipains in an individual to which the vaccine has been administered.

### BRIEF DESCRIPTION OF THE FIGURES

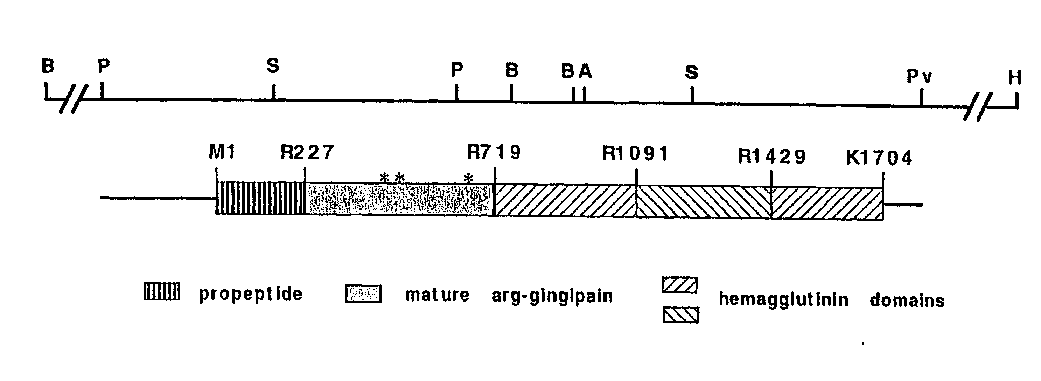

Figure 1 illustrates the composite physical map of an Arg-gingipain locus. The first codon of the mature Arg-gingipain proteolytic component is indicated. Only major restriction sites employed in cloning are indicated: B, *Bam*HI; P, *Pst*I; S, *Sma*I; A, Asp 718; Pv, *Pvu*II; H, *Hind*III. The four arginine cleavage sites (R227, R719, R1091 and R1429) are each indicated with an asterisk (*). The three residues forming the active site (C412, H438 and N669, respectively) are also shown.

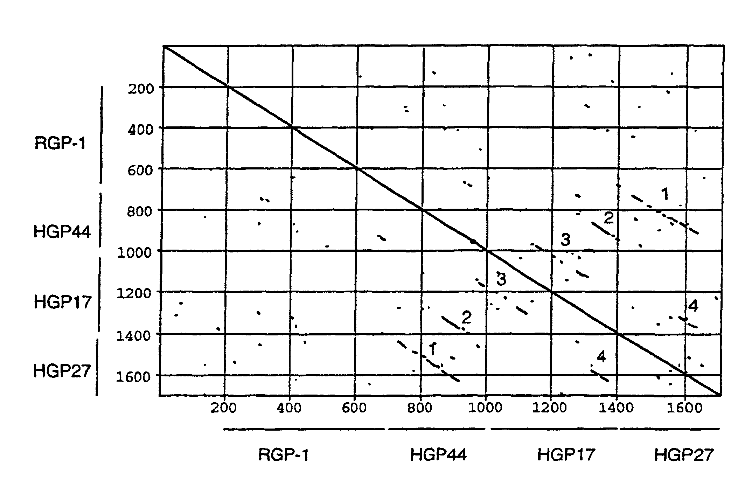

Figure 2 is a protein matrix plot, which presents analysis of regions of similarity between hemagglutinin domains using Pustell Protein Matrix from MacVector, Release 4.0. The complete prepolyprotein sequence (SEQ ID NO:11) was used as X-axis and Y-axis. The perfect diagonal row is the line of identity, whereas structure in the pattern near that diagonal corresponds to internal repeats. The four different domains are represented (Arg-gingipain protease, 44 kDa hemagglutinin, 17 kDa hemagglutinin and 27 kDa hemagglutinin). Four regions of high homology are identified. The main homologies between hemagglutinin domains is shown in detail in Table 4.

### DETAILED DESCRIPTION OF THE INVENTION

Abbreviations used herein for amino acids are standard in the art: X or Xaa represents an amino acid residue that has not yet been identified but may be any amino acid residue including but not limited to phosphorylated tyrosine, threonine or serine, as well as cysteine or a glycosylated amino acid residue. The abbreviations for amino acid residues as used herein are as follows: A, Ala, alanine; V, Val, valine; L, Leu, leucine; I, Ile, isoleucine; P, Pro, proline; F, Phe, phenylalanine; W, Trp, tryptophan; M, Met, methionine; G, Gly, glycine; S, Ser, serine; T, Thr, threonine; C, Cys, cysteine; Y, Tyr, tyrosine; N, Asn, asparagine; Q, Gln, glutamine; D, Asp, aspartic acid; E, Glu, glutamic acid; K, Lys, lysine; R, Arg, arginine; and H, His, histidine. Other abbreviations used herein include Bz, benzoyl; Cbz, carboxybenzoyl; pNA, p-nitroanilide; MeO, methoxy; Suc, succinyl; OR, ornithyl; Pip, pipecolyl; SDS, sodium dodecyl sulfate; TLCK, tosyl-L-lysine chloromethyl ketone; TPCK, tosyl-L-phenylalanine chloromethyl ketone; S-2238, D-Phe-Pip-Arg-pNA, S-2222, Bz-Ile-Glu-(γ-OR)-Gly-pNA; S-2288, D-Ile-Pro-Arg-pNA; S-2251, D-Val-Leu-Lys-pNA; Bis-Tris, 2-[bis(2-hydroxyethyl)amino]-2-(hydroxymethyl)-propane-1,3-diol; FPLC, fast protein liquid chromatography; HPLC, high performance liquid chromatography; Tricine, N-[2-hydroxy-1,1-bis(hydroxymethyl)ethyl]glycine;EGTA, [ethylene-bis(oxyethylene-nitrile)tetraacetic acid; EDTA, ethylenediamine-tetraacetic acid; Z-L-Lys-pNa, Z-L-Lysine-p-Nitroanilide; HMW, high molecular weight.

Arg-gingipain is the term given to a P. gingivalis enzyme with specificity for proteolytic and/or amidolytic activity for cleavage of an amide bond, in which L-arginine contributes the carboxyl group. The Arg-gingipains described herein have identifying characteristics of cysteine dependence, inhibition response as described, Ca²⁺ - stabilization and glycine stimulation. Particular forms of Arg-gingipain are distinguished by their apparent molecular masses of the mature proteins (as measured without boiling before SDS-PAGE). Arg-gingipains of the present invention have no amidolytic or proteolytic activity for amide bonds in which L-lysine contributes the -COOH moiety.

Arg-gingipain-1 is the name given herein to a protein characterized as having a molecular mass of 50 kDa as measured by SDS-PAGE and 44 kDa as measured by gel filtration over Sephadex G-150, having amidolytic and/or proteolytic activity for substrates having L-Arg in the Pₗ position, i.e. on the N-terminal side of the peptide bond to be hydrolyzed but having no activity against corresponding lysine-containing substrates being dependent on cysteine (or other thiol groups for full activity), having sensitivity to cysteine protease group-specific inhibitors including iodoacetamide, iodoacetic acid, and N-methylmaleimide, leupeptin, antipain, trans-epoxysuccinyl-L-leucylamido-(4-guanidino)butane, TLCK, TPCK, p-aminobenzamidine, N-chlorosuccinamide, and chelating agents including EDTA and EGTA, but being resistant to inhibition by human cystatin C, α2-macroglobulin, α1-proteinase inhibitor, antithrombin III, α2-antiplasmin, serine protease group-specific inhibitors including diisopropylfluorophosphate, phenylmethyl sulfonylfluoride and 3,4-diisochlorocoumarin, and wherein the amidolytic and/or proteolytic activities of gingipain-1 are stabilized by Ca²⁺ and wherein the amidolytic and/or proteolytic activities of said gingipain-1 are stimulated by glycine-containing peptides and glycine analogues.

An exemplified Arg-gingipain described and termed Arg-gingipain-2 herein exists in the native form in a high molecular weight form, having an apparent molecular mass of 95 kDa as determined by SDS-PAGE, without boiling of samples. When boiled, the high molecular weight form appears to dissociate into components of 50 kDa, 43 kDa, 27 kDa and 17 kDa. Arg-gingipain-2 is the name given to the 50 kDa, enzymatically active component of the high molecular weight complex.

The complete amino acid sequence of an exemplified mature Arg-gingipain is given in SEQ ID NO:11, from amino acid 228 through amino acid 719. A second possible exemplary amino acid sequence is given in SEQ ID NO:4, amino acids 1 through 510. In nature these proteins are produced by the archebacterium Porphyromonas gingivalis; it can be purified from cells or from culture supernatant or as a recombinant expression product using the methods provided herein. Without wishing to be bound by any theory, it is proposed that these sequences correspond to Arg-gingipain-2.

As used herein with respect to Arg-gingipain-1, a substantially pure Arg-gingipain preparation means that there is only one protein band visible after silver-staining an SDS polyacrylamide gel run with the preparation, and the only amidolytic and/or proteolytic activities are those with specificity for L-arginine in the Pₗ position relative to the bond cleaved. A substantially pure high molecular weight Arg-gingipain preparation has only one band (95 kDa) on SDS-PAGE (sample not boiled) or four bands (50 kDa, 43 kDa, 27 kDa, 17 kDa; sample boiled). No amidolytic or proteolytic activity for substrates with lysine in the Pₗ position is evident in a substantially pure high molecular weight or Arg-gingipain-2 preparation. Furthermore, a substantially pure preparation of Arg-gingipain has been separated from components with which it occurs in nature. Substantially pure Arg-gingipain is substantially free of naturally associated components when separated from the native contaminants which accompany them in their natural state. Thus, Arg-gingipain that is chemically synthesized or recombinantly synthesized in a cellular system different from the cell from which it naturally originates will be substantially free from its naturally associated components. Techniques for synthesis of polypeptides are described, for example, in Merrifield (1963) *J. Amer. chem. Soc.,* 85, 2149-2156.

A chemically synthesized Arg-gingipain protein is considered an "isolated" polypeptide, as is an Arg-gingipain produced as an expression product of an isolated proteinase-encoding polynucleotide which is part of an expression vector (i.e., a "recombinant proteinase") , even if expressed in a homologous cell type.

Recombinant Arg-gingipain-1, Arg-gingipain-2 and HMW Arg-gingipain can be obtained by culturing host cells transformed with the recombinant polynucleotides comprising nucleotide sequences encoding an Arg-gingipain as described herein under conditions suitable to attain expression of the proteinase-encoding sequence.

Example 1 below and Chen et al. (1992) supra describe the purification of Arg-gingipain-1 and HMW Arg-gingipain from P. gingivalis culture supernatant, i.e., from a natural source. Various methods for the isolation of an Arg-gingipain from other biological material, such as from nonexemplified strains of P. gingivalis or from cells transformed with recombinant polynucleotides encoding such proteins, may be accomplished by methods known in the art. Various methods of protein purification are known in the art, including those described, e.g., in Guide to Protein Purification, ed. Deutscher, Vol. 182 of Methods in Enzymology (Academic Press, Inc.: San Diego, 1990) and Scopes, Protein purification: Principles and Practice (Springer-Verlag: New York, 1982).

Chromatography over Sephadex G-150 yielded four peaks with Bz-L-Arg-pNA-hydrolyzing activity. In each of these fractions, the hydrolytic activity was dependent on cysteine and enhanced many-fold by the addition of glycyl-glycine or glycine amide. Antibody specific for Arg-gingipain-1 immunoprecipitates proteinase from all four Sephadex G-150 peaks. Without wishing to be bound by any particular theory, it is postulated that the four-peak Bz-L-Arg-pNA-amidolytic profile is an anomaly resulting from the binding of gingipain-1 to membrane or nucleic acid fragments. Alternatively, those peaks containing higher molecular weight protein may contain partially processed gingipain-1 precursors. Although the purification of gingipain-1 as exemplified is from extracellular protein, it can also be purified from the bacterial cells.

Further analysis (see Example 1) of the high molecular weight fractions containing Arg-specific amidolytic and proteolytic activity revealed that Arg-gingipain-2 (50 kDa) occurred non-covalently bound to proteins of 44 kDa, 27 kDa and 17 kDa, which have hemagglutinin activity. The empirically determined N-terminal amino acid sequence of the complexed 44 kDa protein corresponds to amino acids 720-736 of SEQ ID NO:11.

Arg-Gingipain-1 was further purified from the Sephadex G-150 Peak 4 protein mixture by further steps of anion exchange chromatography over DEAE-cellulose and two runs over Mono S FPLC. Arg-gingipain-1 recovery was markedly reduced if an affinity chromatography step (L-Arginyl-Sepharose 4B) was used to remove trace amounts of a contaminating proteinase with specificity for cleavage after lysine residues.

Purified Arg-gingipain-1 exhibits an apparent molecular mass of about 50 KDa as determined by SDS-polyacrylamide gel electrophoresis. The size estimate obtained by gel filtration on Superose 12 (Pharmacia, Piscataway, NJ) is 44 kDa. Amino-terminal sequence analysis through 43 residues gave a unique structure which showed no homology with any other proteins, based on a comparison in the protein NBRS data base, release 39.0. The sequence obtained is as follows: The C-terminal amino acid sequence of the gingipain-1 (major form recognized in zymography SDS-PAGE, 0.1% gelatin in gel), was found to be Glu-Leu-Leu-Arg. (SEQ ID NO:5). This corresponds to the amino acids 716-719 in SEQ ID NO:4 and nucleotides 3094-3105 in SEQ ID NO:3. This is consistent with the model for autoproteolytic processing of the precursor polyprotein to produce the mature 50 kDa gingipain-1 protein.

Comparison of SEQ ID NO:1 with SEQ ID NO:4 and 11 shows differences at amino acids 37-38 of the mature Arg-gingipain. Without wishing to be bound by any theory, it is proposed that SEQ ID NO:3 (or SEQ ID NO:10) comprises the coding sequence for Arg-gingipain-2, the enzymatically active component of the high molecular weight form of Arg-gingipain. This is consistent with the observation that there are at least two genes with substantial nucleic acid homology to the Arg-gingipain-specific probe.

The enzymatic activity of Arg-gingipain-1 is stimulated by glycine and glycine-containing compounds. In the absence of a glycine-containing compound, the enzyme has essentially the same amidolytic activity in the pH range 7.5-9.0. However, in the presence of glycyl-glycine, e.g., substantial sharpening of the pH range for activity is observed, with the optimum being between pH 7.4 and 8.0. Preliminary kinetic data indicate that the effect of glycine and glycine analogues is to raise both k_{cat} and Kₘ equally so that the k_{cat}/Kₘ ratio does not change. It is therefore likely that these compounds bind to the enzyme and/or substrate after an enzyme-substrate complex has already formed. The high molecular weight form is stimulated only about half as much by glycine compounds.

Arg-gingipain-1 requires cysteine for full amidolytic activity, and, although it is stimulated by other thiol-containing compounds, the effect was less pronounced. Cysteine and cysteamine are most efficient, presumably because they perform the dual roles of reducing agents and glycine analogues.

The amidolytic activity of Arg-gingipain-1 is inhibited by a number of -SH blocking group reagents, oxidants, Ca²⁺ chelating agents, and Zn²⁺. The effect of chelating agents EDTA and EGTA was reversed completely by the addition of excess Ca²⁺, whereas in the case of Zn²⁺, it was necessary to add o-phenanthroline prior to Ca²⁺.

Typical serine proteinase group-specific inhibitors have no effect on enzyme activity, and it is likely that inhibition by both TLCK and TPCK was caused by reaction with an essential cysteine residue in the enzyme, a known property of chloromethyl ketone derivatives. Significantly, Arg-gingipain-1 was inhibited by such cysteine proteinase inhibitors as trans-epoxysuccinyl-L-leucylamido-(4-guanidino)butane, leupeptin and antipain. Although the reactions were not stoichiometric, the inhibition was concentration-dependent. However, human cystatin C, an inhibitor of mammalian and plant cysteine proteinases, does not inhibit Arg-gingipain-1, nor did any of the trypsin-specific inhibitors from human plasma, including α2-macroglobulin, α1-proteinase inhibitor, antithrombin III, and α2-antiplasmin. Indeed, preliminary investigations actually suggested that the inhibitor in each case was being inactivated by Arg-gingipain-1.

Calcium ion stabilizes Arg-gingipain-1 without directly affecting activity. With Ca²⁺ present the enzyme is stable in the pH range between 4.5 and 7.5 for several days at 4°C. However, below pH 4.0 or in the absence of Ca²⁺, enzyme activity is quickly lost. At **37°C** Ca²⁺ considerably increases stability, although activity is lost more rapidly than at the lower temperature. At -20°C Arg-gingipain-1 is stable for several months. During lyophilization, however, it irreversibly loses more than 90% of its catalytic activity.

The amidolytic activity of the purified Arg-gingipain-1 on synthetic peptide substrates was limited to substrates with a P₁-Arg residue. Even then Arg-gingipain-1 had significantly different turnover rates on individual substrates, being most effective against S-2238 (D-Phe-Pip-Arg-pNA) and S-2222 (Bz-Ile-Glu-(γ-OR)-Gly Arg-pNA). Lesser, comparable activity was observed using S2288 (D-Ile-Pro-Arg-pNA) and Bz-Arg-pNA. D-Val-Leu-Lys-pNA (S-2251), Suc-Ala-Ala-Ala-pNA, MeO-Suc-Ala-Ala--Pro-Val-pNA, Suc-Ala-Ala-Pro-Phe-pNA, Gly-Pro-pNA and Cbz-Phe-Leu-Glu-pNA had essentially no substrate activity. This narrow specificity was confirmed by examination of the cleavage products after incubation with the insulin B chain or mellitin; it was found that cleavage occurred specifically after only Arg residues, but not after Lys or any other amino acids unless the last affinity chromatography step over L-Arginine-Sepharose 4B was omitted.

Because progressive periodontitis is characterized by tissue degradation, collagen destruction and a strong inflammatory response, and because P. gingivalis was known to exhibit complement-hydrolyzing activity, purified Arg-gingipain-1 was tested for proteinase activity using purified human complement C3 and C5 as substrates (See Wingrove et al. (1992) J. Biol. Chem. 267: 18902-18907). Low molecular weight Arg-gingipain selectively cleaved the α-chain, generating what initially appeared to be the α'-chain of C3b. Further breakdown fragments of the C3 α'-chain were observed and a decreasing intensity of the α'-band suggested that degradation continued. Visual evidence suggested that the C3 β-chain is resistant to this proteinase. Attempts to demonstrate C3a biological activity in the C3 digestion mixture were unsuccessful, and the C3a-like fragment released from the α-chain was extensively degraded by Arg-gingipain-1.

Human C5 was also digested by Arg-gingipain-1, with initial cleavage specific for the C5 α-chain, as in the case of C3. The α-1 (86 kDa) and the α-2 (30 kDa) fragments were the first polypeptides to be formed from cleavage of C5 by gingipain-1, and they equal the molecular weight of the intact α-chain, a fragment in the size range of C5a was observed. C5a is more resistant to the Arg-gingipain-1 than C3a, and functional C5a may accumulate without further appreciable degradation. C5a biological activity was detected after digestion of human C5 with Arg-gingipain-1. Characteristic morphologic changes in human neutrophils, known as polarization, were scored by counting deformed cells relative to normally rounded cells.

To test for in vivo biological activity, the purified low molecular weight Arg-gingipain enzyme was injected into guinea pig skin. It induced vascular permeability enhancement at concentrations greater than 10⁻⁸ M in dose-dependent and proteolytic activity dependent manners. Vascular permeability enhancement activity was not inhibited by diphenhydramine (an antihistamine), and the activity was enhanced by SQ 20,881 (angiotensin-converting enzyme inhibitor). The vascular permeability enhancement by Arg-gingipain-1 was inhibited by soybean trypsin inhibitor (SBTI) at a concentration of 10⁻⁵ M, a concentration at which SBTI did not inhibit enzymatic activity, as measured with Bz-L-Arg-pNA and azocasein as the substrates.

Human plasma or guinea pig plasma treated with Arg-gingipain-1 (10⁻⁸ to 10⁻⁶ M) induced vascular permeability enhancement in the guinea pig skin assay. Vascular permeability enhancement by Arg-gingipain-1 treated plasma was increased by addition of 1,10-phenanthroline (kinase inhibitor, chelating agent for Zn ions) to a final concentration of 1 mM. Vascular permeability enhancement by Arg-gingipain-1 treated plasmas was markedly reduced when plasmas deficient in Hageman factor, prekallikrein or high molecular weight kininogen were used. These results indicate that vascular permeabilizing enhancement by Arg-gingipain-1 acts via activation of Hageman factor and the subsequent release of bradykinin from high molecular weight kininogen by kallikrein.

Intradermal injection of Arg-gingipain-1 in the guinea pig also resulted in neutrophil accumulation at the site of injection, an activity which was dependent on proteolytic activity.

The foregoing results demonstrate the ability of Arg-gingipain to elicit inflammatory responses in a guinea pig animal model.

Recombinant Arg-gingipain is useful in methods of identifying agents that modulate Arg-gingipain proteinase activity, whether by acting on the proteinase itself or preventing the interaction of a proteinase with a protein in gingival area, such as C3 or C5. One such method comprises the steps of incubating a proteinase with a putative therapeutic, i.e., Arg-gingipain-inhibiting agent; determining the activity of the proteinase incubated with the agent; and comparing the activity obtained in step with the activity of a control sample of proteinase that has not been incubated with the agent.

SDS-PAGE analysis (without boiling) of the purified high molecular weight form of Arg-gingipain revealed a single band of apparent molecular mass of 95 kDa. This estimate was confirmed by analytical chromatography over a TSK 3000SW gel filtration column. When the enzyme preparation was boiled before SDS-PAGE, however, bands of apparent molecular masses of approximately 50 kDa, 44 kDa, 27 kDa and 17 kDa were observed. These bands were not generated by treatments at temperatures below boiling, by reducing agents or detergents. It was concluded that the 95 kDa band was the result of strong non-covalent binding between the lower molecular weight proteins.

The 50 kDa proteolytic component of the high molecular weight Arg-gingipain was characterized with respect to N-terminal amino acid sequence over 22 amino acids. The sequence was identical to the first 22 amino acids of the 50 kDa, low molecular weight Arg-gingipain-1. Characterization of the high molecular weight Arg-gingipain activity showed the same dependence on cysteine (or other thiols) and the same spectrum of response to potential inhibitors. Although the high molecular weight Arg-gingipain was stimulated by glycine compounds, the response was only about half that observed for the low molecular weight form.

The primary structure of the NH₂-terminus of low molecular weight Arg-gingipain determined by direct amino acid sequencing (SEQ ID NO:1) was used to prepare a mixture of synthetic primer oligonucleotides GIN-1-32 (SEQ ID NO:6) coding for amino acids 2 to 8 of the mature protein and primer GIN-2-30 (SEQ ID NO:7) coding for amino acids 25-32 of the mature protein. These primers were used in PCR on P. gingivalis DNA. A single 105-base pair product (P105) resulted. This was cloned into pCR-Script^{TM}SK(-) (Stratagene) and sequenced. Sequence analysis of P105 generated 49 nucleotides from an Arg-gingipain coding sequence. On the basis of the sequence of P105, another primer (GIN-8S-48) SEQ ID NO:8 corresponding to the coding strand of the partial Arg-gingipain gene (48-mers) was synthesized in order to screen the λDASH DNA library using a ³²P-labeled GIN-8S-48 probe. A partial sequence of the Arg-gingipain gene (nucleotides 1-3159, SEQ ID NO:3) was determined by screening the P. gingivalis DNA library using ³²P-labeled hybridization GIN-8S-48 probe (SEQ ID NO;8). From a total of 2x10⁵ independent plaques screened, seven positive clones were isolated and purified. After extraction and purification, the DNA was analyzed by restriction enzymes: One clone (A1) has a 3.5 kb *Bam*HI fragment and a 3 kb *PstI* fragment; another clone (B1 has a 9.4 kb *Bam*HI fragment and a 9.4 kb PstI fragment; and 5 clones have a 9.4 kb *Bam*HI fragment and a 10 kb *Pst*I fragment. These results are similar to those obtained by Southern analysis of P. gingivalis DNA and are consistent with the existence of at least two Arg-gingipain genes. The A1 clone was chosen for sequencing because the expected DNA size to encode a 50-kDa protein is approximately 1.35 kb. The 3.159 kb *Pst*I/*Bam*HI fragment from clone A1 was subsequently subcloned into pBluescript SK(-) as a *Pst*I fragment and a *Sma*I/*Bam*HI fragment and into M13mp18 and 19 as a *Pst*I fragment and a *Pst*I/*Bam*HI fragment and sequenced. In order to clone the stop codon of gingipain-1, which was missing in the *Pst*I/*Bam*HI fragment, *Pst*I/*Hind*III double digested P. gingivalis DNA clones were hybridized with ³²P-labeled GIN-14-20 (SEQ ID NO:9) (nucleotides 2911-2930 of SEQ ID NO:3) localized at the 3' end of this clone. A *Pst*I/*Hind*III fragment of approximately 4.3 kb was identified and cloned into pbluescript SK(-). Smaller fragment *(Pst*I/*Asp*713 and *Bam*HI/*Hind*III) was also subcloned into M13mp18 and 19.

SEQ ID NO:3 is the DNA sequence of the 3159 bp *Pst*I/*Bam*HI fragment (see Table 1).

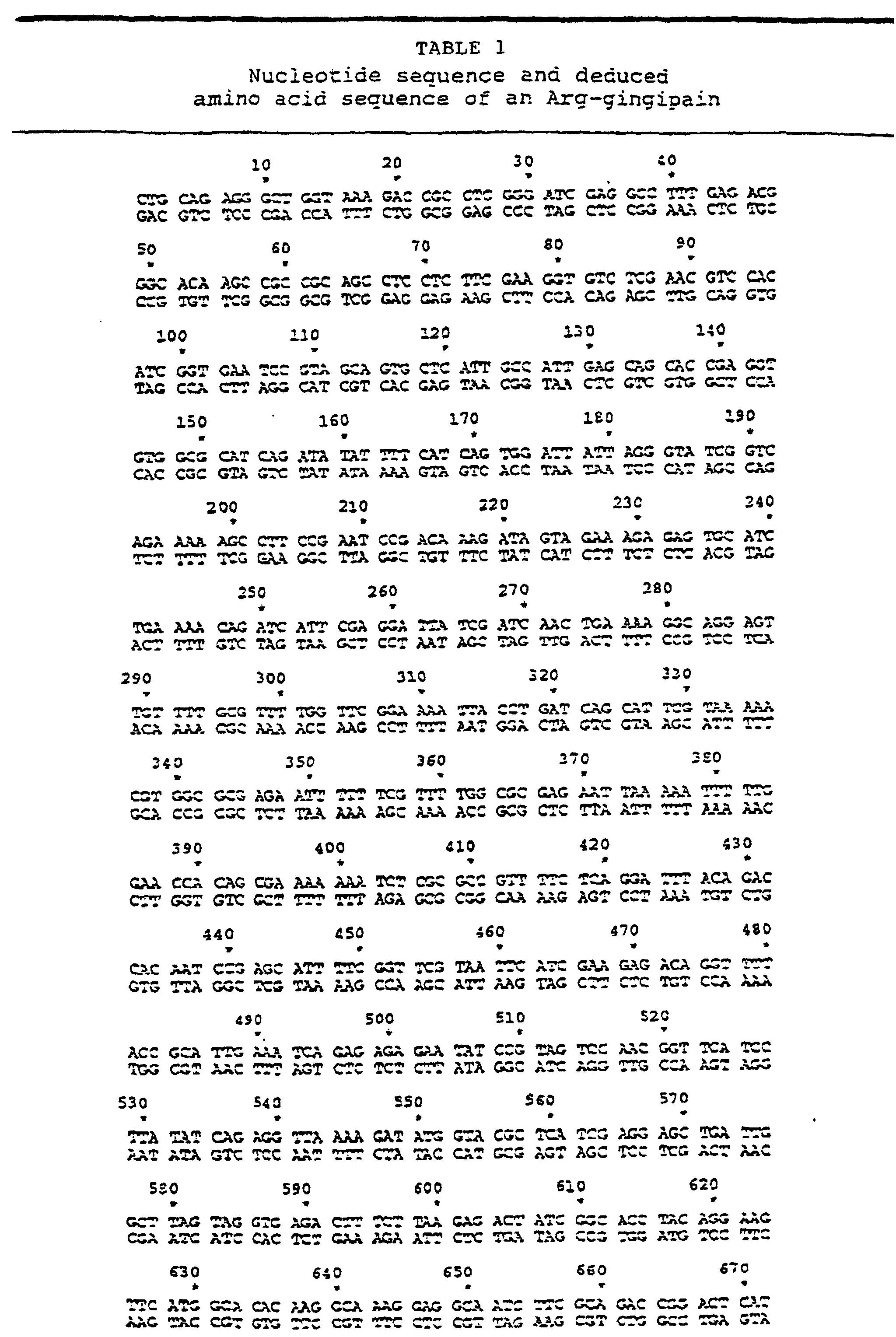

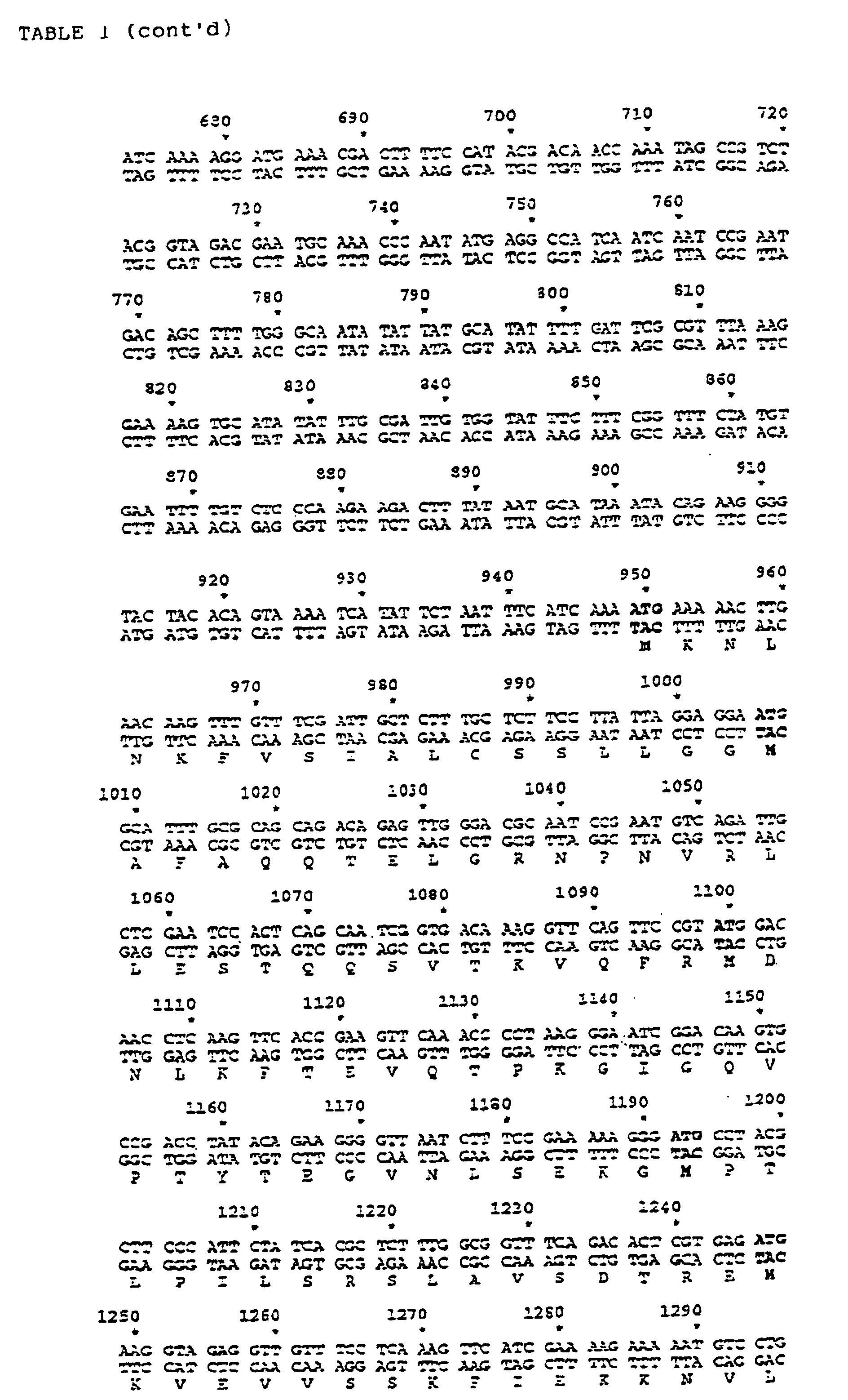

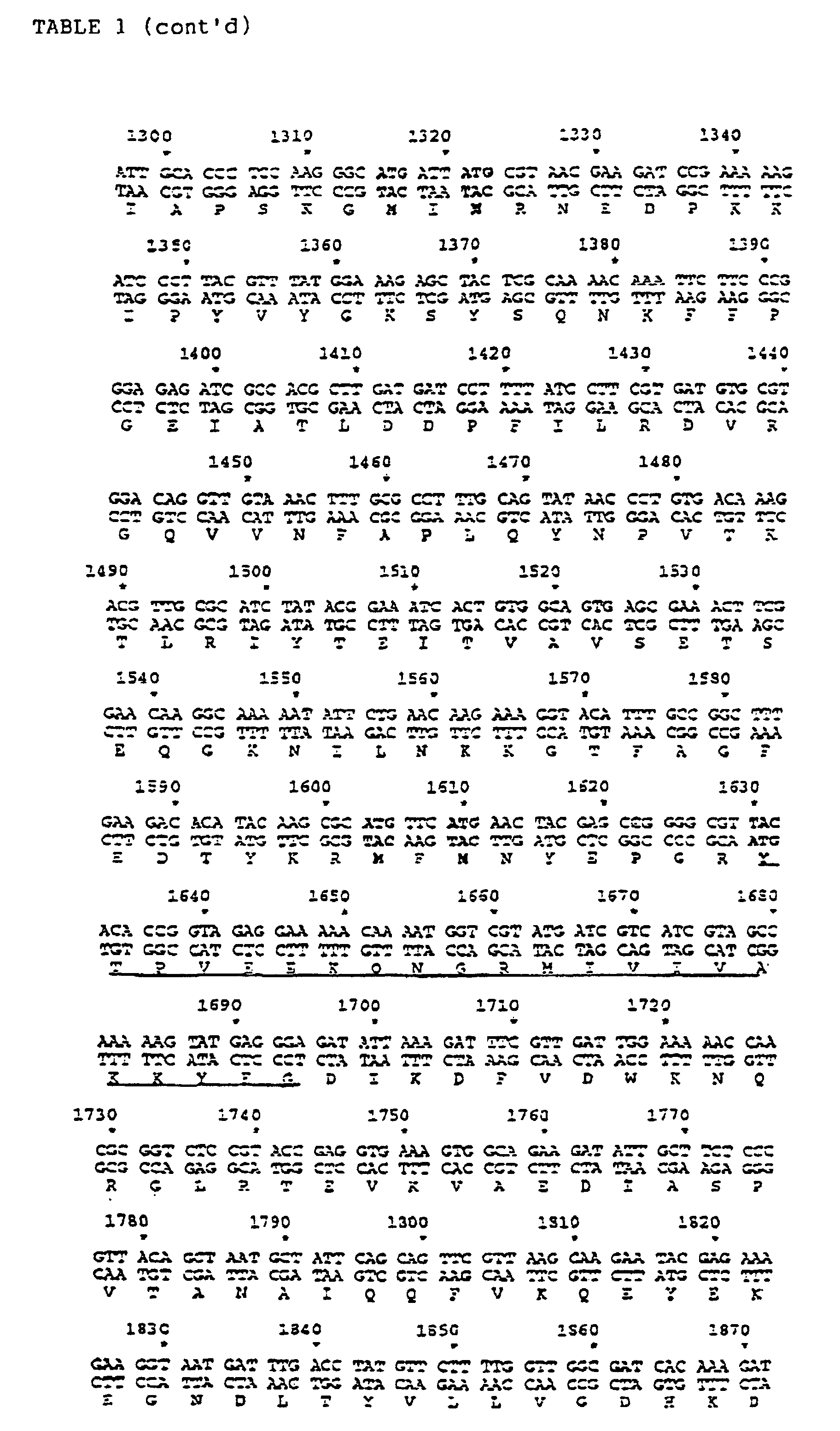

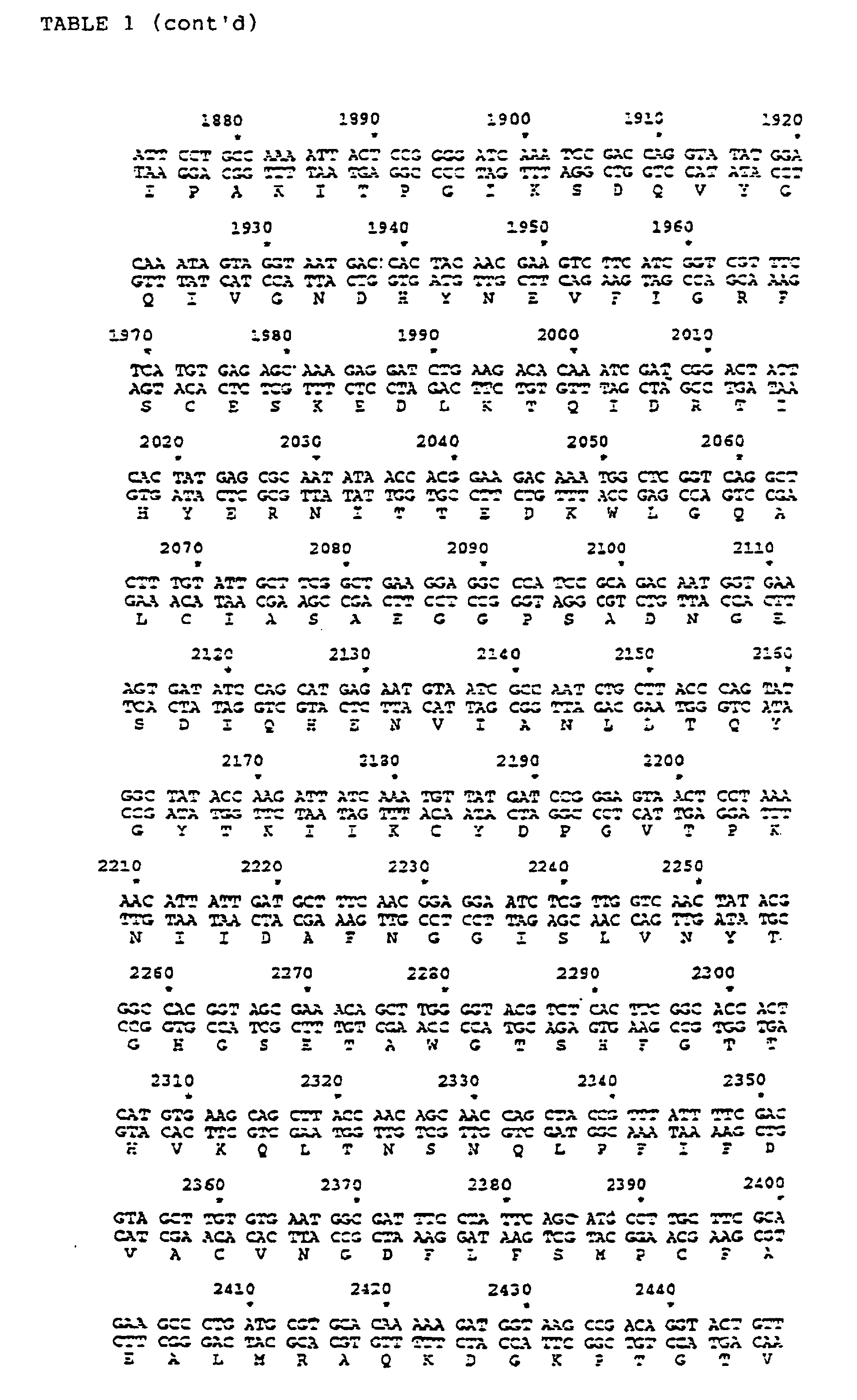

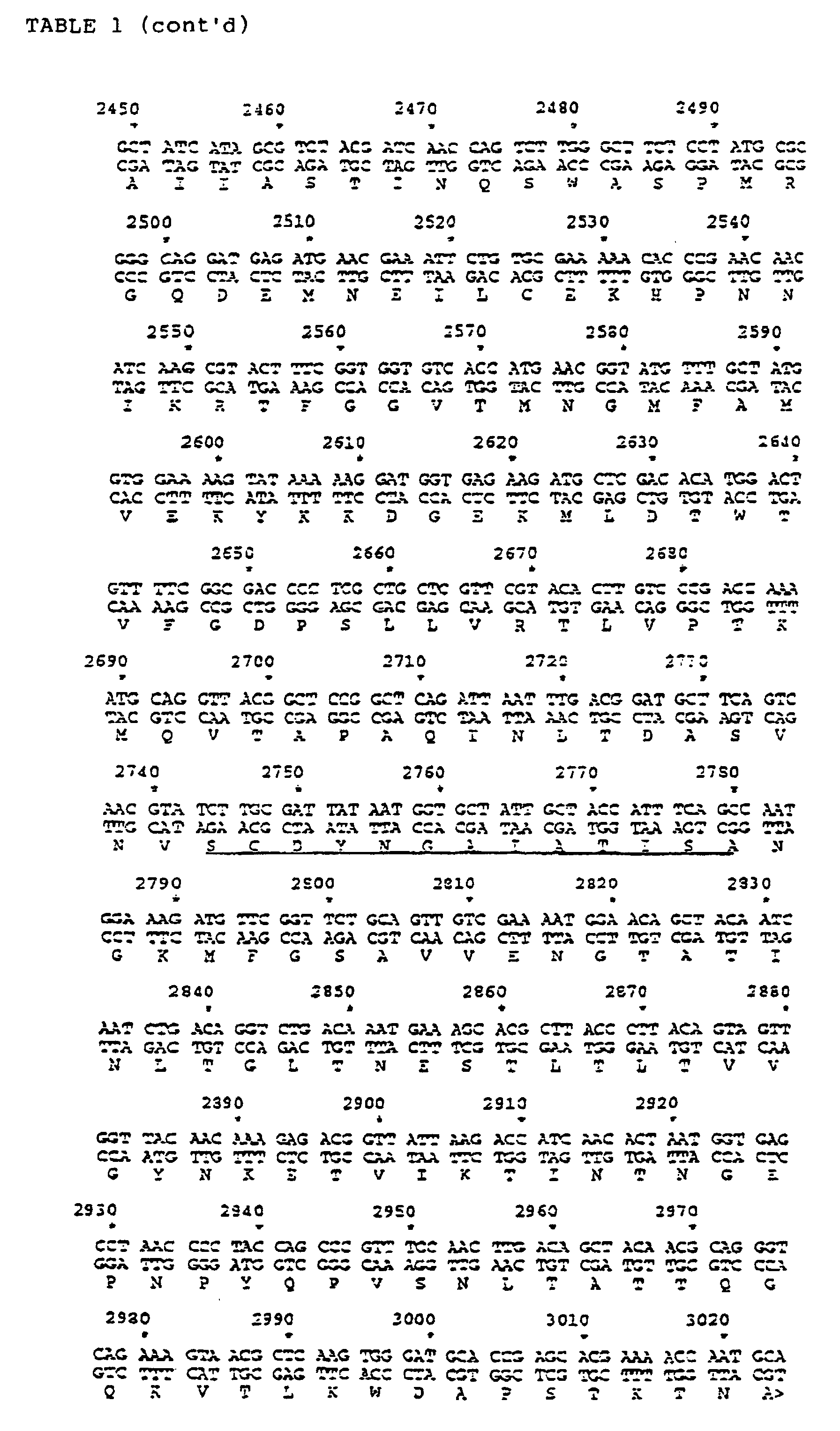

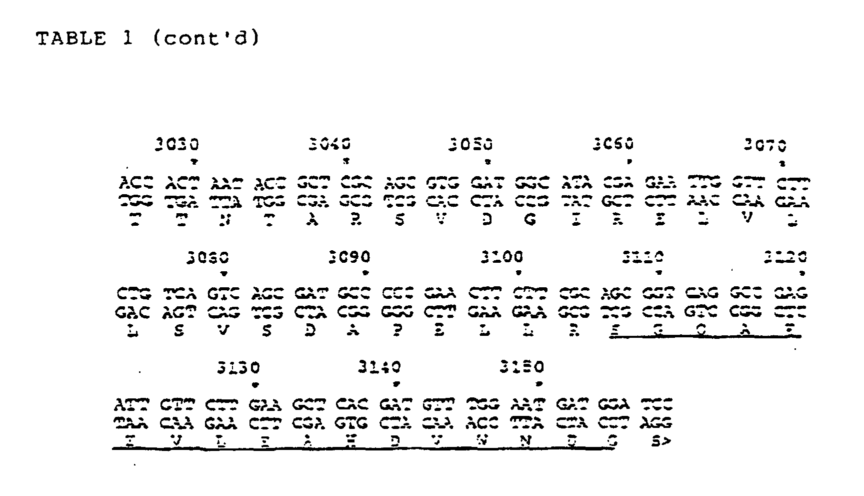

Exemplified nucleotide sequences encoding a mature Arg-gingipain, termed an Arg-gingipain-2 herein, extends from 1630-3105 in SEQ ID NO:3 and in SEQ ID NO:10. The first ATG appears at nucleotide 949 and is followed by a long open reading frame (ORF), of 5111 bp in Table 2 (SEQ ID NO:10). This ORF was the largest one observed. However, the first ATG is following by 8 others in frame (at nucleotides 1006, 1099, 1192, 1246, 1315, 1321, 1603, and 1609). The most likely candidate to initiate translation is currently unknown. Which of these initiation codons are used in translation of the Arg-gingipain-2 precursor can be determined by expression of the polyprotein in bacteria and subsequent amino-terminal sequence analysis of proprotein intermediates. The sequence derived from 5' noncoding sequences is composed of 948 bp. The primary structure of the mature Arg-gingipain molecule can be inferred from the empirical amino-terminal and carboxy-terminal sequences and molecular mass. Thus, mature Arg-gingipain-2 has an amino terminus starting at nucleotide residue 1630 in SEQ ID NO:3 and at amino acid 1 in SEQ ID NO:4. As expected for an arginine-specific protease, the mature protein is cleaved after an arginine residue. The 50 kDa and the 44 kDa bands from Bz-L-Arg-pNa activity peaks have an identical sequence to that deduced amino acid sequence of gingipain, encoded respectively at nucleotides 1630-1695 and at nucleotides 3106-3156. From these data, the carboxyl terminus is most likely derived from autoproteolytic processing after the arginine residue encoded at 3103-3105 where the amino terminus encoding sequence of a hemagglutinin component starts (nucleotide 3106). The deduced 492 amino acids of gingipain-2 give rise to a protease molecule with a calculated molecular weight of 54 kDa which correlates well with the molecular mass of 50 kDa determined by SDS-PAGE analysis. Tables 1 and 2 (see also SEQ ID NO:10 and 11) presents the coding sequence and deduced amino acid sequence of gingipain-2. The first nucleotide presented in the sequence belongs to the *Pst*I cloning site and is referred as nucleotide 1. Bold face letters indicate the potential sites of initiation ATG and the first codon of the mature gingipain-2. The amino terminal sequence of gingipain-2 and the amino terminal sequence of 44 kDa bands from Bz-L-Arg-pNa activity peaks are underlined.

Table 2 (corresponding to SEQ ID NOS:10-11) presents the nucleotide sequence encoding the complete prepolyprotein sequence, including both the protease component and the hemagglutinin ccmponent(s) of HMW Arg-gingipain. The coding sequence extends from an ATG at nucleotide 949 through a TAG stop codon at nucleotide 6063 in SEQ ID NO:10. The deduced amino acid sequence is given in SEQ ID NO:11.

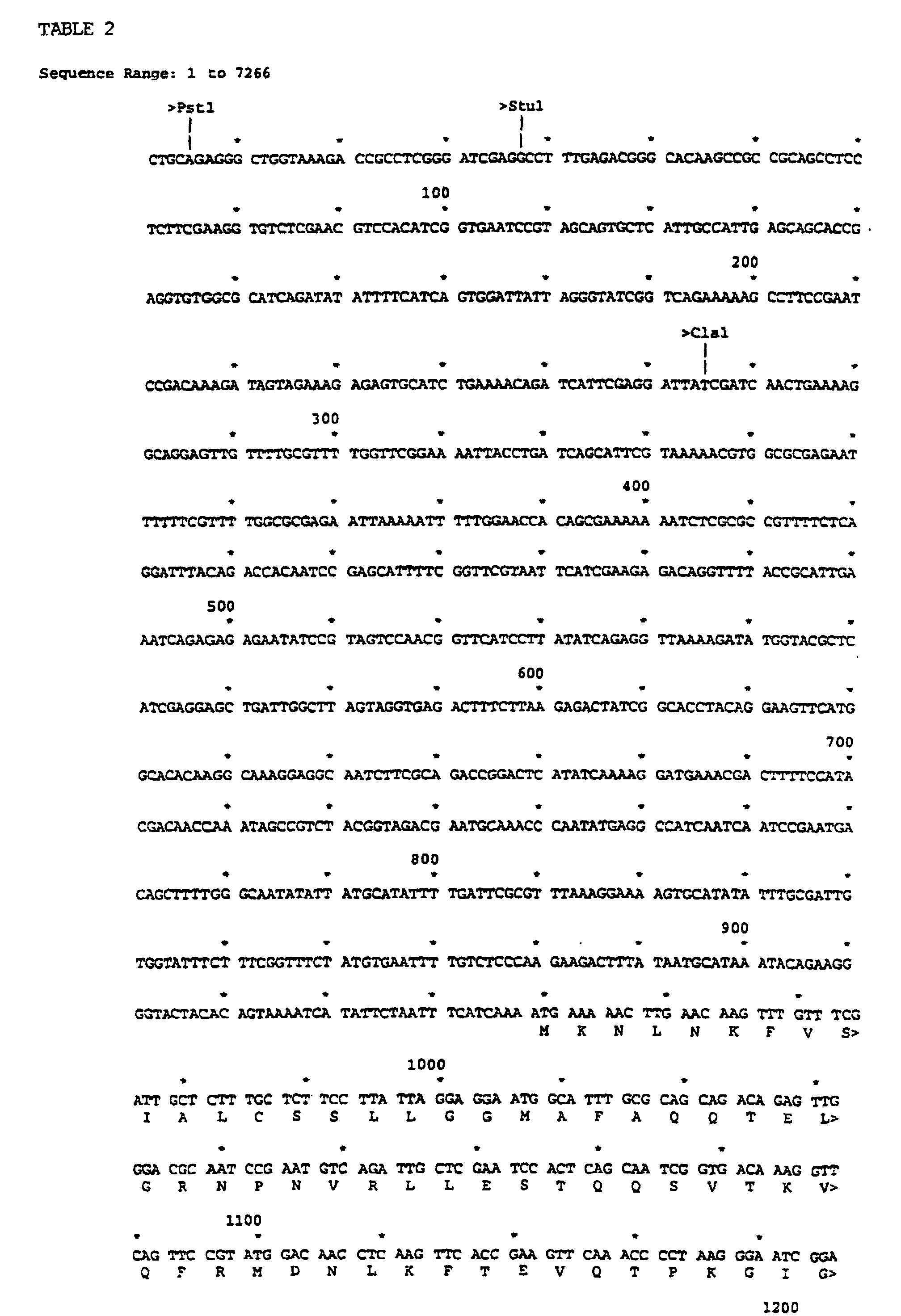

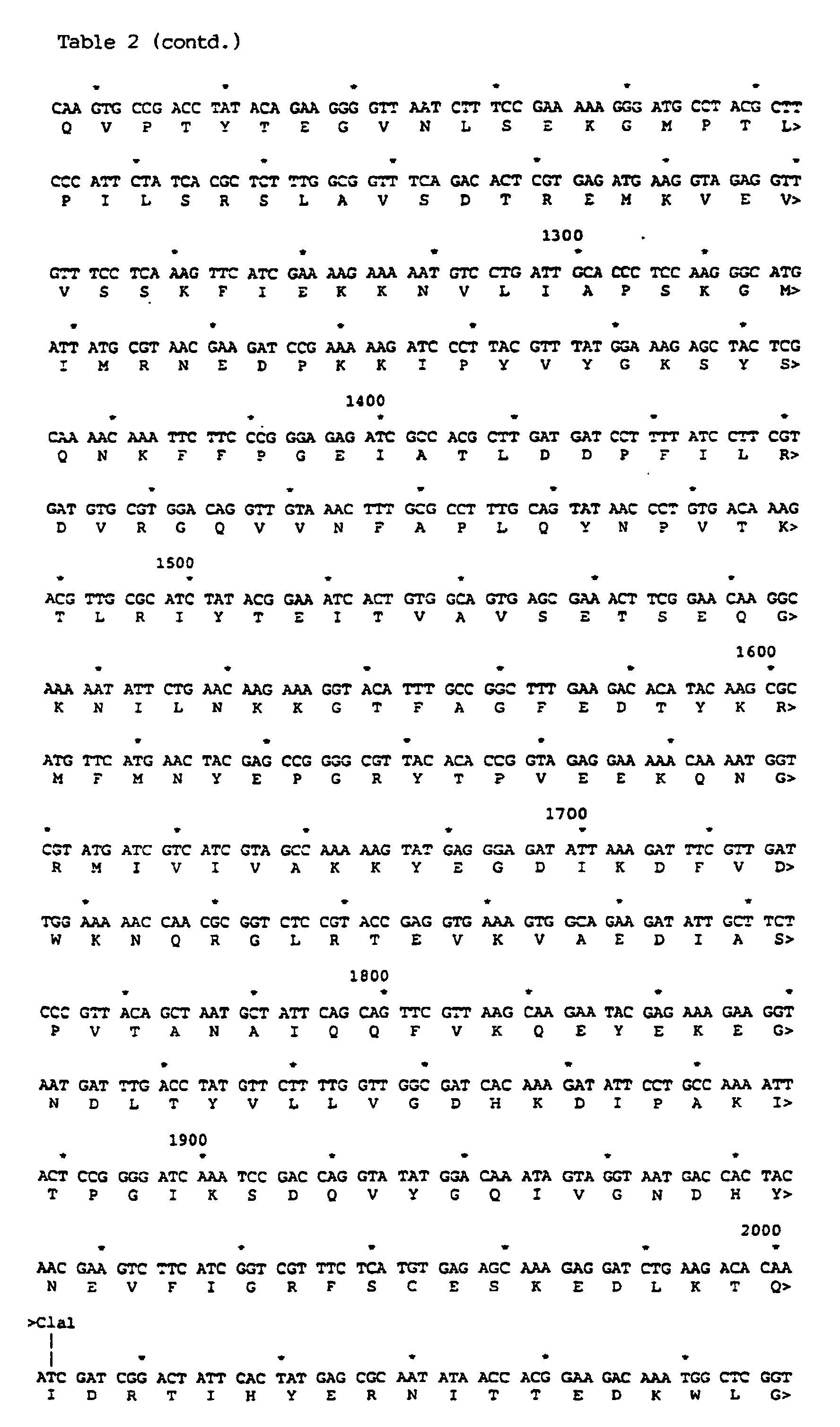

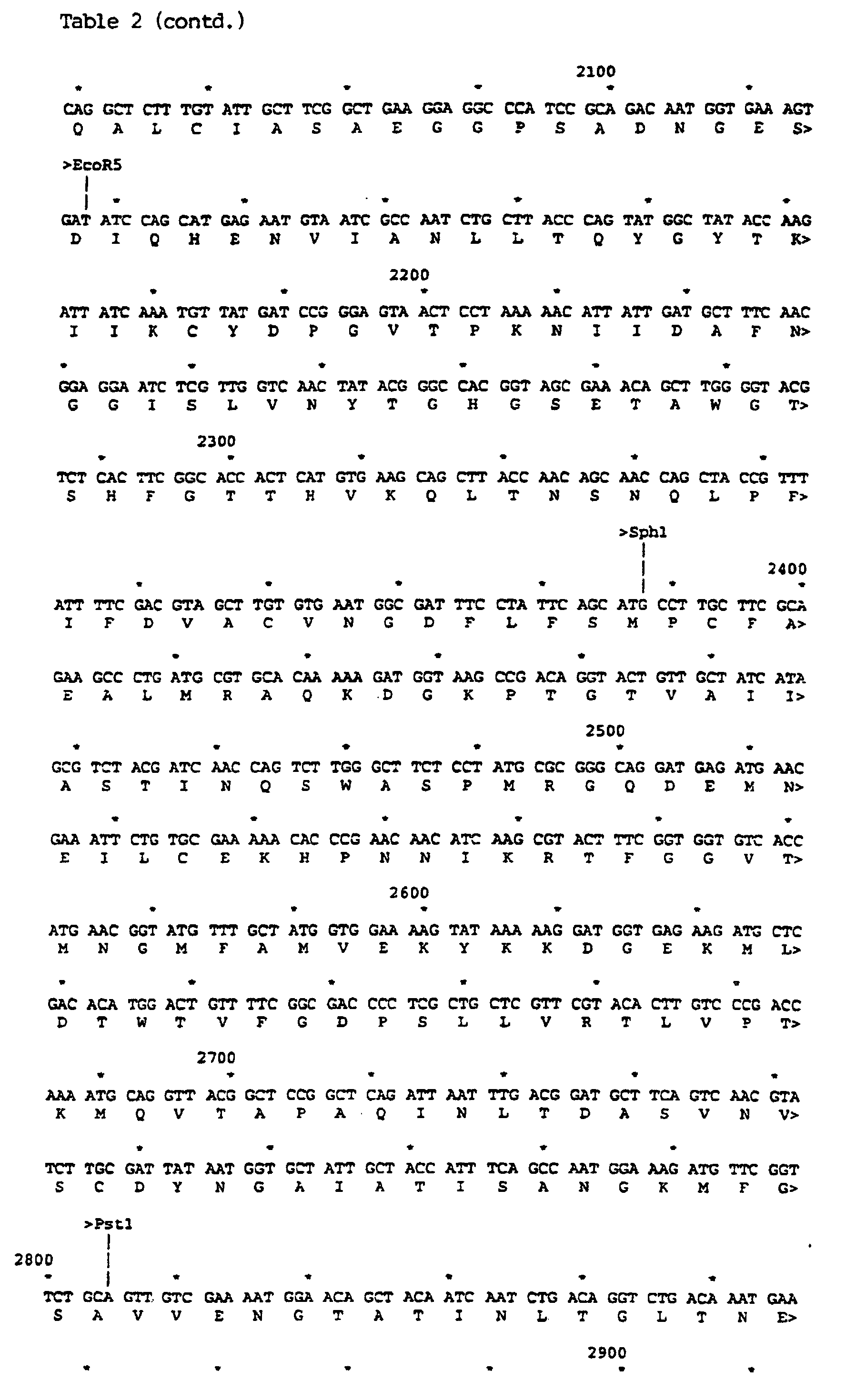

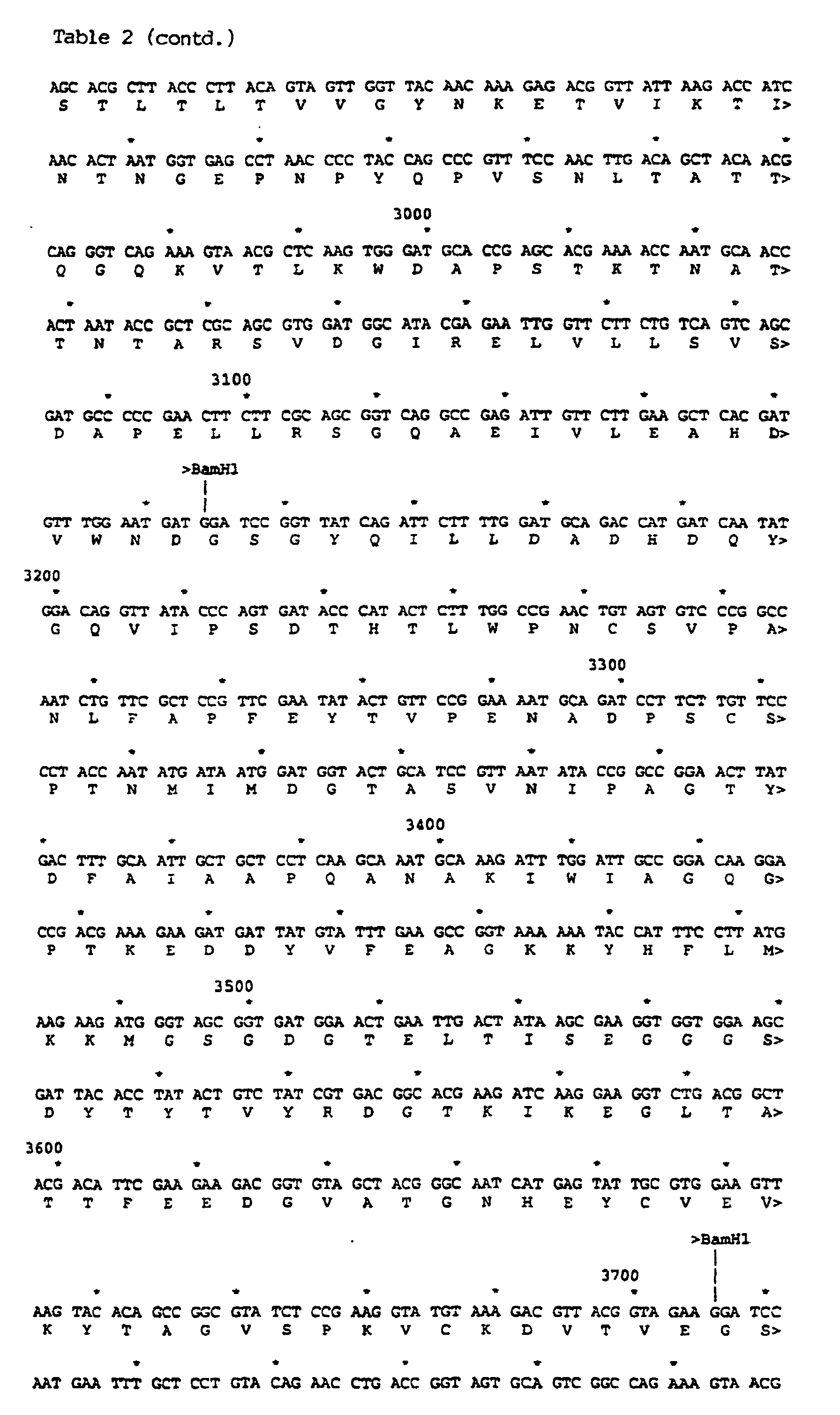

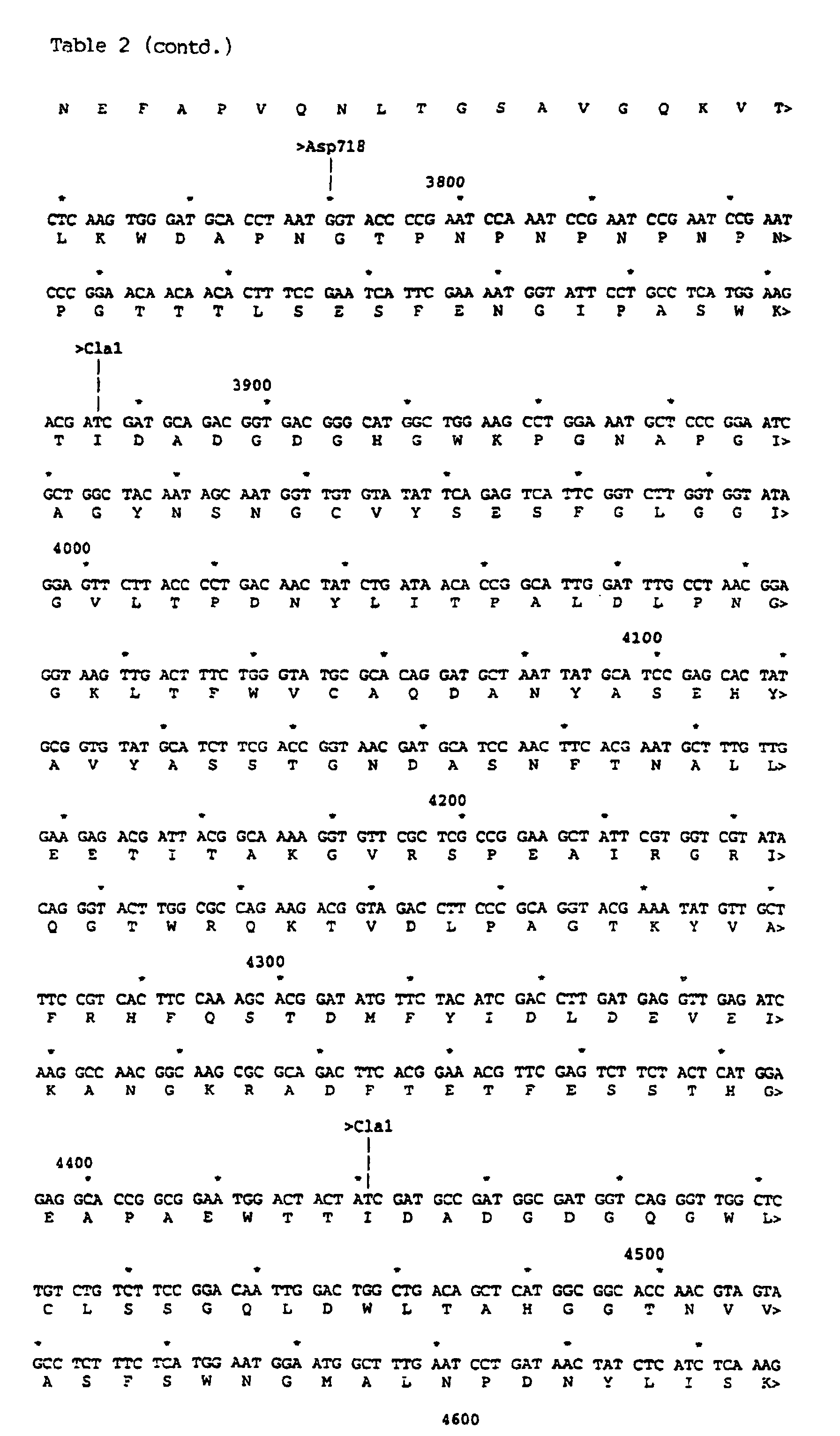

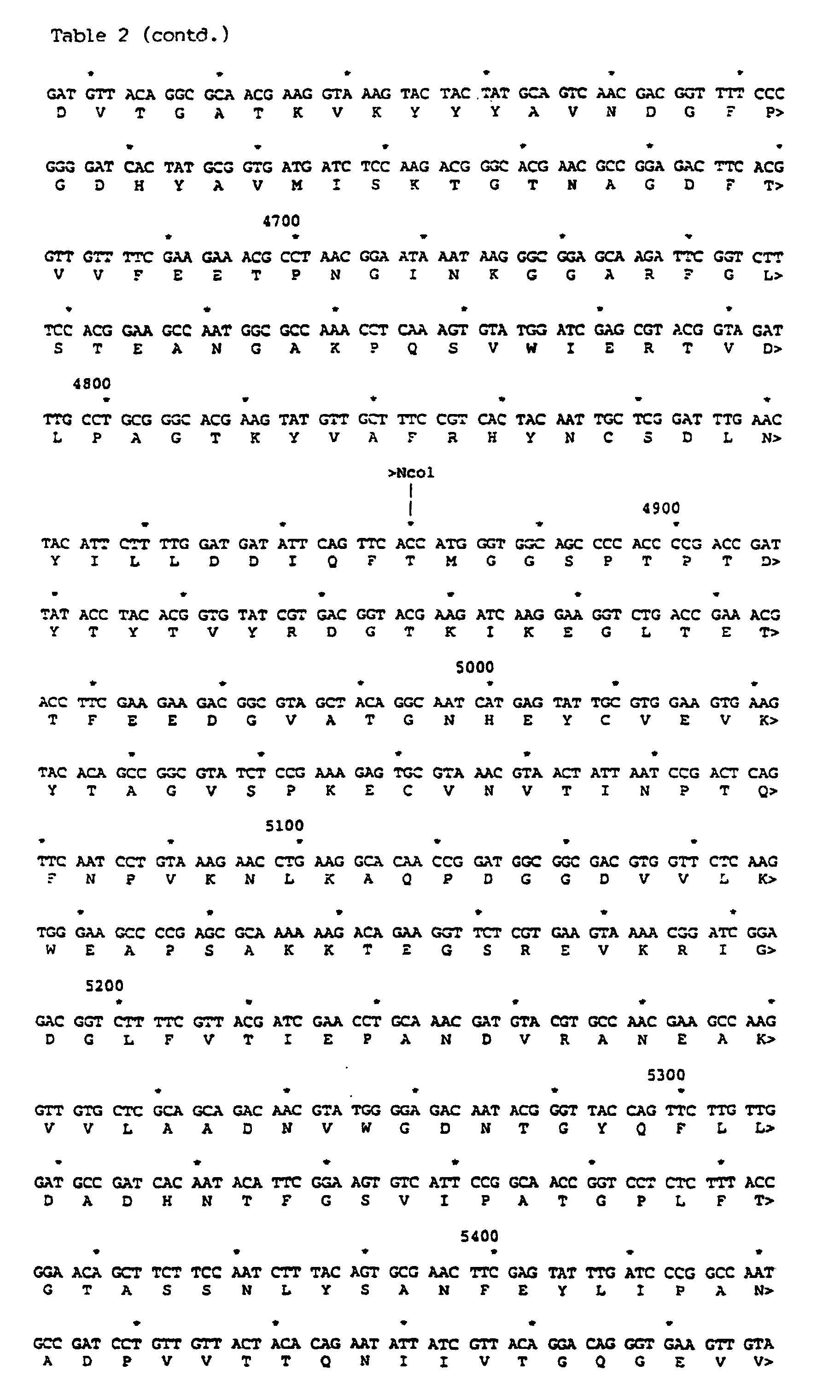

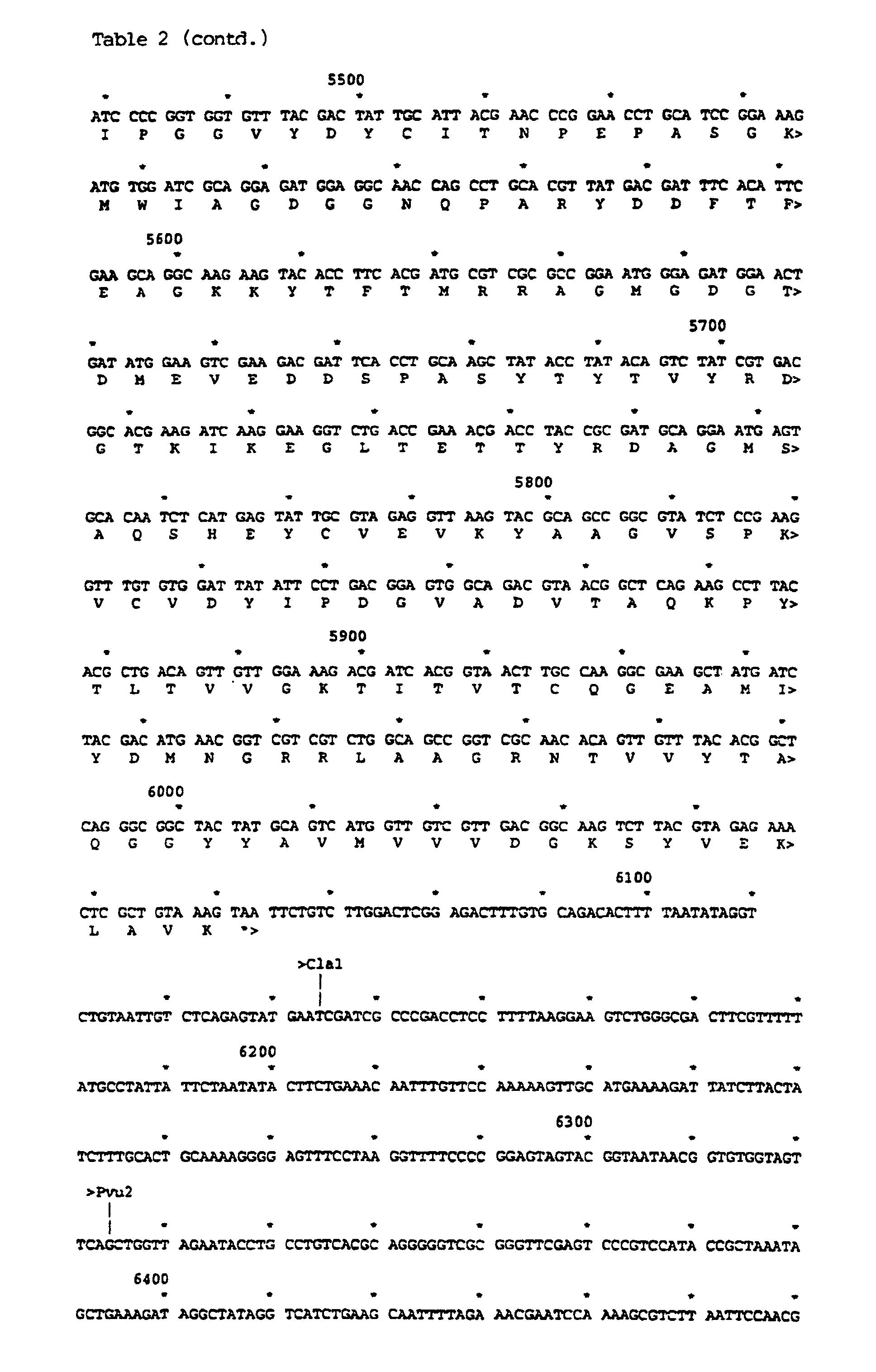

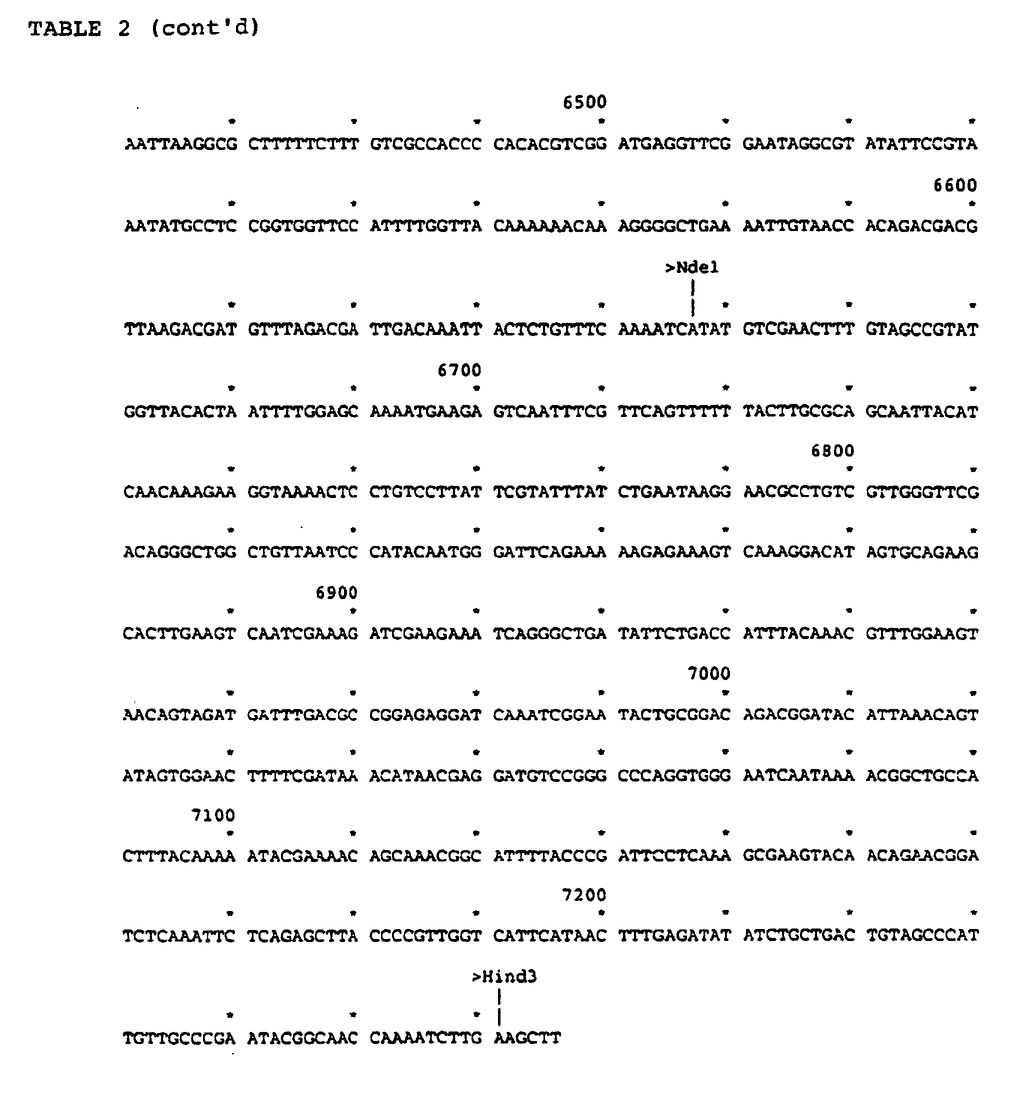

Cleavage of the precursor protein after the Arg residue at amino acid 227 removes the N-terminal precursor portion and after the Arg residue at amino acid 719, 1091 and 1429 releases a low molecular weight Arg-gingipain and three hemagglutinin components. The 44 kDa hemagglutin component has an amino acid sequence as given in SEQ ID NO:11 from 720-1091, with calculated molecular weight of 39.4 kDa, consistent with that estimated by gel electrophoresis. The 17 kDa hemagglutinin component has an amino acid sequence as given in SEQ ID NO:11 at amino acids 1092-1429, and a calculated molecular weight of 37.1 kDa. The 27 kDa hemagglutinin component has an amino acid sequence extending from amino acids 1430-1704 in SEQ ID NO:11, and a calculated molecular weight of 29.6 kDa.

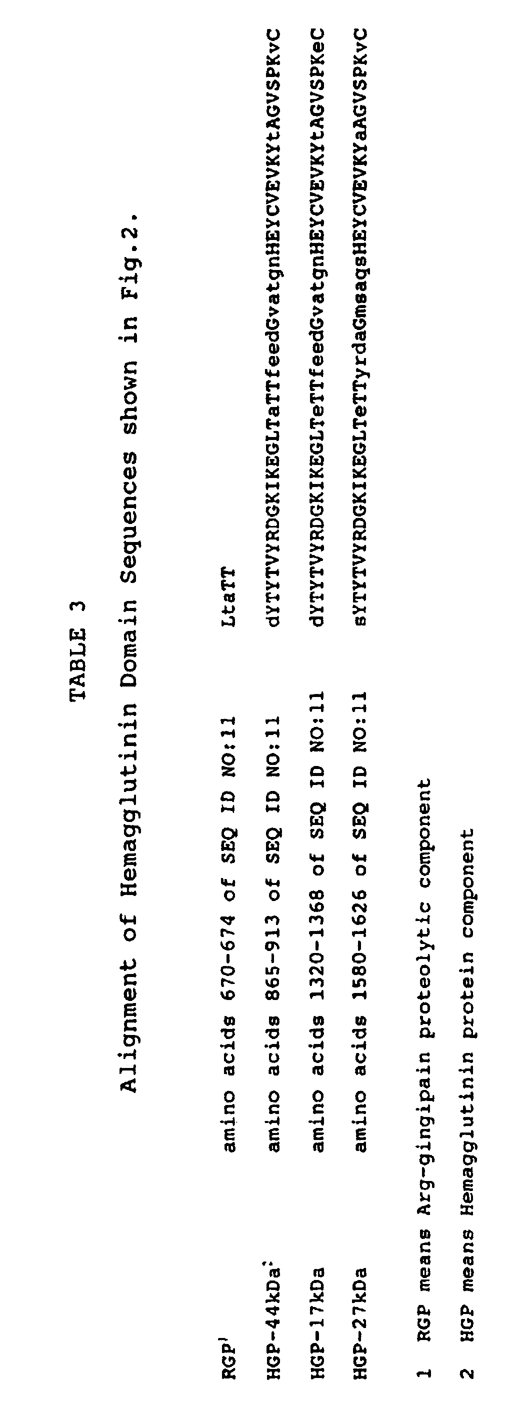

Table 3 is the result of sequence comparison of the 44 kDa, 27 kDa and 17 kDa hemagglutinin domains of Arg-gingipain complexes, alignment of regions of amino acid identity, which without wishing to be bound by any particular theory, are postulated to be the domains responsible for hemagglutinin activity. Identical amino acids among all hemagglutinin domains are in capital letters, and amino acids which are not conserved are shown in lower case letters. In the case of the proteolytic component, only a limited region with significant match is shown.

A genomic DNA library was also prepared from virulent P. gingivalis W50. Two clones were identified as containing Arg-gingipain coding sequence. 0.5 and 3.5 kb *Bam*HI fragments were sequenced; it exhibited 99% nucleotide sequence identity with about 3160 plus 557 bp of P. gingivalis H66 DNA containing Arg-gingipain coding sequence. A comparison of the deduced amino acid sequences of the encoded Arg-gingipain sequences revealed 99% identity.

Tables 1 and 2 both represent sequences from P. gingivalis. However, it is understood that there will be some variations in the amino acid sequences and encoding nucleic acid sequences for Arg-gingipain from different P. gingivalis strains. The ordinary skilled artisan can readily identify and isolate Arg-gingipain-encoding sequences from other strains where there is at least 70% homology to the specifically exemplified sequences herein using the sequences provided herein taken with what is well known to the art. Also within the scope of the present invention are Arg-gingipain where the protease or proteolytic component has at least about 85% amino acid sequence identity with an amino acid sequence exemplified herein.

It is also understood by the skilled artisan that there can be limited numbers of amino acid substitutions in a protein without significantly affecting function, and that nonexemplified gingipain-1 proteins can have some amino acid sequence diversion from the exemplified amino acid sequence. Such naturally occurring variants can be identified, e.g., by hybridization to the exemplified (mature) Arg-gingipain-2 coding sequence (or a portion thereof capable of specific hybridization to Arg-gingipain sequences) under conditions appropriate to detect at least about 70% nucleotide sequence homology, preferably about 80%, more preferably about 90% and most preferably 95-100% sequence homology. Preferably the encoded Arg-gingipain protease or proteolytic component has at least about 85% amino acid sequence identity to an exemplified Arg-gingipain amino acid sequence.

It is well known in the biological arts that certain amino acid substitutions can be made in protein sequences without affecting the function of the protein. Generally, conservative amino acids are tolerated without affecting protein function. Similar amino acids can be those that are similar in size and/or charge properties, for example, aspartate and glutamate and isoleucine and valine are both pairs of similar amino acids. Similarity between amino acid pairs has been assessed in the art in a number of ways. For example, Dayhoff et al. (1978) in *Atlas of Protein Sequence and Structure,* Volume 5, Supplement 3, Chapter 22, pages 345-352, which is incorporated by reference herein, provides frequency tables for amino acid substitutions which can be employed as a measure of amino acid similarity. Dayhoff et al.'s frequency tables are based on comparisons of amino acid sequences for proteins having the same function from a variety of evolutionarily different sources.

The skilled artisan recognizes that other P. gingivalis strains can have coding sequences for a protein with the distinguishing characteristics of an Arg-gingipain; those coding sequences may be identical to or synonymous with the exemplified coding sequence, or there may be some variation(s) in the encoded amino acid sequence. An Arg-gingipain coding sequence from a P. gingivalis strain other than H66 can be identified by, e.g. hybridization to a polynucleotide or an oligonucleotide having the whole or a portion of the exemplified coding sequence for mature gingipain, under stringency conditions appropriate to detect a sequence of at least 70% homology.

A polynucleotide or fragment thereof is "substantially homologous" (or "substantially similar") to another polynucleotide if, when optimally aligned (with appropriate nucleotide insertions or deletions) with another polynucleotide, there is nucleotide sequence identity for approximately 60% of the nucleotide bases, usually approximately 70%, more usually about 80%, preferably about 90%, and more preferably about 95% to 100% of the nucleotide bases.

Alternatively, substantial homology (or similarity) exists when a polynucleotide or fragment thereof will hybridize to another under polynucleotide under selective hybridization conditions. Selectivity of hybridization exists under hybridization conditions which allow one to distinguish the target polynucleotide of interest from other polynucleotides. Typically, selective hybridization will occur when there is approximately 55% similarity over a stretch of about 14 nucleotides, preferably approximately 65%, more preferably approximately 75%, and most preferably approximately 90%. See Kanehisa (1984) *Nuc. Acids Res.,* 12:203-213. The length of homology comparison, as described, may be over longer stretches, and in certain embodiments will often be over a stretch of about 17 to 20 nucleotides, and preferably about 36 or more nucleotides.

The hybridization of polynucleotides is affected by such conditions as salt concentration, temperature, or organic solvents, in addition to the base composition, length of the complementary strands, and the number of nucleotide base mismatches between the hybridizing polynucleotides, as will be readily appreciated by those skilled in the art. Stringent temperature conditions will generally include temperatures in excess of 30°C, typically in excess of 37°C, and preferably in excess of 45°C. Stringent salt conditions will ordinarily be less than 1 M, typically less than 500 mM, and preferably less than 200 mM. However, the combination of parameters is much more important than the measure of any single parameter (Wetmur and Davidson (1968) *J. Mol. Biol.* 31, 349-370).

An "isolated" or "substantially pure" polynucleotide is a polynucleotide which is substantially separated from other polynucleotide sequences which naturally accompany a native gingipain-1 sequence. The term embraces a polynucleotide sequence which has been removed from its naturally occurring environment, and includes recombinant or cloned DNA isolates, chemically synthesized analogues and analogues biologically synthesized by heterologous systems.

A polynucleotide is said to "encode" a polypeptide if, in its native state or when manipulated by methods known to those skilled in the art, it can be transcribed and/or translated to produce the polypeptide of a fragment thereof. The anti-sense strand of such a polynucleotide is also said to encode the sequence.

A nucleotide sequence is operably linked when it is placed into a functional relationship with another nucleotide sequence. For instance, a promoter is operably linked to a coding sequence if the promoter affects its transcription or expression. Generally, operably linked means that the sequences being linked are contiguous and, where necessary to join two protein coding regions, contiguous and in reading frame. However, it is well known that certain genetic elements, such as enhancers, may be operably linked even at a distance, i.e., even if not contiguous.

The term "recombinant" polynucleotide refers to a polynucleotide which is made by the combination of two otherwise separated segments of sequence accomplished by the artificial manipulation of isolated segments of polynucleotides by genetic engineering techniques or by chemical synthesis. In so doing one may join together polynucleotide segments of desired functions to generate a desired combination of functions.

Polynucleotide probes include an isolated polynucleotide attached to a label or reporter molecule and may be used to identify and isolate other Arg-gingipain coding sequences. Probes comprising synthetic oligonucleotides or other polynucleotides may be derived from naturally occurring or recombinant single or double stranded nucleic acids or be chemically synthesized. Polynucleotide probes may be labelled by any of the methods known in the art, e.g., random hexamer labeling, nick translation, or the Klenow fill-in reaction.

Large amounts of the polynucleotides may be produced by replication in a suitable host cell. Natural or synthetic DNA fragments coding for a proteinase or a fragment thereof will be incorporated into recombinant polynucleotide constructs, typically DNA constructs, capable of introduction into and replication in a prokaryotic or eukaryotic cell. Usually the construct will be suitable for replication in a unicellular host, such as yeast or bacteria, but a multicellular eukaryotic host may also be appropriate, with or without integration within the genome of the host cells. Commonly used prokaryotic hosts include strains of Escherichia coli, although other prokaryotes, such as Bacillus subtilis or Pseudomonas may also be used. Mammalian or other eukaryotic host cells include yeast, filamentous fungi, plant, insect, amphibian and avian species. Such factors as ease of manipulation, ability to appropriately glycosylate expressed proteins, degree and control of protein expression, ease of purification of expressed proteins away from cellular contaminants or other factors may determine the choice of the host cell.

The polynucleotides may also be produced by chemical synthesis, e.g., by the phosphoramidite method described by Beaucage and Caruthers (1981) *Tetra. Letts.,* 22: 1859-1862 or the triester method according to Matteuci et al. (1981) *J. Am. Chem.* *Soc.,* 103: 3185, and may be performed on commercial automated oligonucleotide synthesizers. A double-stranded fragment may be obtained from the single stranded product of chemical synthesis either by synthesizing the complementary strand and annealing the strand together under appropriate conditions or by adding the complementary strand using DNA polymerase with an appropriate primer sequence.

DNA constructs prepared for introduction into a prokaryotic or eukaryotic host will typically comprise a replication system (i.e. vector) recognized by the host, including the intended DNA fragment encoding the desired polypeptide, and will preferably also include transcription and translational initiation regulatory sequences operably linked to the polypeptide-encoding segment. Expression systems (expression vectors) may include, for example, an origin of replication or autonomously replicating sequence (ARS) and expression control sequences, a promoter, an enhancer and necessary processing information sites, such as ribosome-binding sites, RNA splice sites, polyadenylation sites, transcriptional terminator sequences, and mRNA stabilizing sequences. Signal peptides may also be included where appropriate from secreted polypeptides of the same or related species, which allow the protein to cross and/or lodge in cell membranes or be secreted from the cell.

An appropriate promoter and other necessary vector sequences will be selected so as to be functional in the host. Examples of workable combinations of cell lines and expression vectors are described in Sambrook et al. (1989) vide infra; Ausubel et al. (Eds.) (1987) Current Protocols in Molecular Biology, Greene Publishing and Wiley Interscience, New York; and Metzger et al. (1988) *Nature,* 334: 31-36. Many useful vectors for expression in bacteria, yeast, mammalian, insect, plant or other cells are well known in the art and may be obtained such vendors as Stratagene, New England Biolabs, Promega Biotech, and others. In addition, the construct may be joined to an amplifiable gene (e.g., DHFR) so that multiple copies of the gene may be made. For appropriate enhancer and other expression control sequences, see also Enhancers and eukaryotic Gene Expression. Cold Spring Harbor Press, N.Y. (1983). While such expression vectors may replicate autonomously, they may less preferably replicate by being inserted into the genome of the host cell.

Expression and cloning vectors will likely contain a selectable marker, that is, a gene encoding a protein necessary for the survival or growth of a host cell transformed with the vector. Although such a marker gene may be carried on another polynucleotide sequence co-introduced into the host cell, it is most often contained on the cloning vector. Only those host cells into which the marker gene has been introduced will survive and/or grow under selective conditions. Typical selection genes encode proteins that (a) confer resistance to antibiotics or other toxic substances, e.g., ampicillin, neomycin, methotrexate, etc.; (b) complement auxotrophic deficiencies; or (c) supply critical nutrients not available from complex media. The choice of the proper selectable marker will depend on the host cell; appropriate markers for different hosts are known in the art.

The recombinant vectors containing the Arg-gingipain coding sequences of interest can be introduced (transformed, transfected) into the host cell by any of a number of appropriate means, including electroporation; transformation or transfection employing calcium chloride, rubidium chloride, calcium phosphate, DEAE-dextran, or other substances; microprojectile bombardment; lipofection; and transfection or infection (where the vector is an infectious agent, such as a viral or retroviral genome). The choice of such means will often depend on the host cell. Large quantities of the polynucleotides and polypeptides of the present invention may be prepared by transforming suitable prokaryotic or eukaryotic host cells with gingipain-1-encoding polynucleotides of the present invention in compatible vectors or other expression vehicles and culturing such transformed host cells under conditions suitable to attain expression of the Arggingipain-encoding gene. The Arg-gingipain may then be recovered from the host cell and purified.

The coding sequence for the "mature" form of Arg-gingipain-2 is expressed after PCR site-directed mutagenesis and cloning into an expression vector suitable for use in E. coli, for example. Exemplary expression vectors for E. coli and other host cells are given, for example in Sambrook et al. (1989), vide infra, and in Pouwels et al. (Eds.) (1986) Cloning Vectors, Elsevier Science Publishers, Amsterdam, the Netherlands.

In order to eliminate leader sequences and precursor sequences at the 5' side of the coding sequence, a combination of restriction endonuclease cutting and site-directed mutagenesis via PCR using an oligonucleotide containing a desired restriction site for cloning (one not present in coding sequence), a ribosome binding site, an translation initiation codon (ATG) and the codons for the first amino acids of the mature Arg-gingipain-2. The oligonucleotide for site-directed mutagenesis at the 3' end of the coding sequence for mature gingipain-1 includes nucleotides encoding the carboxyterminal amino acids of mature gingipain-1, a translation termination codon (TAA, TGA or TAG), and a second suitable restriction endonuclease recognition site not present in the remainder of the DNA sequence to be inserted into the expression vector. The site-directed mutagenesis strategy is similar to that of Boone et al. (1990) *Proc. Natl*. *Acad. Sci. USA* 87: 2800-2804, as modified for use with PCR.

In another embodiment, polyclonal and/or monoclonal antibodies capable of specifically binding to a proteinase or fragments thereof are provided. The term antibody is used to refer both to a homogenous molecular entity, or a mixture such as a serum product made up of a plurality of different molecular entities. Monoclonal or polyclonal antibodies specifically reacting with the Arg-gingipains may be made by methods known in the art. See, e.g., Harlow and Lane (1988) Antibodies: A Laboratory Manual, Cold Spring Harbor Laboratories; Goding (1986) Monoclonal Antibodies: Principles and Practice, 2d ed., Academic Press, New York; and Ausubel et al. (1987) supra. Also, recombinant immunoglobulins may be produced by methods known in the art, including but not limited to the methods described in U.S. Patent No. 4,816,567. Monoclonal antibodies with affinities of 10⁸ M⁻¹, preferably 10⁹ to 10¹⁰ or more are preferred.

Antibodies specific for Arg-gingipains may be useful, for example, as probes for screening DNA expression libraries or for detecting the presence of Arg-gingipains in a test sample. Frequently, the polypeptides and antibodies will be labeled by joining, either covalently or noncovalently, a substance which provides a detectable signal. Suitable labels include but are not limited to radionuclides, enzymes, substrates, cofactors, inhibitors, fluorescent agents, chemiluminescent agents, magnetic particles and the like. United States Patents describing the use of such labels include but are not limited to Nos. 3,817,837; 3,850,752; 3,939,350; 3,996,345; 4,277,437; 4,275,149; and 4,366,241.

Antibodies specific for Arg-gingipain(s) and capable of inhibiting its proteinase activity may be useful in treating animals, including man, suffering from periodontal disease. Such antibodies can be obtained by the methods described above and subsequently screening the Arg-gingipain-specific antibodies for their ability to inhibit proteinase activity.

Compositions and immunogenic preparations including vaccine compositions comprising substantially purified recombinant Arggingipain(s) and a suitable carrier therefor are provided. Alternatively, hydrophilic regions of the proteolytic component or hemagglutinin component(s) of Arg-gingipain can be identified by the skilled artisan, and peptide antigens can be synthesized and conjugated to a suitable carrier protein (e.g., bovine serum albumin or keyhole limpet hemocyanin) for use in vaccines or in raising antibody specific for Arg-gingipains. Immunogenic compositions are those which result in specific antibody production when injected into a human or an animal. Such vaccines are useful, for example, in immunizing an animal, including humans, against inflammatory response and tissue damage caused by P. gingivalis in periodontal disease. The vaccine preparations comprise an immunogenic amount of one or more Arg-gingipains or an immunogenic fragment(s) or subunit(s) thereof. Such vaccines may comprise one or more Arg-gingipain proteinases, or in combination with another protein or other immunogen. By "immunogenic amount" is meant an amount capable of eliciting the production of antibodies directed against Arg-gingipain(s) in an individual to which the vaccine has been administered.

Immunogenic carriers may be used to enhance the immunogenicity of the proteinases. Such carriers include but are not limited to proteins and polysaccharides, liposomes, and bacterial cells and membranes. Protein carriers may be joined to the proteinases to form fusion proteins by recombinant or synthetic means or by chemical coupling. Useful carriers and means of coupling such carriers to polypeptide antigens are known in the art.

The vaccines may be formulated by any of the means known in the art. Such vaccines are typically prepared as injectables, either as liquid solutions or suspensions. Solid forms suitable for solution in, or suspension in, liquid prior to injection may also be prepared. The preparation may also, for example, be emulsified, or the protein encapsulated in liposomes.

The active immunogenic ingredients are often mixed with excipients or carriers which are pharmaceutically acceptable and compatible with the active ingredient. Suitable excipients include but are not limited to water, saline, dextrose, glycerol, ethanol, or the like and combinations thereof. The concentration of the immunogenic polypeptide in injectable formulations is usually in the range of 0.2 to 5 mg/ml.

In addition, if desired, the vaccines may contain minor amounts of auxiliary substances such as wetting or emulsifying agents, pH buffering agents, and/or adjuvants which enhance the effectiveness of the vaccine. Examples of adjuvants which may be effective include but are not limited to: aluminum hydroxide; N-acetyl-muramyl-L-threonyl-D-isoglutamine (thr-MDP); N-acetyl-nor-muramyl-L-alanyl-D-isoglutamine (CGP 11637, referred to as nor-MDP); N-acetylmuramyl-L-alanyl-D-isoglutaminyl-L-alanine-2-(1'-2'-dipalmitoyl-sn-glycero-3hydroxyphosphorylOxy)-ethylamine (CGP 19835A, referred to as MTP-PE); and RIBI, which contains three components extracted from bacteria, monophosphoryl lipid A, trehalose dimycolate and cell wall skeleton (MPL+TDM+CWS) in a 2% squalene/Tween 80 emulsion. The effectiveness of an adjuvant may be determined by measuring the amount of antibodies directed against the immunogen resulting from administration of the immunogen in vaccines which are also comprised of the various adjuvants. Such additional formulations and modes of administration as are known in the art may also be used.

50 kDa Arg-gingipain or high molecular weight Arg-gingipain and fragments thereof may be formulated into vaccines as neutral or salt forms. Pharmaceutically acceptable salts include but are not limited to the acid addition salts (formed with free amino groups of the peptide) which are formed with inorganic acids, e.g., hydrochloric acid or phosphoric acids; and organic acids, e.g., acetic, oxalic, tartaric, or maleic acid. Salts formed with the free carboxyl groups may also be derived from inorganic bases, e.g., sodium, potassium, ammonium, calcium, or ferric hydroxides, and organic bases, e.g., isopropylamine, trimethylamine, 2-ethylamino-ethanol, histidine, and procaine.

The vaccines are administered in a manner compatible with the dosage formulation, and in such amount as will be prophylactically and/or therapeutically effective. The quantity to be administered, which is generally in the range of about 100 to 1,000 µg of protein per dose, more generally in the range of about 5 to 500 µg of protein per dose, depends on the subject to be treated, the capacity of the individual's immune system to synthesize antibodies, and the degree of protection desired. Precise amounts of the active ingredient required to be administered may depend on the judgment of the physician or doctor of dental medicine and may be peculiar to each individual, but such a determination is within the skill of such a practitioner.

The vaccine or other immunogenic composition may be given in a single dose or multiple dose schedule. A multiple dose schedule is one in which a primary course of vaccination may include 1 to 10 or more separate doses, followed by other doses administered at subsequent time intervals as required to maintain and or reinforce the immune response, e.g., at 1 to 4 months for a second dose, and if needed, a subsequent dose(s) after several months.

Recombinant Arg-gingipains are useful in methods of identifying agents that modulate proteinase activity, e.g., by acting on the proteinase itself. One such method comprises the steps of incubating Arg-gingipain-1 (or high molecular weight Arg-proteinase) with a putative therapeutic agent; determining the activity of the proteinase incubated with the agent; and comparing the activity obtained in step with the activity of a control sample of proteinase that has not been incubated with the agent.

All references cited herein are hereby incorporated by reference in their entirety.

Except as noted hereafter, standard techniques for cloning, DNA isolation, amplification and purification, for enzymatic reactions involving DNA ligase, DNA polymerase, restriction endonucleases and the like, and various separation techniques are those known and commonly employed by those skilled in the art. A number of standard techniques are described in Sambrook et al. (1989) Molecular Cloning, Second Edition, Cold Spring Harbor Laboratory, Plainview, New York; Maniatis et al. (1982) Molecular Cloning, Cold Spring Harbor Laboratory, Plainview, New York; Wu (ed.) (1993) Meth. Enzymol. 218, Part I; Wu (ed.) (1979) *Meth Enzymol.* 68; Wu et al. (eds.) (1983) *Meth. Enzymol.* 100 and 101; Grossman and Moldave (eds.) *Meth. Enzymol.* 65; Miller (ed.) (1972) Experiments in Molecular Genetics, Cold spring Harbor Laboratory, Cold Spring Harbor, New York, Old Primrose (1981) Principles of Gene Manipulation, University of California Press, Berkeley; Schleif and Wensink (1982) Practical Methods in Molecular Biology; Glover (ed.) (1985) DNA Cloning Vol. I and II, IRL Press, Oxford, UK; Hames and Higgins (eds.) (1985) Nucleic Acid Hybridization, IRL Press, Oxford, UK; Setlow and Hollaender (1979) Genetic Engineering: Principles and Methods, Vols. 1-4, Plenum Press, New York. Abbreviations and nomenclature, where employed, are deemed standard in the field and commonly used in professional journals such as those cited herein.

The foregoing discussion and the following examples illustrate but are not intended to limit the invention. The skilled artisan will understand that alternative methods may be used to implement the invention.

### EXAMPLES

### Example 1 Purification of Gingipain Enzymes

### Example 1.1 Bacterial Cultivation

P. gingivalis strains H66 (ATCC 33277) and W50 (ATCC 53978) (virulent) were used in these studies. Cells were grown in 500 ml of broth containing 15.0 g Trypticase Soy Broth (Difco, Detroit, Michigan), 2.5 g yeast extract, 2.5 mg hemin, 0.25 g cysteine, 0.05 g dithiothreitol, 0.5 mg menadione (all from Sigma Chemical Company, St. Louis, MO) anaerobically at 37°C for 48 hr in an atmosphere of 85% N₂, 10% CO₂, 5% H₂. The entire 500 ml culture was used to inoculate 20 liters of the same medium, and the latter was incubated in a fermentation tank at 37°C for 48 hr (to a final optical density of 1.8 at 650 nm).

### Example 1.2 Purification of Low Molecular Weight Arg-gingipain

1200 ml cell-free supernatant was obtained from the 48 hr culture by centrifugation at 18,000 x g for 30 min. at 4°C. Proteins in the supernatant were precipitated out by 90% saturation with ammonium sulfate. After 2 hr at 4°C, the suspension was centrifuged at 18,000 x g for 30 min. The resulting pellet was dissolved in 0.05 M sodium acetate buffer, pH 4.5, 0.15 NaCl, 5 mM CaCl₂; the solution was dialyzed against the same buffer overnight at 4°C, with three changes with a buffer:protein solution larger than 150:1. The dialysate was then centrifuged at 25,000 x g for 30 min., and the dark brown supernatant (26 ml) was then chromatographed over an agarose gel filtration column (5.0 x 150 cm; Sephadex G-150, Pharmacia, Piscataway, NJ) which had been pre-equilibrated with the same buffer. The column was developed with said buffer at a flow rate of 36 ml/hr. 6 ml fractions were collected and assayed for both amidolytic and proteolytic activities, using Bz-L-Arg-pNA and azocasein as substrates. Four peaks containing amidolytic activity were identified (Fig. 1). The fractions corresponding to peak 4 were combined, concentrated by ultrafiltration (Amicon PM-10 membrane; Amicon, Beverly, MA )and then dialyzed overnight against 0.05 Bis-Tris, 5 mM CaCl₂, pH 6.0. The volume of the dialysate was 14 ml.

The 14 ml dialysate from the previous step was then applied to a DEAE-cellulose (Whatman, Maidstone, England) column (1 x 10 cm) equilibrated with 0.05 mM Bis-Tris, 5 mM CaCl₂, pH 6.0. The column was then washed with an additional 100 ml of the same buffer. About 75% of the amidolytic activity, but only about 50% of the protein, passed through the column. The column wash fluid was dialyzed against 0.05 M sodium acetate buffer containing 5 mM CaCl₂ (pH 4.5). This 19 ml dialysate was applied to a Mono S FPLC column (Pharmacia LKB Biotechnology Inc., Piscataway, NJ) equilibrated with the same buffer. The column was washed with the starting buffer at a flow rate of 1.0 ml/min for 20 min. Bound proteins were eluted first with a linear NaCl gradient (0 to 0.1 M) followed by a second linear NaCl gradient (0.1 to 0.25 M), each gradient applied over a 25 min time period. Fractions were assayed for amidolytic activity using Bz-L-Arg-pNA. Fractions with activity were pooled and re-chromatographed using the same conditions. Although not detectable by gel electrophoresis, trace contamination by a proteinase capable of cleaving after lysyl residues was sometimes observed. This contaminating activity was readily removed by applying the sample to an arginyl-agarose column (L-Arginyl-SEPHAROSE 4B) equilibrated with 0.025 M Tris-HCl, 5 mM CaCl₂, 0.15 M NaCl, pH 7.5. After washing with the same buffer, purified enzyme was eluted with 0.05 M sodium acetate buffer, 5 mM CaCl₂, pH 4.5. Yields of gingipain-1 were markedly reduced by this step (about 60%).

### Example 1.3 High Molecular Weight Arg-gingipain Purification

The culture supernatant (2,900 ml) was obtained by centrifugation of the whole culture (6,000 x g, 30 min, 4°C). Chilled acetone (4,350 ml) was added to this fraction over a period of 15 min, with the temperature of the solution maintained below 0°C at all times, using an ice/salt bath and this mixture was centrifuged (6,000 x g, 30 min, -15°C). The precipitate was dissolved in 290 ml of 20 mM Bis-Tris-HCl, 150 mM NaCl, 5 mM CaCl₂, 0.02% (w/v) NaN₃, pH 6.8 (Buffer A), and dialyzed against Buffer A containing 1.5 mM 4,4'-Dithiodipyridine disulfide for 4h, followed by 2 changes of buffer A overnight. The dialyzed fraction was centrifuged (27,000 x g, 30 min, 4°C), following which it was concentrated to 40 ml by ultrafiltration using an Amicon PM-10 membrane. This concentrated fraction was applied to a Sephadex G-150 column (5 x 115 cm = 2260 ml; Pharmacia, Piscataway, NJ) which had previously been equilibrated with Buffer A, and the fractionation was carried out at 30 ml/h (1.5 cm/h). Fractions (9 ml) were assayed for activity against Bz-L-Arg-pNa and Z-L-Lys-pNa (Novabiochem; 0.5 mM). Amidolytic activities for Bz-L-Arg-pNa (0.5 mM) or Z-L-Lys-pNa were measured in 0.2 M Tris.Hcl, 1 mM CaCl₂, 0.02% (w/v) NaN₃, 10 mM L-cysteine, pH 7.6. General proteolytic activity was measured with azocasein (2% w/v) as described by Barrett and Kirschke (1981) *Meth.* *Enzymol.* 80, 535-561 for cathepsin L. Three peaks with activity against the two substrates were found. The first (highest molecular weight) peak of activity was pooled, concentrated to 60 ml using ultrafiltration and dialyzed overnight against two changes of 50 mM Tris-HCl, 1 mM CaCl₂, 0.02% NaN₃, pH 7.4 (Buffer B).

This high MW fraction was applied to an L-Arginine-Sepharose column (1.5 x 30 cm = 50 ml), which had previously been equilibrated with Buffer B at a flow rate of 20 ml/hr (11.3 cm/h), following which the column was washed with two column volumes of Buffer B. Following this, a step gradient of 500 mM NaCl was applied in Buffer B and the column was washed with this concentration of NaCl until the A₂₈₀ baseline fell to zero. After re-equilibration of the column in Buffer B, a gradient from 0-750 mM L-Lysine was applied in a total volume of 300 ml, followed by 100 ml of 750 mM L-Lysine. The column was once again reequilibrated with Buffer B and a further gradient to 100 mM L-arginine in 300 ml was applied in the same way. Fractions (6 ml) from the Arg wash were assayed for activity against the two substrates as described previously. The arginine gradient eluted a major peak for an enzyme degrading Bz-L-Arg-pNa. The active fractions were pooled and dialyzed against two changes of 20 mM Bis-Tris-HCl, 1 mM CaCl₂, 0.02% (v/w) NaN₃, pH 6.4 (Buffer C) and concentrated down to 10 ml using an Amicon PM-10 membrane.

The concentrate with activity for cleaving Bz-L-Arg-pNa was applied to a Mono Q FPLC column (Pharmacia LKB Biotechnology Inc, Piscataway, NJ) equilibrated in Buffer C, the column was washed with 5 column volumes of Buffer C at 1.0 ml/min, following which bound protein was eluted with a 3 step gradient [0-200 mM NaCl (10 min), followed by 200-250 mM NaCl (15 min) and 250-500 mM NaCl (5 min) ]. The active fractions from Mono Q were pooled and used for further analyses.

### Example 2 Molecular Weight Determination

The molecular weight of the purified Arg-gingipain-1 was estimated by gel filtration on a Superose 12 column (Pharmacia, Piscataway, NJ) and by Tricine-SDS polyacrylamide gel electrophoresis. In the latter case, 1 mM TLCK was used to inactivate the protease prior to boiling, thus preventing autoproteolytic digestion.

### Example 3 Enzyme Assays

Amidolytic activities of P. gingivalis proteinases were measured with the substrates MeO-Suc-Ala-Ala-Pro-Val-pNA at a concentration of 0.5 mM, Suc-Ala-Ala-Ala-pNA (0.5 mM), Suc-Ala-Ala-Pro-Phe-pNA (0.5 mM), Bz-Arg-pNA (1.0 mM), Cbz-Phe-Leu-Glu-pNA) (0.2 mM); S-2238, S-2222, S-2288 and S-2251 each at a concentration of 0.05 mM; in 1.0 ml of 0.2 M Tris-HCl, 5mM CaCl₂, pH 7.5. In some cases either 5 mM cysteine and/or 50 mM glycyl-glycine (Gly-Gly) was also added to the reaction mixture.

For routine assays, pH optimum determination and measurement of the effect of stimulating agents and inhibitors on trypsin-like enzymes, only Bz-L-Arg-pNA was used as substrate. Potential inhibitory or stimulatory compounds were preincubated with enzyme for up to 20 min at room temperature at pH 7.5, in the presence of 5 mM CaCl₂ (except when testing the effects of chelating agents) prior to the assay for enzyme activity.

General proteolytic activity was assayed using the same buffer system as described for detecting amidolytic activity, but using azocoll or azocasein (1% w/v) as substrate.

A unit of Arg-gingipain-1 enzymatic activity is based on the spectroscopic assay using benzoyl-Arg-p-nitroanilide as substrate and recording Δ absorbance units at 405 nm/min/absorbance unit at 280 nm according to the method of Chen et al. (1992) supra.

### Example 4 Enzyme Specificity

Purified Arg-gingipain-1 (0.8 µg) in 50 mM ammonium bicarbonate buffer, pH 7.7, 5 mM CaCl₂, was preincubated with 2 mM cysteine for 10 min, followed by the addition of either oxidized insulin B chain (225 µg) or melittin (225 µg) at 25°C. Samples were removed after various time intervals, and the reaction mixtures were subjected to HPLC (reverse phase column, MicroPak SP C-18 column) using linear gradients (0.08% trifluoroacetic acid to 0.08% trifluoroacetic acid plus 80% acetonitrile, over a 45 min period (flow rate 1.0 ml/min). Peptides were detected by monitoring A₂₂₀. Product peaks were collected and subjected to amino acid analysis and/or amino-terminal sequence analysis.

### Example 5 Amino Acid Sequence Analysis

Amino-terminal amino acid sequence analysis of either Arg-gingipain-1 or degradation products from proteolytic reactions was carried out using an Applied Biosystems 4760A gas-phase sequenator, using the program designed by the manufacturer.

The amino acid sequence of the COOH terminus of SDS-denatured Arg-gingipain-1 and of Arg-gingipain-2 was determined. 10 nmol aliquots of gingipain-1 were digested in 0.2 M N-ethylmorpholine acetate buffer, pH 8.0, with carboxypeptidase A and B at room temperature, using 1:100 and 1:50 molar ratios, respectively. Samples were removed at intervals spanning 0 to 12 hours, boiled to inactivate the carboxypeptidase, and protein was precipitated with 20% trichloracetic acid. Amino acid analysis was performed on the supernatants.

### Example 6 Materials

MeO-Suc-Ala-Ala-Pro-Val-pNA, Suc-Ala-Ala-Pro-Phe-pNA, Gly-Pro-pNA, Suc-Ala-Ala-Ala-pNA, Bz-Arg-pNA, diisopropylfluorophosphate, phenylmethylsulfonyl fluoride, tosyl-L-lysine chloromethyl ketone (TLCK), tosyl-L-phenylalanine chloromethyl ketone (TPCK), trans-epoxysuccinyl-L-leucylamide- (4-guanidino)butane), an inhibitor of cysteine proteinases, leupeptin, antipain and azocasein were obtained from Sigma Chemical Co., St. Louis, MO. 3,4-Dichloroisocoumarin was obtained from Boehringer, Indianapolis, IN and CBz-Phe-Leu-Glu-pNA and azocoll were obtained from Calbiochem, La Jolla, CA. S-2238 (D-Phe-Pip-Arg-pNA), S-2222 (Bz-Ile-Glu-(γ-OR) -Gly-Arg-pNA), S-2288 (D-Ile-Pro-Arg-pNA), and S-2251 (D-Val-Leu-Lys-pNA) were from Kabi-Vitrum, (Beaumont, Texas).

### Example 7 Electrophoresis

SDS-PAGE of Arg-gingipain-1 was performed as in Laemmli (1970) *Nature* 227: 680-685. Prior to electrophoresis the samples were boiled in a buffer containing 20% glycerol, 4% SDS, and 0.1% bromphenol blue. The samples were run under reducing conditions by adding 2% β-mercaptoethanol unless otherwise noted. Samples were heated for 5 min at 100°C prior to loading onto gels. A 5-15% gradient gel was used for the initial digests of C3 and C5, and the gels were subsequently stained with Coomassie Brilliant Blue R. The C5 digest used to visualize breakdown products before and after reduction of the disulfide bonds were electrophoresed in a 8% gel. Attempts to visualize C5a in the C5 digest were carried out using 13% gels that were developed with silver stain according to the method of Merril et al. (1979) *Proc. Natl. Acad. Sci USA* 76*,* 4335-4340.

In some experiments (high molecular weight forms) SDS-PAGE using Tris-HCl/Tricine buffer was carried out per Shagger and Van Jagow (1987) *Analyt. Biochem*. 166, 368-379.

Electrophoresis on cellulose acetate strips were performed in 0.075 barbital buffer at pH 8.5 and 4°C for 30 min. at 200 V. The Beckman Microzone apparatus (model R101). used for the electrophoresis of the protein, and the strips were stained using Amido Black.

### Example 8 Oligonucleotide Synthesis

Oligonucleotide primers for PCR probes and sequencing were synthesized by the phosphoramidite method with an Applied Biosystems model 394 automated DNA synthesizer (Applied Biosystems, Foster City, CA) and purified by PAGE and desalted on Sep-Pak (Millipore Corp., Beverly, MA) using standard protocols. Primer GIN-1-32 was designed to bind to the noncoding strand of Arg-gingipain DNA corresponding to the NH₂-terminal portion of the mature protein, i.e., to the sequence encoding amino acids 2-8 within SEQ ID NO:1. The sequence of the 32-base primer consists of 20 bases specific for Arg-gingipain and six additional bases at the 5' end (underlined), as follows: 5'-GGCTTTACNCCNGTNGARGARYTNGA-3' (SEQ ID NO:6), where N is A or G or C or T. Primer GIN-2-30 was designed to bind to the coding strand of Arg-gingipain DNA corresponding to the amino acids 25-32 of the mature protein, i.e., residues 25-32 of SEQ ID NO:1. The sequence of the 30-base primer consists of 24 bases specific for gingipain-1 (and gingipain-2) DNA and six additional bases at the 5' end (underlined), as follows: 5'-GGCTTTRTTYTTCCARTC NACRAARTCYTT-3', where R is A or G, Y is C or T and N is A or G or C or T (SEQ ID NO:7). Primer GIN-8S-48: 5'-CCTGGAGAATTCTCG TATGATCGTCATCGTAGCCAAAAAGTATGAGGG-3' (SEQ ID NO:8) was designed to bind to the noncoding strand of Arg-gingipain DNA corresponding to the amino acids 11-22 of the mature protein, i.e., amino acids 11-22 of SEQ ID NO:1, and was designed on the basis of partial DNA sequence information for the Arg-gingipain coding sequence (nucleotides 1659-1694 of SEQ ID NO:3) and included a 6-base *Eco*RI restriction site plus six additional bases at the 5' end (underlined). This primer was used as a probe to screen a λDASH P. gingivalis genomic DNA library (see below). One additional oligonucleotide GIN-14-20 (20-mers), initially designed to sequence Arg-gingipain DNA, was used as a probe to identify and then clone the 3' end of the gingipain-1 coding sequence, as a *Pst*I-*Hind*III sequence. Primer GIN-14-20 was designed to bind to the noncoding strand of gingipain-1 DNA corresponding to 20 bases specific for 3' end of Arg-gingipain (nucleotides 2911-2930 within SEQ ID NO:3): 5'-ATCAACACTAATGGTGAGCC-3' (SEQ ID NO:9). A total of 71 20-mers internal primers were designed using empirically determined sequence to sequence the Arg-gingipain locus.

### Example 9 Polymerase Chain Reaction

The DNA templates used in PCR was P. gingivalis strain H66 total cellular DNA. The PCR was run using primer GIN-1-32 (SEQ ID NO:6) along with primer GIN-2-30 (SEQ ID NO:7); PCR consistently yielded a single 105-base pair product (P105) detected on a 7% acrylamide gel representing a partial gingipain DNA. After treatment with the Klenow enzyme, P105 was cloned in pCR-Script^{TM}SK(+) (Stratagene, La Jolla, CA). After sequence analysis of P105, specific primer GIN-8S-48 (SEQ ID NO:8) was designed to use as a probe. The ³²P-labeled GIN-8S-48 probe, was generated by kinase reaction for use in subsequent hybridization screening of the λDASH library. Incorporated nucleotides were separated from unincorporated nucleotides on a Sephadex G-25 column (Boehringer Mannheim Corporation, Indianapolis, IN).

### Example 10 Construction and Screening of the genomic DNA library

λDASH and λZAP DNA libraries were constructed according to the protocols of Stratagene, using the lambda DASH^{TM} II/BamHI cloning kit and DNA preparations from P. gingivalis strains H66 and W50. Libraries of 3x10⁵ independent recombinant clones was obtained using P. gingivalis H66 DNA, and 1.5x10⁵ independent recombinant clones were obtained from P. gingivalis W50 DNA.

Approximately 3x10⁵ phages were grown on 5x150 mm agar plates, lifted in duplicate onto supported nitrocellulose transfer membrane (BAS-NC, Schleicher & Schuell, Keene, NH), hybridized to the ³²P-labeled GIN-8S-48 probe described above. Hybridizations were performed overnight at 42°C in 2X Denhardt's solution (Denhardt, D.T. (1966), *Biochem. Biophys. Res. Comm.* 23, 641-646), 6X SSC (SSC is 15 mM sodium citrate, 150 mM NaCl), 0.4% SDS (w/v), 500 µg/ml fish sperm DNA. The filters were washed in 2X SSC containing 0.05% SDS (w/v) at 48°C. Seven positively hybridizing plaques were purified. After extraction and purification, the DNA was analyzed by restriction enzyme digestion and agarose gel electrophoresis. The 3 kb-*Pst*I fragment from clone A1 (P. gingivalis H66) was subsequently cloned into pBluescript SK(-) (Stratagene, La Jolla, CA) and M13mp18 and 19 and sequenced. After restriction analysis of the A1 clone, a *Sma*I/*Bam*HI fragment was then cloned into pBluescript SK(-). A *Pst*I/*Bam*HI smaller fragment was subcloned into M13mp18 and 19 for sequencing purposes. 3.5 and 0.5 kb-*Bam*HI fragments from the λZAP P. gingivalis W50 DNA library were cloned into pBluescript SK(-) and M13mp18 and 19 and sequenced. Standard protocols for cDNA library screening, lambda phage purification, agarose gel electrophoresis and plasmid cloning were employed (Maniatis et al. (1982), supra). Standard protocols for cDNA library screening, lambda phage purification, agarose gel electrophoresis and plasmid cloning were employed (Maniatis et al., 1982 supra).

### Example 11 Southern Blot Analysis

The membranes were washed as described above. BamHI, HindIII- or PstI-digested P. gingivalis H66 DNA samples were hybridized with ³²P-labeled GIN-8S-48. Two *Bam*HI fragments of approximately 9.4 and 3.5 kb, and two *Pst*I fragments of approximately 9.4 and 3 kb were found. No *Hind*III fragment was seen. *Bam*HI- and *Pst*I-digested λDASH DNA after screening and purification of positive recombinant clones from the library revealed one clone (A1) with a 3.5 kb *Bam*HI fragment and a 3 kb *Pst*I fragment; one clone (B1) with a 9.4 kb *Bam*HI fragment and a 9.4 kb *Pst*I fragment; and 5 clones with a 9.4 kb *Bam*HI fragment and a 10 kb *Pst*I fragment. The A1 clone was sequenced because the DNA predicted to encode a 50-kDa protein is approximately 1.35 kb. In order to clone the stop codon of Arg-gingipain-2, double *Pst*I/*Hind*III-digested P. gingivalis DNA were hybridized with ³²P-labeled GIN-14-20. One *Pst*I/*Hind*III fragment of approximately 4.3 kb was found. This fragment was gel purified and cloned into pBluescript SK(-) for sequencing. Smaller fragments (*Pst*I/*Sma*I and *Bam*HI/*Hind*III) were also subcloned into M13mp18 and 19 and sequenced, and was found to include the stop codon. Table 2 hereinabove (see also SEQ ID NO:10) which presents about 7 kb of sequence extending from a *Pst*I site upstream of the start codon through a *Hind*III site downstream of the end of the prepolyprotein's stop codon.

### Example 12 DNA Sequencing

Double-stranded DNA cloned into pBluescript SK(-) and single-stranded DNA cloned into M13mp18 and 19 were sequenced by the dideoxy terminator method [Sanger et al. (1977) *Proc. Natl. Acad. Sci. USA* 74, 5463-5467] using sequencing kits purchased from United States Biochemicals (Cleveland, OH; Sequenase version 2.0). The DNA was sequenced using M13 universal primer, reverse sequencing primer and internal primers as well understood in the art.

## Claims

1. A recombinant DNA molecule comprising a nucleotide sequence encoding an Arg-gingipain protein having an enzymatically active protease component and at least one hemagglutinin component, the at least one hemagglutinin component comprising an amino acid sequence selected from the group consisting of amino acid 720 to amino acid 1091, amino acid 1092 to amino acid 1429 and amino acid 1430 to amino acid 1704, as given in SEQ ID NO:11.

2. The recombinant DNA molecule of claim 1 wherein the nucleotide sequence encoding the at least one hemagglutinin component is selected from the group consisting of nucleotide 3106 to nucleotide 4221, nucleotide 4222 to nucleotide 5235, and nucleotide 5236 to nucleotide 6160, as given in SEQ ID NO:10.

3. The recombinant DNA molecule of claim 1 wherein the amino acid sequence of the enzymatically active protease component comprises an amino acid sequence selected from the group consisting of amino acid 1 to amino acid 510 as given in SEQ ID NO:4, amino acid 228 to amino acid 719 as given in SEQ ID NO:11, and an amino acid sequence having at least 85% amino acid sequence identity with amino acid 228 to amino acid 719 as given in SEQ ID NO:11.

4. The recombinant DNA molecule of claim 3 wherein the nucleotide sequence encoding the enzymatically active protease component is as given in one of SEQ ID NO:3 from nucleotide 1630 to nucleotide 3105 and SEQ ID NO:10 from nucleotide 1630 to nucleotide 3105, or a nucleotide sequence having at least 70% homology to one of said sequences.

5. The recombinant DNA molecule of claim 1 wherein said Arg-gingipain is encoded within a sequence of nucleotide 949 to nucleotide 6063 as given in SEQ ID NO:10, or a nucleotide sequence having at least 70% sequence homology thereto.

6. The recombinant DNA molecule of claim 5 wherein said Arg-gingipain is expressed as a prepolyprotein having an amino acid sequence as given in SEQ ID NO:11.

7. The recombinant DNA molecule of claim 6 wherein the nucleotide sequence encoding said polyprotein is nucleotide 949 to nucleotide 6063 as given in SEQ ID NO:10.

8. A pure P.gingivalis high molecular weight arginine-specific preparation comprising an Arg-gingipain protein, the Arg-gingipain protein comprising an amino acid sequence selected from the group consisting of amino acid 720 to amino acid 1091, amino acid 1092 to amino acid 1429 and amino acid 1430-1704, as given in SEQ ID NO:11

9. The preparation of claim 8 wherein the protease component has an amino acid sequence as given in one of SEQ ID NO:4 from amino acid 1 to amino acid 510 and SEQ ID NO;11 from amino acid 228 to amino acid 719.

10. The preparation of claim 8 wherein the at least one hemagglutinin component has an apparent molecular mass selected from the group consisting of 44 kDa, 27 kDa, and 17 kDa.

11. An immunogenic composition comprising an Arg-gingipain preparation as claimed in claim 8 and a suitable carrier therefor.

12. A composition comprising a Arg-gingipain preparation as claimed in claim 8 and a suitable carrier therefor wherein the Arg-gingipain is recombinant and derived from P. gingivalis.

13. Use of a purified recombinant Arg-gingipain derived from *P. gingivalis* for the manufacture of a vaccine composition for protecting an animal against inflammatory response and tissue damage caused by *P. gingivalis* in periodontal disease.

## Patentansprüche

1. Rekombinantes DNA-Molekül, umfassend eine Nucleotid-Sequenz, die ein Arg-gingipain-Protein mit einer enzymatisch aktiven Protease-Komponente und mindestens eine Hämagglutinin-Komponente kodiert, wobei die mindestens eine Hämagglutinin-Komponente eine Aminosäuresequenz umfaßt, die ausgewählt wird aus der Gruppe bestehend aus Aminosäure 720 bis Aminosäure 1091, Aminosäure 1092 bis Aminosäure 1429 und Aminosäure 1430 bis Aminosäure 1704, wie in SEQ ID NO. 11 angegeben.

2. Rekombinantes DNA-Molekül nach Anspruch 1, wobei die Nucleotid-Sequenz, die die mindestens eine Hämagglutinin-Komponente kodiert, ausgewählt wird aus der Gruppe bestehend aus Nucleotid 3106 bis Nucleotid 4221, Nucleotid 4222 bis Nucleotid 5235 und Nucleotid 5236 bis Nucleotid 6160, wie in SEQ ID NO. 10 angegeben.

3. Rekombinantes DNA-Molekül nach Anspruch 1, wobei die Aminosäuresequenz der enzymatisch aktiven Protease-Komponente eine Aminosäuresequenz umfaßt, die ausgewählt wird aus der Gruppe bestehend aus Aminosäure 1 bis Aminosäure 510, wie in SEQ ID NO. 4 angegeben, Aminosäure 228 bis Aminosäure 719, wie in SEQ ID NO. 11 angegeben, und einer Aminosäuresequenz mit mindestens 85% Aminosäuresequenzidentität mit Aminosäure 228 bis Aminosäure 719, wie in SEQ ID NO. 11 angegeben.

4. Rekombinantes DNA-Molekül nach Anspruch 3, wobei die Nucleotidsequenz, die die enzymatisch aktive Protease-Komponente kodiert, eine Sequenz ist, wie in einer der SEQ ID NO. 3 von Nucleotid 1630 bis Nucleotid 3105 und SEQ ID NO. 10 von Nucleotid 1630 bis Nucleotid 3105 angegeben, oder eine Nucleotidsequenz mit mindestens 70% Homologie zu einer der genannten Sequenzen ist.

5. Rekombinantes DNA-Molekül nach Anspruch 1, wobei das Arg-gingipain innerhalb einer Sequenz von Nucleotid 949 bis Nucleotid 6063, wie in SEQ ID NO. 10 angegeben, oder einer Nucleotidsequenz mit mindestens 70% Sequenzhomologie dazu kodiert wird.

6. Rekombinantes DNA-Molekül nach Anspruch 5, wobei das Arg-gingipain als ein Prepolyprotein mit einer Aminosäuresequenz, wie in SEQ ID NO. 11 angegeben, exprimiert wird.

7. Rekombinantes DNA-Molekül nach Anspruch 6, wobei die Nucleotidsequenz, die das Polyprotein kodiert, Nucleotid 949 bis Nucleotid 6063, wie in SEQ ID NO. 10 angegeben, ist.

8. Eine reine *P. gingivalis* Arginin-spezifische Präparation mit hohem Molekulargewicht, umfassend ein Arg-gingipain-Protein, wobei das Arg-gingipain-Protein eine Aminosäuresequenz, ausgewählt aus der Gruppe bestehend aus Aminosäure 720 bis Aminosäure 1091, Aminosäure 1092 bis Aminosäure 1429 und Aminosäure 1430-1704, wie in SEQ ID NO. 11 angegeben, umfaßt.

9. Präparation nach Anspruch 8, wobei die Protease-Komponente eine Aminosäuresequenz hat, wie in einer der SEQ ID No. 4 von Aminosäure 1 bis Aminosäure 510 und SEQ ID NO. 11 von Aminosäure 228 bis Aminosäure 719 angegeben.

10. Präparation nach Anspruch 8, wobei die mindestens eine Hämagglutinin-Komponente eine scheinbares Molekulargewicht hat, das ausgewählt wird aus der Gruppe bestehend aus 44 kDa, 27 kDa und 17 kDa.

11. Immunisierende Zusammensetzung, umfassend eine Arg-gingipain-Präparation nach Anspruch 8 und einen geeigneten Träger dafür.

12. Zusammensetzung, umfassend eine Arg-gingipain-Präparation nach Anspruch 8 und einen geeigneten Träger dafür, wobei das Arg-gingipain rekombinant ist und von *P. gingivalis* erhalten wird.

13. Verwendung eines gereinigten rekombinanten Arg-gingipain, erhalten von *P. gingivalis,* zur Herstellung einer Impfstoffzusammensetzung zum Schützen eines Tieres gegen entzündliche Reaktion und Gewebeschaden, ausgelöst durch *P. gingivalis* bei einer peridontalen Krankheit.

## Revendications

1. Molécule d'ADN recombinant comprenant une séquence nucléotidique codant pour une protéine Arg-gingipaïne possédant un composant protéase enzymatiquement actif et au moins un composant hémagglutinine, le au moins un composant hémagglutinine comprenant une séquence d'acides aminés choisie au sein du groupe constitué par la séquence d'acides aminés 720 à 1091, la séquence d'acides aminés 1092 à 1429 et la séquence d'acides aminés 1430 à 1704, telles que figurant dans la SEQ ID N°11.

2. Molécule d'ADN recombinant selon la revendication 1, dans laquelle la séquence nucléotidique codant pour le au moins un composant hémagglutinine est choisie au sein du groupe constitué par la séquence de nucléotides 3106 à 4221, la séquence de nucléotides 4222 à 5235 et la séquence de nucléotides 5236 à 6160, telles que figurant dans la SEQ ID N°10.

3. Molécule d'ADN recombinant selon la revendication 1, où la séquence d'acides aminés du composant protéase enzymatiquement actif comprend une séquence d'acides aminés choisie au sein du groupe constitué par la séquence d'acides aminés 1 à 510 telle que figurant dans la SEQ ID N°4, la séquence d'acides aminés 228 à 719 telle que figurant dans la SEQ ID N°11, et une séquence d'acides aminés présentant un degré d'identité d'au moins 85% avec la séquence d'acides aminés 228 à 719 telle que figurant dans la SEQ ID N° 11.

4. Molécule d'ADN recombinant selon la revendication 3, dans laquelle la séquence nucléotidique codant pour le composant protéase enzymatiquement actif est telle que donnée dans l'une des SEQ ID N°3, du nucléotide 1630 au nucléotide 3105, et SEQ ID N°10, du nucléotide 1630 au nucléotide 3105, ou une séquence nucléotidique présentant un degré d'homologie d'au moins 70% avec l'une desdites séquences.

5. Molécule d'ADN recombinant selon la revendication 1, où ladite Arg-gingipaïne est codée au sein d'une séquence du nucléotide 949 au nucléotide 6063 telle que figurant dans la SEQ ID N° 10, ou d'une séquence nucléotidique présentant un degré d'homologie d'au moins 70% avec celle-ci.

6. Molécule d'ADN recombinant selon la revendication 5, où ladite Arg-gingipaïne est exprimée sous la forme d'une prépolyprotéine possédant une séquence d'acides aminés telle que figurant dans la SEQ ID N°11.

7. Molécule d'ADN recombinant selon la revendication 6 dans laquelle la séquence nucléotidique codant pour ladite polyprotéine est la séquence du nucléotide 949 à 6063 telle que figurant dans la SEQ ID N°10.

8. Préparation de P. gingivalis pure d'un poids moléculaire élevé, spécifique de l'arginine, comprenant une protéine Arg-gingipaïne, la protéine Arg-gingipaïne comprenant une séquence d'acides aminés choisie au sein du groupe constitué par la séquence de l'acide aminé 720 à l'acide aminé 1091, la séquence de l'acide aminé 1092 à l'acide aminé 1429 et la séquence de l'acide aminé 1430 à l'acide aminé 1704, telles que figurant dans la SEQ ID n° 11.

9. Préparation selon la revendication 8, dans laquelle le composant protéase possède une séquence d'acides aminés telle que donnée dans la SEQ ID N°4 de l'acide aminé 1 à l'acide aminé 510 et dans la SEQ ID N°11 de l'acide aminé 228 à l'acide aminé 719.

10. Préparation selon la revendication 8, dans laquelle le au moins un composant hémagglutinine possède une masse moléculaire apparente choisie au sein du groupe constitué par les masses moléculaires de 44 kDa, 27 kDa et 17 kDa.

11. Composition immunogène comprenant une préparation d'Arg-gingipaïne selon la revendication 8 et un véhicule approprié pour celle-ci.

12. Composition comprenant une préparation d'Arg-gingipaïne selon la revendication 8 et un véhicule approprié pour celle-ci dans laquelle l'Arg-gingipaïne est recombinante et dérivée de P. gingivalis.

13. Utilisation d'une Arg-gingipaïne recombinante purifiée dérivée de P. gingivalis pour la fabrication d'une composition vaccinale destinée à protéger un animal de la réponse inflammatoire et de l'endommagement des tissus causé par P. gingivalis dans la périodontose.


### SEQUENCE LISTING

(1) GENERAL INFORMATION:
   (i) APPLICANT: UNIVERSITY OF GEORGIA, RESEARCH FOUNDATION INC.
   (ii) TITLE OF INVENTION: Porphyromonas Gingivalis Arginine-Specific Proteinase Coding Sequences
   (iii) NUMBER OF SEQUENCES: 11
   (iv) CORRESPONDENCE ADDRESS:
      (A) ADDRESSEE: Greenlee and Winner, P.C.
      (B) STREET: 5370 Manhattan Circle, Suite 201
      (C) CITY: Boulder
      (D) STATE: CO
      (E) COUNTRY: USA
      (F) ZIP: 80303
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1,0, Version #1.25
   (vi) CURRENT APPLICATION DATA:
      (A) APPLICATION NUMBER: Unassigned
      (B) FILING DATE: 09-SEP-1994
      (C) CLASSIFICATION:
   (vii) PRIOR APPLICATION DATA:
      (A) APPLICATION NUMBER: US 08/119,361
      (B) FILING DATE: 10-SEP-1993
   (vii) PRIOR APPLICATION DATA:
      (A) APPLICATION NUMBER: US 08/265,441
      (B) FILING DATE: 24-JUN-1994
   (vii) PRIOR APPLICATION DATA:
      (A) APPLICATION NUMBER: US 08/141,324
      (B) FILING DATE: 21-OCT-1993
   (viii) ATTORNEY/AGENT INFORMATION:
      (A) NAME: Ferber, Donna M.
      (B) REGISTRATION NUMBER: 33,878
      (C) REFERENCE/DOCKET NUMBER: 21-93B PCT
   (ix) TELECOMMUNICATION INFORMATION:
      (A) TELEPHONE: 303-499-8080
      (B) TELEFAX: 303-499-8089
      (C) TELEX: 49617824
(2) INFORMATION FOR SEQ ID NO:1:
   (i) SEQUENCE CHARACTERISTICS:
      (A) LENGTH: 43 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: Porphyromonas gingivalis
      (B) STRAIN: H66
   (xi) SEQUENCE DESCRIPTION: SEQ ID NO:1:
   Tyr Thr Pro Val Glu Glu Lys Gln Asn Gly Arg Met Ile Val Ile Val
   1               5                   10                  15
   Ala Lys Lys Tyr Glu Gly Asp Ile Lys Asp Phe Val Asp Trp Lys Asn
               20                  25                  30
   Gln Arg Gly Leu Thr Lys Xaa Val Lys Xaa Ala
           35                  40
(2) INFORMATION FOR SEQ ID NO:2:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: protein
   (iii) HYPOTHETICAL: NO
   (iv) ANTI-SENSE: NO
   (v) FRAGMENT TYPE: internal
   (xi) SEQUENCE DESCRIPTION: SEQ ID NO:2:
   Gly Tyr Gly Asp Ser Asn Tyr Lys
   1               5
(2) INFORMATION FOR SEQ ID NO:3:
   (i) SEQUENCE CHARACTERISTICS:
      (A) LENGTH: 3159 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 949. .3159
   (ix) FEATURE:
      (A) NAME/KEY: mat_peptide
      (B) LOCATION: 1630..3105
   (xi) SEQUENCE DESCRIPTION: SEQ ID NO:3:
   CTGCAGAGGG CTGGTAAAGA CCGCCTCGGG ATCGAGGCCT TTGAGACGGG CACAAGCCGC    60
   CGCAGCCTCC TCTTCGAAGG TGTCTCGAAC GTCCACATCG GTGAATCCGT AGCAGTGCTC   120
   ATTGCCATTG AGCAGCACCG AGGTGTGGCG CATCAGATAT ATTTTCATCA GTGGATTATT   180
   AGGGTATCGG TCAGAAAAAG CCTTCCGAAT CCGACAAAGA TAGTAGAAAG AGAGTGCATC   240
   TGAAAACAGA TCATTCGAGG ATTATCGATC AACTGAAAAG GCAGGAGTTG TTTTGCGTTT   300
   TGGTTCGGAA AATTACCTGA TCAGCATTCG TAAAAACGTG GCGCGAGAAT TTTTTCGTTT   360
   TGGCGCGAGA ATTAAAAATT TTTGGAACCA CAGCGAAAAA AATCTCGCGC CGTTTTCTCA   420
   GGATTTACAG ACCACAATCC GAGCATTTTC GGTTCGTAAT TCATCGAAGA GACAGGTTTT   480
   ACCGCATTGA AATCAGAGAG AGAATATCCG TAGTCCAACG GTTCATCCTT ATATCAGAGG   540
   TTAAAAGATA TGGTACGCTC ATCGAGGAGC TGATTGGCTT AGTAGGTGAG ACTTTCTTAA   600
   GAGACTATCG GCACCTACAG GAAGTTCATG GCACACAAGG CAAAGGAGGC AATCTTCGCA   660
   GACCGGACTC ATATCAAAAG GATGAAACGA CTTTTCCATA CGACAACCAA ATAGCCGTCT   720
   ACGGTAGACG AATGCAAACC CAATATGAGG CCATCAATCA ATCCGAATGA CAGCTTTTGG   780
   GCAATATATT ATGCATATTT TGATTCGCGT TTAAAGGAAA AGTGCATATA TTTGCGATTG   840
   TGGTATTTCT TTCGGTTTCT ATGTGAATTT TGTCTCCCAA GAAGACTTTA TAATGCATAA   900
   ATACAGAAGG GGTACTACAC AGTAAAATCA TATTCTAATT TCATCAAA ATG AAA AAC    957
                                                        Met Lys Asn
                                                        -227    -225
   TTG AAC AAG TTT GTT TCG ATT GCT CTT TGC TCT TCC TTA TTA GGA GGA    1005
   Leu Asn Lys Phe Val Ser Ile Ala Leu Cys Ser Ser Leu Leu Gly Gly
                   -220                -215                -210
   ATG GCA TTT GCG CAG CAG ACA GAG TTG GGA CGC AAT CCG AAT GTC AGA    1053
   Met Ala Phe Ala Gln Gln Thr Glu Leu Gly Arg Asn Pro Asn Val Arg
               -205                -200                -195
   TTG CTC GAA TCC ACT CAG CAA TCG GTG ACA AAG GTT CAG TTC CGT ATG    1101
   Leu Leu Glu Ser Thr Gln Gln Ser Val Thr Lys Val Gln Phe Arg Met
           -190                -185                -180
   GAC AAC CTC AAG TTC ACC GAA GTT CAA ACC CCT AAG GGA ATC GGA CAA    1149
   Asp Asn Leu Lys Phe Thr Glu Val Gln Thr Pro Lys Gly Ile Gly Gln
       -175                -170                -165
   GTG CCG ACC TAT ACA GAA GGG GTT AAT CTT TCC GAA AAA GGG ATG CCT    1197
   Val Pro Thr Tyr Thr Glu Gly Val Asn Leu Ser Glu Lys Gly Met Pro
   -160                -155                -150                -145
   ACG CTT CCC ATT CTA TCA CGC TCT TTG GCG GTT TCA GAC ACT CGT GAG    1245
   Thr Leu Pro Ile Leu Ser Arg Ser Leu Ala Val Ser Asp Thr Arg Glu
                   -140                -135                -130
   ATG AAG GTA GAG GTT GTT TCC TCA AAG TTC ATC GAA AAG AAA AAT GTC    1293
   Met Lys Val Glu Val Val Ser Ser Lys Phe Ile Glu Lys Lys Asn Val
               -125                -120                -115
   CTG ATT GCA CCC TCC AAG GGC ATG ATT ATG CGT AAC GAA GAT CCG AAA    1341
   Leu Ile Ala Pro Ser Lys Gly Met Ile Met Arg Asn Glu Asp Pro Lys
           -110                -105                -100
   AAG ATC CCT TAC GTT TAT GGA AAG AGC TAC TCG CAA AAC AAA TTC TTC        1389
   Lys Ile Pro Tyr Val Tyr Gly Lys Ser Tyr Ser Gln Asn Lys Phe Phe
       -95                 -90                 -85
   CCG GGA GAG ATC GCC ACG CTT GAT GAT CCT TTT ATC CTT CGT GAT GTG        1437
   Pro Gly Glu Ile Ala Thr Leu Asp Asp Pro Phe Ile Leu Arg Asp Val
   -80                 -75                 -70                 -65
   CGT GGA CAG GTT GTA AAC TTT GCG CCT TTG CAG TAT AAC CCT GTG ACA        1485
   Arg Gly Gln Val Val Asn Phe Ala Pro Leu Gln Tyr Asn Pro Val Thr
                   -60                 -55                 -50
   AAG ACG TTG CGC ATC TAT ACG GAA ATC ACT GTG GCA GTG AGC GAA ACT        1533
   Lys Thr Leu Arg Ile Tyr Thr Glu Ile Thr Val Ala Val Ser Glu Thr
               -45                 -40                 -35
   TCG GAA CAA GGC AAA AAT ATT CTG AAC AAG AAA GGT ACA TTT GCC GGC        1581
   Ser Glu Gln Gly Lys Asn Ile Leu Asn Lys Lys Gly Thr Phe Ala Gly
           -30                 -25                 -20
   TTT GAA GAC ACA TAC AAG CGC ATG TTC ATG AAC TAC GAG CCG GGG CGT        1629
   Phe Glu Asp Thr Tyr Lys Arg Met Phe Met Asn Tyr Glu Pro Gly Arg
       -15                 -10                 -5
   TAC ACA CCG GTA GAG GAA AAA CAA AAT GGT CGT ATG ATC GTC ATC GTA        1677
   Tyr Thr Pro Val Glu Glu Lys Gln Asn Gly Arg Met Ile Val Ile Val
    1               5                  10                  15
   GCC AAA AAG TAT GAG GGA GAT ATT AAA GAT TTC GTT GAT TGG AAA AAC        1725
   Ala Lys Lys Tyr Glu Gly Asp Ile Lys Asp Phe Val Asp Trp Lys Asn
               20                  25                  30
   CAA CGC GGT CTC CGT ACC GAG GTG AAA GTG GCA GAA GAT ATT GCT TCT        1773
   Gln Arg Gly Leu Arg Thr Glu Val Lys Val Ala Glu Asp Ile Ala Ser
           35                  40                  45
   CCC GTT ACA GCT AAT GCT ATT CAG CAG TTC GTT AAG CAA GAA TAC GAG        1821
   Pro Val Thr Ala Asn Ala Ile Gln Gln Phe Val Lys Gln Glu Tyr Glu
       50                  55                  60
   AAA GAA GGT AAT GAT TTG ACC TAT GTT CTT TTG GTT GGC GAT CAC AAA        1869
   Lys Glu Gly Asn Asp Leu Thr Tyr Val Leu Leu Val Gly Asp His Lys
   65                  70                  75                  80
   GAT ATT CCT GCC AAA ATT ACT CCG GGG ATC AAA TCC GAC CAG GTA TAT        1917
   Asp Ile Pro Ala Lys Ile Thr Pro Gly Ile Lys Ser Asp Gln Val Tyr
                   85                  90                  95
   GGA CAA ATA GTA GGT AAT GAC CAC TAC AAC GAA GTC TTC ATC GGT CGT        1965
   Gly Gln Ile Val Gly Asn Asp His Tyr Asn Glu Val Phe Ile Gly Arg
               100                 105                 110
   TTC TCA TGT GAG AGC AAA GAG GAT CTG AAG ACA CAA ATC GAT CGG ACT        2013
   Phe Ser Cys Glu Ser Lys Glu Asp Leu Lys Thr Gln Ile Asp Arg Thr
           115                 120                 125
   ATT CAC TAT GAG CGC AAT ATA ACC ACG GAA GAC AAA TGG CTC GGT CAG        2061
   Ile His Tyr Glu Arg Asn Ile Thr Thr Glu Asp Lys Trp Leu Gly Gln
       130                 135                 140
   GCT CTT TGT ATT GCT TCG GCT GAA GGA GGC CCA TCC GCA GAC AAT GGT        2109
   Ala Leu Cys Ile Ala Ser Ala Glu Gly Gly Pro Ser Ala Asp Asn Gly
   145                 150                 155                 160
   GAA AGT GAT ATC CAG CAT GAG AAT GTA ATC GCC AAT CTG CTT ACC CAG        2157
   Glu Ser Asp Ile Gln His Glu Asn Val Ile Ala Asn Leu Leu Thr Gln
                   165                 170                 175
   TAT GGC TAT ACC AAG ATT ATC AAA TGT TAT GAT CCG GGA GTA ACT CCT        2205
   Tyr Gly Tyr Thr Lys Ile Ile Lys Cys Tyr Asp Pro Gly Val Thr Pro
               180                 185                 190
   AAA AAC ATT ATT GAT GCT TTC AAC GGA GGA ATC TCG TTG GTC AAC TAT        2253
   Lys Asn Ile Ile Asp Ala Phe Asn Gly Gly Ile Ser Leu Val Asn Tyr
           195                 200                 205
   ACG GGC CAC GGT AGC GAA ACA GCT TGG GGT ACG TCT CAC TTC GGC ACC        2301
   Thr Gly His Gly Ser Glu Thr Ala Trp Gly Thr Ser His Phe Gly Thr
       210                 215                 220
   ACT CAT GTG AAG CAG CTT ACC AAC AGC AAC CAG CTA CCG TTT ATT TTC        2349
   Thr His Val Lys Gln Leu Thr Asn Ser Asn Gln Leu Pro Phe Ile Phe
   225                 230                 235                 240
   GAC GTA GCT TGT GTG AAT GGC GAT TTC CTA TTC AGC ATG CCT TGC TTC        2397
   Asp Val Ala Cys Val Asn Gly Asp Phe Leu Phe Ser Met Pro Cys Phe
                   245                 250                 255
   GCA GAA GCC CTG ATG CGT GCA CAA AAA GAT GGT AAG CCG ACA GGT ACT        2445
   Ala Glu Ala Leu Met Arg Ala Gln Lys Asp Gly Lys Pro Thr Gly Thr
               260                 265                 270
   GTT GCT ATC ATA GCG TCT ACG ATC AAC CAG TCT TGG GCT TCT CCT ATG        2493
   Val Ala Ile Ile Ala Ser Thr Ile Asn Gln Ser Trp Ala Ser Pro Met
           275                 280                 285
   CGC GGG CAG GAT GAG ATG AAC GAA ATT CTG TGC GAA AAA CAC CCG AAC        2541
   Arg Gly Gln Asp Glu Met Asn Glu Ile Leu Cys Glu Lys His Pro Asn
       290                 295                 300
   AAC ATC AAG CGT ACT TTC GGT GGT GTC ACC ATG AAC GGT ATG TTT GCT        2589
   Asn Ile Lys Arg Thr Phe Gly Gly Val Thr Met Asn Gly Met Phe Ala
   305                 310                 315                 320
   ATG GTG GAA AAG TAT AAA AAG GAT GGT GAG AAG ATG CTC GAC ACA TGG        2637
   Met Val Glu Lys Tyr Lys Lys Asp Gly Glu Lys Met Leu Asp Thr Trp
                   325                 330                 335
   ACT GTT TTC GGC GAC CCC TCG CTG CTC GTT CGT ACA CTT GTC CCG ACC        2685
   Thr Val Phe Gly Asp Pro Ser Leu Leu Val Arg Thr Leu Val Pro Thr
               340                 345                 350
   AAA ATG CAG GTT ACG GCT CCG GCT CAG ATT AAT TTG ACG GAT GCT TCA        2733
   Lys Met Gln Val Thr Ala Pro Ala Gln Ile Asn Leu Thr Asp Ala Ser
           355                 360                 365
   GTC AAC GTA TCT TGC GAT TAT AAT GGT GCT ATT GCT ACC ATT TCA GCC        2781
   Val Asn Val Ser Cys Asp Tyr Asn Gly Ala Ile Ala Thr Ile Ser Ala
       370                 375                 380
   AAT GGA AAG ATG TTC GGT TCT GCA GTT GTC GAA AAT GGA ACA GCT ACA        2829
   Asn Gly Lys Met Phe Gly Ser Ala Val Val Glu Asn Gly Thr Ala Thr
   385                 390                 395                 400
   ATC AAT CTG ACA GGT CTG ACA AAT GAA AGC ACG CTT ACC CTT ACA GTA        2877
   Ile Asn Leu Thr Gly Leu Thr Asn Glu Ser Thr Leu Thr Leu Thr Val
                   405                 410                 415
   GTT GGT TAC AAC AAA GAG ACG GTT ATT AAG ACC ATC AAC ACT AAT GGT        2925
   Val Gly Tyr Asn Lys Glu Thr Val Ile Lys Thr Ile Asn Thr Asn Gly
               420                 425                 430
   GAG CCT AAC CCC TAC CAG CCC GTT TCC AAC TTG ACA GCT ACA ACG CAG        2973
   Glu Pro Asn Pro Tyr Gln Pro Val Ser Asn Leu Thr Ala Thr Thr Gln
           435                 440                 445
   GGT CAG AAA GTA ACG CTC AAG TGG GAT GCA CCG AGC ACG AAA ACC AAT      3021
   Gly Gln Lys Val Thr Leu Lys Trp Asp Ala Pro Ser Thr Lys Thr Asn
       450                 455                 460
   GCA ACC ACT AAT ACC GCT CGC AGC GTG GAT GGC ATA CGA GAA TTG GTT      3069
   Ala Thr Thr Asn Thr Ala Arg Ser Val Asp Gly Ile Arg Glu Leu Val
   465                 470                 475                 480
   CTT CTG TCA GTC AGC GAT GCC CCC GAA CTT CTT CGC AGC GGT CAG GCC      3117
   Leu Leu Ser Val Ser Asp Ala Pro Glu Leu Leu Arg Ser Gly Gln Ala
                   485                 490                 495
   GAG ATT GTT CTT GAA GCT CAC GAT GTT TGG AAT GAT GGA TCC              3159
   Glu Ile Val Leu Glu Ala His Asp Val Trp Asn Asp Gly Ser
               500                 505                 510
(2) INFORMATION FOR SEQ ID NO:4:
   (i) SEQUENCE CHARACTERISTICS:
      (A) LENGTH: 737 amino acids
      (B) TYPE: amino acid
      (D) TOPOLOGY: linear
   (ii) MOLECULE TYPE: protein
   (xi) SEQUENCE DESCRIPTION: SEQ ID NO:4:
   Met Lys Asn Leu Asn Lys Phe Val Ser Ile Ala Leu Cys Ser Ser Leu
   -227     -225                    -220                    -215
   Leu Gly Gly Met Ala Phe Ala Gln Gln Thr Glu Leu Gly Arg Asn Pro
       -210                    -205                    -200
   Asn Val Arg Leu Leu Glu Ser Thr Gln Gln Ser Val Thr Lys Val Gln
   -195                    -190                    -185                    -180
   Phe Arg Met Asp Asn Leu Lys Phe Thr Glu Val Gln Thr Pro Lys Gly
                   -175                    -170                    -165
   Ile Gly Gln Val Pro Thr Tyr Thr Glu Gly Val Asn Leu Ser Glu Lys
               -160                    -155                    -150
   Gly Met Pro Thr Leu Pro Ile Leu Ser Arg Ser Leu Ala Val Ser Asp
           -145                    -140                    -135
   Thr Arg Glu Met Lys Val Glu Val Val Ser Ser Lys Phe Ile Glu Lys
       -130                    -125                    -120
   Lys Asn Val Leu Ile Ala Pro Ser Lys Gly Met Ile Met Arg Asn Glu
   -115                    -110                    -105                    -100
   Asp Pro Lys Lys Ile Pro Tyr Val Tyr Gly Lys Ser Tyr Ser Gln Asn
                   -95                     -90                     -85
   Lys Phe Phe Pro Gly Glu Ile Ala Thr Leu Asp Asp Pro Phe Ile Leu
               -80                     -75                     -70
   Arg Asp Val Arg Gly Gln Val Val Asn Phe Ala Pro Leu Gln Tyr Asn
           -65                     -60                     -55
   Pro Val Thr Lys Thr Leu Arg Ile Tyr Thr Glu Ile Thr Val Ala Val
       -50                     -45                     -40
   Ser Glu Thr Ser Glu Gln Gly Lys Asn Ile Leu Asn Lys Lys Gly Thr
   -35                     -30                     -25                     -20
   Phe Ala Gly Phe Glu Asp Thr Tyr Lys Arg Met Phe Met Asn Tyr Glu
   -15 -10 -5
   Pro Gly Arg Tyr Thr Pro Val Glu Glu Lys Gln Asn Gly Arg Met Ile
   1 5 10
   Val Ile Val Ala Lys Lys Tyr Glu Gly Asp Ile Lys Asp Phe Val Asp
   15 20 25
   Trp Lys Asn Gln Arg Gly Leu Arg Thr Glu Val Lys Val Ala Glu Asp
   30 35 40 45
   Ile Ala Ser Pro Val Thr Ala Asn Ala Ile Gln Gln Phe Val Lys Gln
   50 55 60
   Glu Tyr Glu Lys Glu Gly Asn Asp Leu Thr Tyr Val Leu Leu Val Gly
   65 70 75
   Asp His Lys Asp Ile Pro Ala Lys Ile Thr Pro Gly Ile Lys Ser Asp
   80 85 90
   Gln Val Tyr Gly Gln Ile Val Gly Asn Asp His Tyr Asn Glu Val Phe
   95 100 105
   Ile Gly Arg Phe Ser Cys Glu Ser Lys Glu Asp Leu Lys Thr Gln Ile
   110 115 120 125
   Asp Arg Thr Ile His Tyr Glu Arg Asn Ile Thr Thr Glu Asp Lys Trp
   130 135 140
   Leu Gly Gln Ala Leu Cys Ile Ala Ser Ala Glu Gly Gly Pro Ser Ala
   145 150 155
   Asp Asn Gly Glu Ser Asp Ile Gln His Glu Asn Val Ile Ala Asn Leu
   160 165 170
   Leu Thr Gln Tyr Gly Tyr Thr Lys Ile Ile Lys Cys Tyr Asp Pro Gly
   175 180 185
   Val Thr Pro Lys Asn Ile Ile Asp Ala Phe Asn Gly Gly Ile Ser Leu
   190 195 200 205
   Val Asn Tyr Thr Gly His Gly Ser Glu Thr Ala Trp Gly Thr Ser His
   210 215 220
   Phe Gly Thr Thr His Val Lys Gln Leu Thr Asn Ser Asn Gln Leu Pro
   225 230 235
   Phe Ile Phe Asp Val Ala Cys Val Asn Gly Asp Phe Leu Phe Ser Met
   240 245 250
   Pro Cys Phe Ala Glu Ala Leu Met Arg Ala Gln Lys Asp Gly Lys Pro
   255 260 265
   Thr Gly Thr Val Ala Ile Ile Ala Ser Thr Ile Asn Gln Ser Trp Ala
   270 275 280 285
   Ser Pro Met Arg Gly Gln Asp Glu Met Asn Glu Ile Leu Cys Glu Lys
   290 295 300
   His Pro Asn Asn Ile Lys Arg Thr Phe Gly Gly Val Thr Met Asn Gly
   305 310 315
   Met Phe Ala Met Val Glu Lys Tyr Lys Lys Asp Gly Glu Lys Met Leu
   320 325 330
   Asp Thr Trp Thr Val Phe Gly Asp Pro Ser Leu Leu Val Arg Thr Leu
   335 340 345
   Val Pro Thr Lys Met Gln Val Thr Ala Pro Ala Gln Ile Asn Leu Thr
   350 355 360 365
   Asp Ala Ser Val Asn Val Ser Cys Asp Tyr Asn Gly Ala Ile Ala Thr
   370 375 380
   Ile Ser Ala Asn Gly Lys Met Phe Gly Ser Ala Val Val Glu Asn Gly
   385 390 395
   Thr Ala Thr Ile Asn Leu Thr Gly Leu Thr Asn Glu Ser Thr Leu Thr
   400 405 410
   Leu Thr Val Val Gly Tyr Asn Lys Glu Thr Val Ile Lys Thr Ile Asn
   415 420 425
   Thr Asn Gly Glu Pro Asn Pro Tyr Gln Pro Val Ser Asn Leu Thr Ala
   430 435 440 445
   Thr Thr Gln Gly Gln Lys Val Thr Leu Lys Trp Asp Ala Pro Ser Thr
   450 455 460
   Lys Thr Asn Ala Thr Thr Asn Thr Ala Arg Ser Val Asp Gly Ile Arg
   465 470 475
   Glu Leu Val Leu Leu Ser Val Ser Asp Ala Pro Glu Leu Leu Arg Ser
   480 485 490
   Gly Gln Ala Glu Ile Val Leu Glu Ala His Asp Val Trp Asn Asp Gly
   495 500 505
   Ser
   510
(2) INFORMATION FOR SEQ ID NO:5:
   (i) SEQUENCE CHARACTERISTICS:
      (A) LENGTH: 4 amino acids
      (B) TYPE: amino acid
      (C) STRANDEDNESS: single
      (D) TOPOLOGY: linear
   (ii) MOLECULE TYPE: protein
   (iii) HYPOTHETICAL: NO
   (iv) ANTI-SENSE: NO
   (v) FRAGMENT TYPE: C-terminal
   (xi) SEQUENCE DESCRIPTION: SEQ ID NO:5:
   Glu Leu Leu Arg
   1
(2) INFORMATION FOR SEQ ID NO:6:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: DNA (other nucleic acid)
   (iii) HYPOTHETICAL: NO
   (xi) SEQUENCE DESCRIPTION: SEQ ID NO:6:
   GGCTTTACNC CNGTNGARGA RYTNGA 26
(2) INFORMATION FOR SEQ ID NO:7:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (other nucleic acid)
   (iii) HYPOTHETICAL: NO
   (xi) SEQUENCE DESCRIPTION: SEQ ID NO: 7:
   GGCTTTRTTY TTCCARTCNA CRAARTCYTT 30
(2) INFORMATION FOR SEQ ID NO:8:
   (i) SEQUENCE CHARACTERISTICS:
      (A) LENGTH: 48 base pairs
      (B) TYPE: nucleic acid
      (C) STRANDEDNESS: single
      (D) TOPOLOGY: linear
   (ii) MOLECULE TYPE: DNA (other nucleic acid)
   (iii) HYPOTHETICAL: NO
   (xi) SEQUENCE DESCRIPTION: SEQ ID NO:8:
   CCTGGAGAAT TCTCGTATGA TCGTCATCGT AGCCAAAAAG TATGAGGG 48
(2) INFORMATION FOR SEQ ID NO:9:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (other nucleic acid)
   (iii) HYPOTHETICAL: NO
   (xi) SEQUENCE DESCRIPTION: SEQ ID NO:9:
   ATCAACACTA ATGGTGAGCC 20
(2) INFORMATION FOR SEQ ID NO:10:
   (i) SEQUENCE CHARACTERISTICS:
      (A) LENGTH: 7266 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 949..6063
   (xi) SEQUENCE DESCRIPTION: SEQ ID NO:10:
   CTGCAGAGGG CTGGTAAAGA CCGCCTCGGG ATCGAGGCCT TTGAGACGGG CACAAGCCGC        60
   CGCAGCCTCC TCTTCGAAGG TGTCTCGAAC GTCCACATCG GTGAATCCGT AGCAGTGCTC       120
   ATTGCCATTG AGCAGCACCG AGGTGTGGCG CATCAGATAT ATTTTCATCA GTGGATTATT       180
   AGGGTATCGG TCAGAAAAAG CCTTCCGAAT CCGACAAAGA TAGTAGAAAG AGAGTGCATC       240
   TGAAAACAGA TCATTCGAGG ATTATCGATC AACTGAAAAG GCAGGAGTTG TTTTGCGTTT       300
   TGGTTCGGAA AATTACCTGA TCAGCATTCG TAAAAACGTG GCGCGAGAAT TTTTTCGTTT       360
   TGGCGCGAGA ATTAAAAATT TTTGGAACCA CAGCGAAAAA AATCTCGCGC CGTTTTCTCA       420
   GGATTTACAG ACCACAATCC GAGCATTTTC GGTTCGTAAT TCATCGAAGA GACAGGTTTT       480
   ACCGCATTGA AATCAGAGAG AGAATATCCG TAGTCCAACG GTTCATCCTT ATATCAGAGG       540
   TTAAAAGATA TGGTACGCTC ATCGAGGAGC TGATTGGCTT AGTAGGTGAG ACTTTCTTAA       600
   GAGACTATCG GCACCTACAG GAAGTTCATG GCACACAAGG CAAAGGAGGC AATCTTCGCA       660
   GACCGGACTC ATATCAAAAG GATGAAACGA CTTTTCCATA CGACAACCAA ATAGCCGTCT       720
   ACGGTAGACG AATGCAAACC CAATATGAGG CCATCAATCA ATCCGAATGA CAGCTTTTGG       780
   GCAATATATT ATGCATATTT TGATTCGCGT TTAAAGGAAA AGTGCATATA TTTGCGATTG       840
   TGGTATTTCT TTCGGTTTCT ATGTGAATTT TGTCTCCCAA GAAGACTTTA TAATGCATAA       900
   ATACAGAAGG GGTACTACAC AGTAAAATCA TATTCTAATT TCATCAAA ATG AAA AAC        957
                                                    Met Lys Asn
                                                     1
   TTG AAC AAG TTT GTT TCG ATT GCT CTT TGC TCT TCC TTA TTA GGA GGA       1005
   Leu Asn Lys Phe Val Ser Ile Ala Leu Cys Ser Ser Leu Leu Gly Gly
        5                  10                  15
   ATG GCA TTT GCG CAG CAG ACA GAG TTG GGA CGC AAT CCG AAT GTC AGA       1053
   Met Ala Phe Ala Gln Gln Thr Glu Leu Gly Arg Asn Pro Asn Val Arg
    20                  25                  30                  35
   TTG CTC GAA TCC ACT CAG CAA TCG GTG ACA AAG GTT CAG TTC CGT ATG       1101
   Leu Leu Glu Ser Thr Gln Gln Ser Val Thr Lys Val Gln Phe Arg Met
                    40                  45                  50
   GAC AAC CTC AAG TTC ACC GAA GTT CAA ACC CCT AAG GGA ATC GGA CAA      1149
   Asp Asn Leu Lys Phe Thr Glu Val Gln Thr Pro Lys Gly Ile Gly Gln
                55                  60                  65
   GTG CCG ACC TAT ACA GAA GGG GTT AAT CTT TCC GAA AAA GGG ATG CCT      1197
   Val Pro Thr Tyr Thr Glu Gly Val Asn Leu Ser Glu Lys Gly Met Pro
            70                  75                  80
   ACG CTT CCC ATT CTA TCA CGC TCT TTG GCG GTT TCA GAC ACT CGT GAG      1245
   Thr Leu Pro Ile Leu Ser Arg Ser Leu Ala Val Ser Asp Thr Arg Glu
        85                  90                  95
   ATG AAG GTA GAG GTT GTT TCC TCA AAG TTC ATC GAA AAG AAA AAT GTC      1293
   Met Lys Val Glu Val Val Ser Ser Lys Phe Ile Glu Lys Lys Asn Val
   100                 105                 110                 115
   CTG ATT GCA CCC TCC AAG GGC ATG ATT ATG CGT AAC GAA GAT CCG AAA      1341
   Leu Ile Ala Pro Ser Lys Gly Met Ile Met Arg Asn Glu Asp Pro Lys
                   120                 125                 130
   AAG ATC CCT TAC GTT TAT GGA AAG AGC TAC TCG CAA AAC AAA TTC TTC      1389
   Lys Ile Pro Tyr Val Tyr Gly Lys Ser Tyr Ser Gln Asn Lys Phe Phe
               135                 140                 145
   CCG GGA GAG ATC GCC ACG CTT GAT GAT CCT TTT ATC CTT CGT GAT GTG      1437
   Pro Gly Glu Ile Ala Thr Leu Asp Asp Pro Phe Ile Leu Arg Asp Val
           150                 155                 160
   CGT GGA CAG GTT GTA AAC TTT GCG CCT TTG CAG TAT AAC CCT GTG ACA      1485
   Arg Gly Gln Val Val Asn Phe Ala Pro Leu Gln Tyr Asn Pro Val Thr
       165                 170                 175
   AAG ACG TTG CGC ATC TAT ACG GAA ATC ACT GTG GCA GTG AGC GAA ACT      1533
   Lys Thr Leu Arg Ile Tyr Thr Glu Ile Thr Val Ala Val Ser Glu Thr
   180                 185                 190                 195
   TCG GAA CAA GGC AAA AAT ATT CTG AAC AAG AAA GGT ACA TTT GCC GGC      1581
   Ser Glu Gln Gly Lys Asn Ile Leu Asn Lys Lys Gly Thr Phe Ala Gly
                   200                 205                 210
   TTT GAA GAC ACA TAC AAG CGC ATG TTC ATG AAC TAC GAG CCG GGG CGT      1629
   Phe Glu Asp Thr Tyr Lys Arg Met Phe Met Asn Tyr Glu Pro Gly Arg
               215                 220                 225
   TAC ACA CCG GTA GAG GAA AAA CAA AAT GGT CGT ATG ATC GTC ATC GTA      1677
   Tyr Thr Pro Val Glu Glu Lys Gln Asn Gly Arg Met Ile Val Ile Val
           230                 235                 240
   GCC AAA AAG TAT GAG GGA GAT ATT AAA GAT TTC GTT GAT TGG AAA AAC      1725
   Ala Lys Lys Tyr Glu Gly Asp Ile Lys Asp Phe Val Asp Trp Lys Asn
       245                 250                 255
   CAA CGC GGT CTC CGT ACC GAG GTG AAA GTG GCA GAA GAT ATT GCT TCT      1773
   Gln Arg Gly Leu Arg Thr Glu Val Lys Val Ala Glu Asp Ile Ala Ser
   260                 265                 270                 275
   CCC GTT ACA GCT AAT GCT ATT CAG CAG TTC GTT AAG CAA GAA TAC GAG      1821
   Pro Val Thr Ala Asn Ala Ile Gln Gln Phe Val Lys Gln Glu Tyr Glu
                   280                 285                 290
   AAA GAA GGT AAT GAT TTG ACC TAT GTT CTT TTG GTT GGC GAT CAC AAA      1869
   Lys Glu Gly Asn Asp Leu Thr Tyr Val Leu Leu Val Gly Asp His Lys
               295                 300                 305
   GAT ATT CCT GCC AAA ATT ACT CCG GGG ATC AAA TCC GAC CAG GTA TAT        1917
   Asp Ile Pro Ala Lys Ile Thr Pro Gly Ile Lys Ser Asp Gln Val Tyr
           310                 315                 320
   GGA CAA ATA GTA GGT AAT GAC CAC TAC AAC GAA GTC TTC ATC GGT CGT        1965
   Gly Gln Ile Val Gly Asn Asp His Tyr Asn Glu Val Phe Ile Gly Arg
       325                 330                 335
   TTC TCA TGT GAG AGC AAA GAG GAT CTG AAG ACA CAA ATC GAT CGG ACT        2013
   Phe Ser Cys Glu Ser Lys Glu Asp Leu Lys Thr Gln Ile Asp Arg Thr
   340                 345                 350                 355
   ATT CAC TAT GAG CGC AAT ATA ACC ACG GAA GAC AAA TGG CTC GGT CAG        2061
   Ile His Tyr Glu Arg Asn Ile Thr Thr Glu Asp Lys Trp Leu Gly Gln
                   360                 365                 370
   GCT CTT TGT ATT GCT TCG GCT GAA GGA GGC CCA TCC GCA GAC AAT GGT        2109
   Ala Leu Cys Ile Ala Ser Ala Glu Gly Gly Pro Ser Ala Asp Asn Gly
               375                 380                 385
   GAA AGT GAT ATC CAG CAT GAG AAT GTA ATC GCC AAT CTG CTT ACC CAG        2157
   Glu Ser Asp Ile Gln His Glu Asn Val Ile Ala Asn Leu Leu Thr Gln
           390                 395                 400
   TAT GGC TAT ACC AAG ATT ATC AAA TGT TAT GAT CCG GGA GTA ACT CCT        2205
   Tyr Gly Tyr Thr Lys Ile Ile Lys Cys Tyr Asp Pro Gly Val Thr Pro
       405                 410                 415
   AAA AAC ATT ATT GAT GCT TTC AAC GGA GGA ATC TCG TTG GTC AAC TAT        2253
   Lys Asn Ile Ile Asp Ala Phe Asn Gly Gly Ile Ser Leu Val Asn Tyr
   420                 425                 430                 435
   ACG GGC CAC GGT AGC GAA ACA GCT TGG GGT ACG TCT CAC TTC GGC ACC        2301
   Thr Gly His Gly Ser Glu Thr Ala Trp Gly Thr Ser His Phe Gly Thr
                   440                 445                 450
   ACT CAT GTG AAG CAG CTT ACC AAC AGC AAC CAG CTA CCG TTT ATT TTC        2349
   Thr His Val Lys Gln Leu Thr Asn Ser Asn Gln Leu Pro Phe Ile Phe
               455                 460                 465
   GAC GTA GCT TGT GTG AAT GGC GAT TTC CTA TTC AGC ATG CCT TGC TTC        2397
   Asp Val Ala Cys Val Asn Gly Asp Phe Leu Phe Ser Met Pro Cys Phe
           470                 475                 480
   GCA GAA GCC CTG ATG CGT GCA CAA AAA GAT GGT AAG CCG ACA GGT ACT        2445
   Ala Glu Ala Leu Met Arg Ala Gln Lys Asp Gly Lys Pro Thr Gly Thr
       485                 490                 495
   GTT GCT ATC ATA GCG TCT ACG ATC AAC CAG TCT TGG GCT TCT CCT ATG        2493
   Val Ala Ile Ile Ala Ser Thr Ile Asn Gln Ser Trp Ala Ser Pro Met
   500                 505                 510                 515
   CGC GGG CAG GAT GAG ATG AAC GAA ATT CTG TGC GAA AAA CAC CCG AAC        2541
   Arg Gly Gln Asp Glu Met Asn Glu Ile Leu Cys Glu Lys His Pro Asn
                   520                 525                 530
   AAC ATC AAG CGT ACT TTC GGT GGT GTC ACC ATG AAC GGT ATG TTT GCT        2589
   Asn Ile Lys Arg Thr Phe Gly Gly Val Thr Met Asn Gly Met Phe Ala
               535                 540                 545
   ATG GTG GAA AAG TAT AAA AAG GAT GGT GAG AAG ATG CTC GAC ACA TGG        2637
   Met Val Glu Lys Tyr Lys Lys Asp Gly Glu Lys Met Leu Asp Thr Trp
           550                 555                 560
   ACT GTT TTC GGC GAC CCC TCG CTG CTC GTT CGT ACA CTT GTC CCG ACC      2685
   Thr Val Phe Gly Asp Pro Ser Leu Leu Val Arg Thr Leu Val Pro Thr
       565                 570                 575
   AAA ATG CAG GTT ACG GCT CCG GCT CAG ATT AAT TTG ACG GAT GCT TCA      2733
   Lys Met Gln Val Thr Ala Pro Ala Gln Ile Asn Leu Thr Asp Ala Ser
   580                 585                 590                 595
   GTC AAC GTA TCT TGC GAT TAT AAT GGT GCT ATT GCT ACC ATT TCA GCC      2781
   Val Asn Val Ser Cys Asp Tyr Asn Gly Ala Ile Ala Thr Ile Ser Ala
                   600                 605                 610
   AAT GGA AAG ATG TTC GGT TCT GCA GTT GTC GAA AAT GGA ACA GCT ACA      2829
   Asn Gly Lys Met Phe Gly Ser Ala Val Val Glu Asn Gly Thr Ala Thr
               615                 620                 625
   ATC AAT CTG ACA GGT CTG ACA AAT GAA AGC ACG CTT ACC CTT ACA GTA      2877
   Ile Asn Leu Thr Gly Leu Thr Asn Glu Ser Thr Leu Thr Leu Thr Val
           630                 635                 640
   GTT GGT TAC AAC AAA GAG ACG GTT ATT AAG ACC ATC AAC ACT AAT GGT      2925
   Val Gly Tyr Asn Lys Glu Thr Val Ile Lys Thr Ile Asn Thr Asn Gly
       645                 650                 655
   GAG CCT AAC CCC TAC CAG CCC GTT TCC AAC TTG ACA GCT ACA ACG CAG      2973
   Glu Pro Asn Pro Tyr Gln Pro Val Ser Asn Leu Thr Ala Thr Thr Gln
   660                 665                 670                 675
   GGT CAG AAA GTA ACG CTC AAG TGG GAT GCA CCG AGC ACG AAA ACC AAT      3021
   Gly Gln Lys Val Thr Leu Lys Trp Asp Ala Pro Ser Thr Lys Thr Asn
                   680                 685                 690
   GCA ACC ACT AAT ACC GCT CGC AGC GTG GAT GGC ATA CGA GAA TTG GTT      3069
   Ala Thr Thr Asn Thr Ala Arg Ser Val Asp Gly Ile Arg Glu Leu Val
               695                 700                 705
   CTT CTG TCA GTC AGC GAT GCC CCC GAA CTT CTT CGC AGC GGT CAG GCC      3117
   Leu Leu Ser Val Ser Asp Ala Pro Glu Leu Leu Arg Ser Gly Gln Ala
           710                 715                 720
   GAG ATT GTT CTT GAA GCT CAC GAT GTT TGG AAT GAT GGA TCC GGT TAT      3165
   Glu Ile Val Leu Glu Ala His Asp Val Trp Asn Asp Gly Ser Gly Tyr
       725                 730                 735
   CAG ATT CTT TTG GAT GCA GAC CAT GAT CAA TAT GGA CAG GTT ATA CCC      3213
   Gln Ile Leu Leu Asp Ala Asp His Asp Gln Tyr Gly Gln Val Ile Pro
   740                 745                 750                 755
   AGT GAT ACC CAT ACT CTT TGG CCG AAC TGT AGT GTC CCG GCC AAT CTG      3261
   Ser Asp Thr His Thr Leu Trp Pro Asn Cys Ser Val Pro Ala Asn Leu
                   760                 765                 770
   TTC GCT CCG TTC GAA TAT ACT GTT CCG GAA AAT GCA GAT CCT TCT TGT      3309
   Phe Ala Pro Phe Glu Tyr Thr Val Pro Glu Asn Ala Asp Pro Ser Cys
               775                 780                 785
   TCC CCT ACC AAT ATG ATA ATG GAT GGT ACT GCA TCC GTT AAT ATA CCG      3357
   Ser Pro Thr Asn Met Ile Met Asp Gly Thr Ala Ser Val Asn Ile Pro
           790                 795                 800
   GCC GGA ACT TAT GAC TTT GCA ATT GCT GCT CCT CAA GCA AAT GCA AAG      3405
   Ala Gly Thr Tyr Asp Phe Ala Ile Ala Ala Pro Gln Ala Asn Ala Lys
       805                 810                 815
   ATT TGG ATT GCC GGA CAA GGA CCG ACG AAA GAA GAT GAT TAT GTA TTT      3453
   Ile Trp Ile Ala Gly Gln Gly Pro Thr Lys Glu Asp Asp Tyr Val Phe
   820                 825                 830                 835
   GAA GCC GGT AAA AAA TAC CAT TTC CTT ATG AAG AAG ATG GGT AGC GGT        3501
   Glu Ala Gly Lys Lys Tyr His Phe Leu Met Lys Lys Met Gly Ser Gly
                   840                 845                 850
   GAT GGA ACT GAA TTG ACT ATA AGC GAA GGT GGT GGA AGC GAT TAC ACC        3549
   Asp Gly Thr Glu Leu Thr Ile Ser Glu Gly Gly Gly Ser Asp Tyr Thr
               855                 860                 865
   TAT ACT GTC TAT CGT GAC GGC ACG AAG ATC AAG GAA GGT CTG ACG GCT        3597
   Tyr Thr Val Tyr Arg Asp Gly Thr Lys Ile Lys Glu Gly Leu Thr Ala
           870                 875                 880
   ACG ACA TTC GAA GAA GAC GGT GTA GCT ACG GGC AAT CAT GAG TAT TGC        3645
   Thr Thr Phe Glu Glu Asp Gly Val Ala Thr Gly Asn His Glu Tyr Cys
       885                 890                 895
   GTG GAA GTT AAG TAC ACA GCC GGC GTA TCT CCG AAG GTA TGT AAA GAC        3693
   Val Glu Val Lys Tyr Thr Ala Gly Val Ser Pro Lys Val Cys Lys Asp
   900                 905                 910                 915
   GTT ACG GTA GAA GGA TCC AAT GAA TTT GCT CCT GTA CAG AAC CTG ACC        3741
   Val Thr Val Glu Gly Ser Asn Glu Phe Ala Pro Val Gln Asn Leu Thr
                   920                 925                 930
   GGT AGT GCA GTC GGC CAG AAA GTA ACG CTC AAG TGG GAT GCA CCT AAT        3789
   Gly Ser Ala Val Gly Gln Lys Val Thr Leu Lys Trp Asp Ala Pro Asn
               935                 940                 945
   GGT ACC CCG AAT CCA AAT CCG AAT CCG AAT CCG AAT CCC GGA ACA ACA        3837
   Gly Thr Pro Asn Pro Asn Pro Asn Pro Asn Pro Asn Pro Gly Thr Thr
           950                 955                 960
   ACA CTT TCC GAA TCA TTC GAA AAT GGT ATT CCT GCC TCA TGG AAG ACG        3885
   Thr Leu Ser Glu Ser Phe Glu Asn Gly Ile Pro Ala Ser Trp Lys Thr
       965                 970                 975
   ATC GAT GCA GAC GGT GAC GGG CAT GGC TGG AAG CCT GGA AAT GCT CCC        3933
   Ile Asp Ala Asp Gly Asp Gly His Gly Trp Lys Pro Gly Asn Ala Pro
   980                 985                 990                 995
   GGA ATC GCT GGC TAC AAT AGC AAT GGT TGT GTA TAT TCA GAG TCA TTC        3981
   Gly Ile Ala Gly Tyr Asn Ser Asn Gly Cys Val Tyr Ser Glu Ser Phe
                   1000                1005                1010
   GGT CTT GGT GGT ATA GGA GTT CTT ACC CCT GAC AAC TAT CTG ATA ACA        4029
   Gly Leu Gly Gly Ile Gly Val Leu Thr Pro Asp Asn Tyr Leu Ile Thr
               1015                1020                1025
   CCG GCA TTG GAT TTG CCT AAC GGA GGT AAG TTG ACT TTC TGG GTA TGC        4077
   Pro Ala Leu Asp Leu Pro Asn Gly Gly Lys Leu Thr Phe Trp Val Cys
           1030                1035                1040
   GCA CAG GAT GCT AAT TAT GCA TCC GAG CAC TAT GCG GTG TAT GCA TCT        4125
   Ala Gln Asp Ala Asn Tyr Ala Ser Glu His Tyr Ala Val Tyr Ala Ser
       1045                1050                1055
   TCG ACC GGT AAC GAT GCA TCC AAC TTC ACG AAT GCT TTG TTG GAA GAG        4173
   Ser Thr Gly Asn Asp Ala Ser Asn Phe Thr Asn Ala Leu Leu Glu Glu
   1060                1065                1070                1075
   ACG ATT ACG GCA AAA GGT GTT CGC TCG CCG GAA GCT ATT CGT GGT CGT        4221
   Thr Ile Thr Ala Lys Gly Val Arg Ser Pro Glu Ala Ile Arg Gly Arg
                   1080                1085                1090
   ATA CAG GGT ACT TGG CGC CAG AAG ACG GTA GAC CTT CCC GCA GGT ACG        4269
   Ile Gln Gly Thr Trp Arg Gln Lys Thr Val Asp Leu Pro Ala Gly Thr
               1095                1100                1105
   AAA TAT GTT GCT TTC CGT CAC TTC CAA AGC ACG GAT ATG TTC TAC ATC      4317
   Lys Tyr Val Ala Phe Arg His Phe Gln Ser Thr Asp Met Phe Tyr Ile
           1110                1115                1120
   GAC CTT GAT GAG GTT GAG ATC AAG GCC AAC GGC AAG CGC GCA GAC TTC      4365
   Asp Leu Asp Glu Val Glu Ile Lys Ala Asn Gly Lys Arg Ala Asp Phe
       1125                1130                1135
   ACG GAA ACG TTC GAG TCT TCT ACT CAT GGA GAG GCA CCG GCG GAA TGG      4413
   Thr Glu Thr Phe Glu Ser Ser Thr His Gly Glu Ala Pro Ala Glu Trp
   1140                1145                1150                1155
   ACT ACT ATC GAT GCC GAT GGC GAT GGT CAG GGT TGG CTC TGT CTG TCT      4461
   Thr Thr Ile Asp Ala Asp Gly Asp Gly Gln Gly Trp Leu Cys Leu Ser
                   1160                1165                1170
   TCC GGA CAA TTG GAC TGG CTG ACA GCT CAT GGC GGC ACC AAC GTA GTA      4509
   Ser Gly Gln Leu Asp Trp Leu Thr Ala His Gly Gly Thr Asn Val Val
               1175                1180                1185
   GCC TCT TTC TCA TGG AAT GGA ATG GCT TTG AAT CCT GAT AAC TAT CTC      4557
   Ala Ser Phe Ser Trp Asn Gly Met Ala Leu Asn Pro Asp Asn Tyr Leu
           1190                1195                1200
   ATC TCA AAG GAT GTT ACA GGC GCA ACG AAG GTA AAG TAC TAC TAT GCA      4605
   Ile Ser Lys Asp Val Thr Gly Ala Thr Lys Val Lys Tyr Tyr Tyr Ala
       1205                1210                1215
   GTC AAC GAC GGT TTT CCC GGG GAT CAC TAT GCG GTG ATG ATC TCC AAG      4653
   Val Asn Asp Gly Phe Pro Gly Asp His Tyr Ala Val Met Ile Ser Lys
   1220                1225                1230                1235
   ACG GGC ACG AAC GCC GGA GAC TTC ACG GTT GTT TTC GAA GAA ACG CCT      4701
   Thr Gly Thr Asn Ala Gly Asp Phe Thr Val Val Phe Glu Glu Thr Pro
                   1240                1245                1250
   AAC GGA ATA AAT AAG GGC GGA GCA AGA TTC GGT CTT TCC ACG GAA GCC      4749
   Asn Gly Ile Asn Lys Gly Gly Ala Arg Phe Gly Leu Ser Thr Glu Ala
               1255                1260                1265
   AAT GGC GCC AAA CCT CAA AGT GTA TGG ATC GAG CGT ACG GTA GAT TTG      4797
   Asn Gly Ala Lys Pro Gln Ser Val Trp Ile Glu Arg Thr Val Asp Leu
           1270                1275                1280
   CCT GCG GGC ACG AAG TAT GTT GCT TTC CGT CAC TAC AAT TGC TCG GAT      4845
   Pro Ala Gly Thr Lys Tyr Val Ala Phe Arg His Tyr Asn Cys Ser Asp
       1285                1290                1295
   TTG AAC TAC ATT CTT TTG GAT GAT ATT CAG TTC ACC ATG GGT GGC AGC      4893
   Leu Asn Tyr Ile Leu Leu Asp Asp Ile Gln Phe Thr Met Gly Gly Ser
   1300                1305                1310                1315
   CCC ACC CCG ACC GAT TAT ACC TAC ACG GTG TAT CGT GAC GGT ACG AAG      4941
   Pro Thr Pro Thr Asp Tyr Thr Tyr Thr Val Tyr Arg Asp Gly Thr Lys
                   1320                1325                1330
   ATC AAG GAA GGT CTG ACC GAA ACG ACC TTC GAA GAA GAC GGC GTA GCT      4989
   Ile Lys Glu Gly Leu Thr Glu Thr Thr Phe Glu Glu Asp Gly Val Ala
               1335                1340                1345
   ACA GGC AAT CAT GAG TAT TGC GTG GAA GTG AAG TAC ACA GCC GGC GTA      5037
   Thr Gly Asn His Glu Tyr Cys Val Glu Val Lys Tyr Thr Ala Gly Val
           1350                1355                1360
   TCT CCG AAA GAG TGC GTA AAC GTA ACT ATT AAT CCG ACT CAG TTC AAT      5085
   Ser Pro Lys Glu Cys Val Asn Val Thr Ile Asn Pro Thr Gln Phe Asn
       1365                1370                1375
   CCT GTA AAG AAC CTG AAG GCA CAA CCG GAT GGC GGC GAC GTG GTT CTC      5133
   Pro Val Lys Asn Leu Lys Ala Gln Pro Asp Gly Gly Asp Val Val Leu
   1380                1385                1390                1395
   AAG TGG GAA GCC CCG AGC GCA AAA AAG ACA GAA GGT TCT CGT GAA GTA      5181
   Lys Trp Glu Ala Pro Ser Ala Lys Lys Thr Glu Gly Ser Arg Glu Val
                   1400                1405                1410
   AAA CGG ATC GGA GAC GGT CTT TTC GTT ACG ATC GAA CCT GCA AAC GAT      5229
   Lys Arg Ile Gly Asp Gly Leu Phe Val Thr Ile Glu Pro Ala Asn Asp
               1415                1420                1425
   GTA CGT GCC AAC GAA GCC AAG GTT GTG CTC GCA GCA GAC AAC GTA TGG      5277
   Val Arg Ala Asn Glu Ala Lys Val Val Leu Ala Ala Asp Asn Val Trp
           1430                1435                1440
   GGA GAC AAT ACG GGT TAC CAG TTC TTG TTG GAT GCC GAT CAC AAT ACA      5325
   Gly Asp Asn Thr Gly Tyr Gln Phe Leu Leu Asp Ala Asp His Asn Thr
       1445                1450                1455
   TTC GGA AGT GTC ATT CCG GCA ACC GGT CCT CTC TTT ACC GGA ACA GCT      5373
   Phe Gly Ser Val Ile Pro Ala Thr Gly Pro Leu Phe Thr Gly Thr Ala
   1460                1465                1470                1475
   TCT TCC AAT CTT TAC AGT GCG AAC TTC GAG TAT TTG ATC CCG GCC AAT      5421
   Ser Ser Asn Leu Tyr Ser Ala Asn Phe Glu Tyr Leu Ile Pro Ala Asn
                   1480                1485                1490
   GCC GAT CCT GTT GTT ACT ACA CAG AAT ATT ATC GTT ACA GGA CAG GGT      5469
   Ala Asp Pro Val Val Thr Thr Gln Asn Ile Ile Val Thr Gly Gln Gly
               1495                1500                1505
   GAA GTT GTA ATC CCC GGT GGT GTT TAC GAC TAT TGC ATT ACG AAC CCG      5517
   Glu Val Val Ile Pro Gly Gly Val Tyr Asp Tyr Cys Ile Thr Asn Pro
           1510                1515                1520
   GAA CCT GCA TCC GGA AAG ATG TGG ATC GCA GGA GAT GGA GGC AAC CAG      5565
   Glu Pro Ala Ser Gly Lys Met Trp Ile Ala Gly Asp Gly Gly Asn Gln
       1525                1530                1535
   CCT GCA CGT TAT GAC GAT TTC ACA TTC GAA GCA GGC AAG AAG TAC ACC      5613
   Pro Ala Arg Tyr Asp Asp Phe Thr Phe Glu Ala Gly Lys Lys Tyr Thr
   1540                1545                1550                1555
   TTC ACG ATG CGT CGC GCC GGA ATG GGA GAT GGA ACT GAT ATG GAA GTC      5661
   Phe Thr Met Arg Arg Ala Gly Met Gly Asp Gly Thr Asp Met Glu Val
                   1560                1565                1570
   GAA GAC GAT TCA CCT GCA AGC TAT ACC TAT ACA GTC TAT CGT GAC GGC      5709
   Glu Asp Asp Ser Pro Ala Ser Tyr Thr Tyr Thr Val Tyr Arg Asp Gly
               1575                1580                1585
   ACG AAG ATC AAG GAA GGT CTG ACC GAA ACG ACC TAC CGC GAT GCA GGA      5757
   Thr Lys Ile Lys Glu Gly Leu Thr Glu Thr Thr Tyr Arg Asp Ala Gly
           1590                1595                1600
   ATG AGT GCA CAA TCT CAT GAG TAT TGC GTA GAG GTT AAG TAC GCA GCC      5805
   Met Ser Ala Gln Ser His Glu Tyr Cys Val Glu Val Lys Tyr Ala Ala
       1605                1610                1615
   GGC GTA TCT CCG AAG GTT TGT GTG GAT TAT ATT CCT GAC GGA GTG GCA      5853
   Gly Val Ser Pro Lys Val Cys Val Asp Tyr Ile Pro Asp Gly Val Ala
   1620                1625                1630                1635
   GAC GTA ACG GCT CAG AAG CCT TAC ACC CTG ACA GTT GTT GGA AAG ACG      5901
   Asp Val Thr Ala Gln Lys Pro Tyr Thr Leu Thr Val Val Gly Lys Thr
                   1640                1645                1650
   ATC ACG GTA ACT TGC CAA GGC GAA GCT ATG ATC TAC GAC ATG AAC GGT       5949
   Ile Thr Val Thr Cys Gln Gly Glu Ala Met Ile Tyr Asp Met Asn Gly
               1655                1660                1665
   CGT CGT CTG GCA GCC GGT CGC AAC ACA GTT GTT TAC ACG GCT CAG GGC       5997
   Arg Arg Leu Ala Ala Gly Arg Asn Thr Val Val Tyr Thr Ala Gln Gly
           1670                1675                1680
   GGC TAC TAT GCA GTC ATG GTT GTC GTT GAC GGC AAG TCT TAC GTA GAG       6045
   Gly Tyr Tyr Ala Val Met Val Val Val Asp Gly Lys Ser Tyr Val Glu
       1685                1690                1695
   AAA CTC GCT GTA AAG TAATTCTGTC TTGGACTCGG AGACTTTGTG CAGACACTTT       6100
   Lys Leu Ala Val Lys
   1700                170
   TAATATAGGT CTGTAATTGT CTCAGAGTAT GAATCGATCG CCCGACCTCC TTTTAAGGAA     6160
   GTCTGGGCGA CTTCGTTTTT ATGCCTATTA TTCTAATATA CTTCTGAAAC AATTTGTTCC     6220
   AAAAAGTTGC ATGAAAAGAT TATCTTACTA TCTTTGCACT GCAAAAGGGG AGTTTCCTAA     6280
   GGTTTTCCCC GGAGTAGTAC GGTAATAACG GTGTGGTAGT TCAGCTGGTT AGAATACCTG     6340
   CCTGTCACGC AGGGGGTCGC GGGTTCGAGT CCCGTCCATA CCGCTAAATA GCTGAAAGAT     6400
   AGGCTATAGG TCATCTGAAG CAATTTTAGA AACGAATCCA AAAGCGTCTT AATTCCAACG     6460
   AATTAAGGCG CTTTTTCTTT GTCGCCACCC CACACGTCGG ATGAGGTTCG GAATAGGCGT     6520
   ATATTCCGTA AATATGCCTC CGGTGGTTCC ATTTTGGTTA CAAAAAACAA AGGGGCTGAA     6580
   AATTGTAACC ACAGACGACG TTAAGACGAT GTTTAGACGA TTGACAAATT ACTCTGTTTC     6640
   AAAATCATAT GTCGAACTTT GTAGCCGTAT GGTTACACTA ATTTTGGAGC AAAATGAAGA     6700
   GTCAATTTCG TTCAGTTTTT TACTTGCGCA GCAATTACAT CAACAAAGAA GGTAAAACTC     6760
   CTGTCCTTAT TCGTATTTAT CTGAATAAGG AACGCCTGTC GTTGGGTTCG ACAGGGCTGG     6820
   CTGTTAATCC CATACAATGG GATTCAGAAA AAGAGAAAGT CAAAGGACAT AGTGCAGAAG     6880
   CACTTGAAGT CAATCGAAAG ATCGAAGAAA TCAGGGCTGA TATTCTGACC ATTTACAAAC     6940
   GTTTGGAAGT AACAGTAGAT GATTTGACGC CGGAGAGGAT CAAATCGGAA TACTGCGGAC     7000
   AGACGGATAC ATTAAACAGT ATAGTGGAAC TTTTCGATAA ACATAACGAG GATGTCCGGG     7060
   CCCAGGTGGG AATCAATAAA ACGGCTGCCA CTTTACAAAA ATACGAAAAC AGCAAACGGC     7120
   ATTTTACCCG ATTCCTCAAA GCGAAGTACA ACAGAACGGA TCTCAAATTC TCAGAGCTTA     7180
   CCCCGTTGGT CATTCATAAC TTTGAGATAT ATCTGCTGAC TGTAGCCCAT TGTTGCCCGA     7240
   ATACGGCAAC CAAAATCTTG AAGCTT                                          7266
(2) INFORMATION FOR SEQ ID NO:11:
   (i) SEQUENCE CHARACTERISTICS:
      (A) LENGTH: 1704 amino acids
      (B) TYPE: amino acid
      (D) TOPOLOGY: linear
   (ii) MOLECULE TYPE: protein
   (xi) SEQUENCE DESCRIPTION: SEQ ID NO:11:
   Met Lys Asn Leu Asn Lys Phe Val Ser Ile Ala Leu Cys Ser Ser Leu
    1               5                  10                  15
   Leu Gly Gly Met Ala Phe Ala Gln Gln Thr Glu Leu Gly Arg Asn Pro
               20                  25                  30
   Asn Val Arg Leu Leu Glu Ser Thr Gln Gln Ser Val Thr Lys Val Gln
           35                  40                  45
   Phe Arg Met Asp Asn Leu Lys Phe Thr Glu Val Gln Thr Pro Lys Gly
       50                  55                  60
   Ile Gly Gln Val Pro Thr Tyr Thr Glu Gly Val Asn Leu Ser Glu Lys
   65                  70                  75                  80
   Gly Met Pro Thr Leu Pro Ile Leu Ser Arg Ser Leu Ala Val Ser Asp
                   85                  90                  95
   Thr Arg Glu Met Lys Val Glu Val Val Ser Ser Lys Phe Ile Glu Lys
               100                 105                 110
   Lys Asn Val Leu Ile Ala Pro Ser Lys Gly Met Ile Met Arg Asn Glu
           115                 120                 125
   Asp Pro Lys Lys Ile Pro Tyr Val Tyr Gly Lys Ser Tyr Ser Gln Asn
       130                 135                 140
   Lys Phe Phe Pro Gly Glu Ile Ala Thr Leu Asp Asp Pro Phe Ile Leu
   145                 150                 155                 160
   Arg Asp Val Arg Gly Gln Val Val Asn Phe Ala Pro Leu Gln Tyr Asn
                   165                 170                 175
   Pro Val Thr Lys Thr Leu Arg Ile Tyr Thr Glu Ile Thr Val Ala Val
               180                 185                 190
   Ser Glu Thr Ser Glu Gln Gly Lys Asn Ile Leu Asn Lys Lys Gly Thr
           195                 200                 205
   Phe Ala Gly Phe Glu Asp Thr Tyr Lys Arg Met Phe Met Asn Tyr Glu
       210                 215                 220
   Pro Gly Arg Tyr Thr Pro Val Glu Glu Lys Gln Asn Gly Arg Met Ile
   225                 230                 235                 240
   Val Ile Val Ala Lys Lys Tyr Glu Gly Asp Ile Lys Asp Phe Val Asp
                   245                 250                 255
   Trp Lys Asn Gln Arg Gly Leu Arg Thr Glu Val Lys Val Ala Glu Asp
               260                 265                 270
   Ile Ala Ser Pro Val Thr Ala Asn Ala Ile Gln Gln Phe Val Lys Gln
           275                 280                 285
   Glu Tyr Glu Lys Glu Gly Asn Asp Leu Thr Tyr Val Leu Leu Val Gly
       290                 295                 300
   Asp His Lys Asp Ile Pro Ala Lys Ile Thr Pro Gly Ile Lys Ser Asp
   305                 310                 315                 320
   Gln Val Tyr Gly Gln Ile Val Gly Asn Asp His Tyr Asn Glu Val Phe
                   325                 330                 335
   Ile Gly Arg Phe Ser Cys Glu Ser Lys Glu Asp Leu Lys Thr Gln Ile
               340                 345                 350
   Asp Arg Thr Ile His Tyr Glu Arg Asn Ile Thr Thr Glu Asp Lys Trp
           355                 360                 365
   Leu Gly Gln Ala Leu Cys Ile Ala Ser Ala Glu Gly Gly Pro Ser Ala
       370                 375                 380
   Asp Asn Gly Glu Ser Asp Ile Gln His Glu Asn Val Ile Ala Asn Leu
   385                 390                 395                 400
   Leu Thr Gln Tyr Gly Tyr Thr Lys Ile Ile Lys Cys Tyr Asp Pro Gly
                   405                 410                 415
   Val Thr Pro Lys Asn Ile Ile Asp Ala Phe Asn Gly Gly Ile Ser Leu
               420                 425                 430
   Val Asn Tyr Thr Gly His Gly Ser Glu Thr Ala Trp Gly Thr Ser His
           435                 440                 445
   Phe Gly Thr Thr His Val Lys Gln Leu Thr Asn Ser Asn Gln Leu Pro
       450                 455                 460
   Phe Ile Phe Asp Val Ala Cys Val Asn Gly Asp Phe Leu Phe Ser Met
   465                 470                 475                 480
   Pro Cys Phe Ala Glu Ala Leu Met Arg Ala Gln Lys Asp Gly Lys Pro
                   485                 490                 495
   Thr Gly Thr Val Ala Ile Ile Ala Ser Thr Ile Asn Gln Ser Trp Ala
               500                 505                 510
   Ser Pro Met Arg Gly Gln Asp Glu Met Asn Glu Ile Leu Cys Glu Lys
           515                 520                 525
   His Pro Asn Asn Ile Lys Arg Thr Phe Gly Gly Val Thr Met Asn Gly
       530                 535                 540
   Met Phe Ala Met Val Glu Lys Tyr Lys Lys Asp Gly Glu Lys Met Leu
   545                 550                 555                 560
   Asp Thr Trp Thr Val Phe Gly Asp Pro Ser Leu Leu Val Arg Thr Leu
                   565                 570                 575
   Val Pro Thr Lys Met Gln Val Thr Ala Pro Ala Gln Ile Asn Leu Thr
               580                 585                 590
   Asp Ala Ser Val Asn Val Ser Cys Asp Tyr Asn Gly Ala Ile Ala Thr
           595                 600                 605
   Ile Ser Ala Asn Gly Lys Met Phe Gly Ser Ala Val Val Glu Asn Gly
       610                 615                 620
   Thr Ala Thr Ile Asn Leu Thr Gly Leu Thr Asn Glu Ser Thr Leu Thr
   625                 630                 635                 640
   Leu Thr Val Val Gly Tyr Asn Lys Glu Thr Val Ile Lys Thr Ile Asn
                   645                 650                 655
   Thr Asn Gly Glu Pro Asn Pro Tyr Gln Pro Val Ser Asn Leu Thr Ala
               660                 665                 670
   Thr Thr Gln Gly Gln Lys Val Thr Leu Lys Trp Asp Ala Pro Ser Thr
           675                 680                 685
   Lys Thr Asn Ala Thr Thr Asn Thr Ala Arg Ser Val Asp Gly Ile Arg
       690                 695                 700
   Glu Leu Val Leu Leu Ser Val Ser Asp Ala Pro Glu Leu Leu Arg Ser
   705                 710                 715                 720
   Gly Gln Ala Glu Ile Val Leu Glu Ala His Asp Val Trp Asn Asp Gly
                   725                 730                 735
   Ser Gly Tyr Gln Ile Leu Leu Asp Ala Asp His Asp Gln Tyr Gly Gln
               740                 745                 750
   Val Ile Pro Ser Asp Thr His Thr Leu Trp Pro Asn Cys Ser Val Pro
           755                 760                 765
   Ala Asn Leu Phe Ala Pro Phe Glu Tyr Thr Val Pro Glu Asn Ala Asp
       770                 775                 780
   Pro Ser Cys Ser Pro Thr Asn Met Ile Met Asp Gly Thr Ala Ser Val
   785                 790                 795                 800
   Asn Ile Pro Ala Gly Thr Tyr Asp Phe Ala Ile Ala Ala Pro Gln Ala
                   805                 810                 815
   Asn Ala Lys Ile Trp Ile Ala Gly Gln Gly Pro Thr Lys Glu Asp Asp
               820                 825                 830
   Tyr Val Phe Glu Ala Gly Lys Lys Tyr His Phe Leu Met Lys Lys Met
           835                 840                 845
   Gly Ser Gly Asp Gly Thr Glu Leu Thr Ile Ser Glu Gly Gly Gly Ser
       850                 855                 860
   Asp Tyr Thr Tyr Thr Val Tyr Arg Asp Gly Thr Lys Ile Lys Glu Gly
   865                 870                 875                 880
   Leu Thr Ala Thr Thr Phe Glu Glu Asp Gly Val Ala Thr Gly Asn His
                   885                 890                 895
   Glu Tyr Cys Val Glu Val Lys Tyr Thr Ala Gly Val Ser Pro Lys Val
               900                 905                 910
   Cys Lys Asp Val Thr Val Glu Gly Ser Asn Glu Phe Ala Pro Val Gln
           915                 920                 925
   Asn Leu Thr Gly Ser Ala Val Gly Gln Lys Val Thr Leu Lys Trp Asp
       930                 935                 940
   Ala Pro Asn Gly Thr Pro Asn Pro Asn Pro Asn Pro Asn Pro Asn Pro
   945                 950                 955                 960
   Gly Thr Thr Thr Leu Ser Glu Ser Phe Glu Asn Gly Ile Pro Ala Ser
                   965                 970                 975
   Trp Lys Thr Ile Asp Ala Asp Gly Asp Gly His Gly Trp Lys Pro Gly
               980                 985                 990
   Asn Ala Pro Gly Ile Ala Gly Tyr Asn Ser Asn Gly Cys Val Tyr Ser
           995                 1000                1005
   Glu Ser Phe Gly Leu Gly Gly Ile Gly Val Leu Thr Pro Asp Asn Tyr
       1010                1015                1020
   Leu Ile Thr Pro Ala Leu Asp Leu Pro Asn Gly Gly Lys Leu Thr Phe
   1025                1030                1035                1040
   Trp Val Cys Ala Gln Asp Ala Asn Tyr Ala Ser Glu His Tyr Ala Val
                   1045                1050                1055
   Tyr Ala Ser Ser Thr Gly Asn Asp Ala Ser Asn Phe Thr Asn Ala Leu
               1060                1065                1070
   Leu Glu Glu Thr Ile Thr Ala Lys Gly Val Arg Ser Pro Glu Ala Ile
           1075                1080                1085
   Arg Gly Arg Ile Gln Gly Thr Trp Arg Gln Lys Thr Val Asp Leu Pro
       1090                1095                1100
   Ala Gly Thr Lys Tyr Val Ala Phe Arg His Phe Gln Ser Thr Asp Met
   1105                1110                1115                1120
   Phe Tyr Ile Asp Leu Asp Glu Val Glu Ile Lys Ala Asn Gly Lys Arg
                   1125                1130                1135
   Ala Asp Phe Thr Glu Thr Phe Glu Ser Ser Thr His Gly Glu Ala Pro
               1140                1145                1150
   Ala Glu Trp Thr Thr Ile Asp Ala Asp Gly Asp Gly Gln Gly Trp Leu
           1155                1160                1165
   Cys Leu Ser Ser Gly Gln Leu Asp Trp Leu Thr Ala His Gly Gly Thr
       1170                1175                1180
   Asn Val Val Ala Ser Phe Ser Trp Asn Gly Met Ala Leu Asn Pro Asp
   1185                1190                1195                1200
   Asn Tyr Leu Ile Ser Lys Asp Val Thr Gly Ala Thr Lys Val Lys Tyr
                   1205                1210                1215
   Tyr Tyr Ala Val Asn Asp Gly Phe Pro Gly Asp His Tyr Ala Val Met
               1220                1225                1230
   Ile Ser Lys Thr Gly Thr Asn Ala Gly Asp Phe Thr Val Val Phe Glu
           1235                1240                1245
   Glu Thr Pro Asn Gly Ile Asn Lys Gly Gly Ala Arg Phe Gly Leu Ser
       1250                1255                1260
   Thr Glu Ala Asn Gly Ala Lys Pro Gln Ser Val Trp Ile Glu Arg Thr
   1265                1270                1275                1280
   Val Asp Leu Pro Ala Gly Thr Lys Tyr Val Ala Phe Arg His Tyr Asn
                   1285                1290                1295
   Cys Ser Asp Leu Asn Tyr Ile Leu Leu Asp Asp Ile Gln Phe Thr Met
               1300                1305                1310
   Gly Gly Ser Pro Thr Pro Thr Asp Tyr Thr Tyr Thr Val Tyr Arg Asp
           1315                1320                1325
   Gly Thr Lys Ile Lys Glu Gly Leu Thr Glu Thr Thr Phe Glu Glu Asp
       1330                1335                1340
   Gly Val Ala Thr Gly Asn His Glu Tyr Cys Val Glu Val Lys Tyr Thr
   1345                1350                1355                1360
   Ala Gly Val Ser Pro Lys Glu Cys Val Asn Val Thr Ile Asn Pro Thr
                   1365                1370                1375
   Gln Phe Asn Pro Val Lys Asn Leu Lys Ala Gln Pro Asp Gly Gly Asp
               1380                1385                1390
   Val Val Leu Lys Trp Glu Ala Pro Ser Ala Lys Lys Thr Glu Gly Ser
           1395                1400                1405
   Arg Glu Val Lys Arg Ile Gly Asp Gly Leu Phe Val Thr Ile Glu Pro
       1410                1415                1420
   Ala Asn Asp Val Arg Ala Asn Glu Ala Lys Val Val Leu Ala Ala Asp
   1425                1430                1435                1440
   Asn Val Trp Gly Asp Asn Thr Gly Tyr Gln Phe Leu Leu Asp Ala Asp
                   1445                1450                1455
   His Asn Thr Phe Gly Ser Val Ile Pro Ala Thr Gly Pro Leu Phe Thr
               1460                1465                1470
   Gly Thr Ala Ser Ser Asn Leu Tyr Ser Ala Asn Phe Glu Tyr Leu Ile
           1475                1480                1485
   Pro Ala Asn Ala Asp Pro Val Val Thr Thr Gln Asn Ile Ile Val Thr
       1490                1495                1500
   Gly Gln Gly Glu Val Val Ile Pro Gly Gly Val Tyr Asp Tyr Cys Ile
   1505                1510                1515                1520
   Thr Asn Pro Glu Pro Ala Ser Gly Lys Met Trp Ile Ala Gly Asp Gly
                   1525                1530                1535
   Gly Asn Gln Pro Ala Arg Tyr Asp Asp Phe Thr Phe Glu Ala Gly Lys
               1540                1545                1550
   Lys Tyr Thr Phe Thr Met Arg Arg Ala Gly Met Gly Asp Gly Thr Asp
           1555                1560                1565
   Met Glu Val Glu Asp Asp Ser Pro Ala Ser Tyr Thr Tyr Thr Val Tyr
       1570                1575                1580
   Arg Asp Gly Thr Lys Ile Lys Glu Gly Leu Thr Glu Thr Thr Tyr Arg
   1585                1590                1595                1600
   Asp Ala Gly Met Ser Ala Gln Ser His Glu Tyr Cys Val Glu Val Lys
                   1605                1610                1615
   Tyr Ala Ala Gly Val Ser Pro Lys Val Cys Val Asp Tyr Ile Pro Asp
               1620                1625                1630
   Gly Val Ala Asp Val Thr Ala Gln Lys Pro Tyr Thr Leu Thr Val Val
           1635                1640                1645
   Gly Lys Thr Ile Thr Val Thr Cys Gln Gly Glu Ala Met Ile Tyr Asp
       1650                1655                1660
   Met Asn Gly Arg Arg Leu Ala Ala Gly Arg Asn Thr Val Val Tyr Thr
   1665                1670                1675                1680
   Ala Gln Gly Gly Tyr Tyr Ala Val Met Val Val Val Asp Gly Lys Ser
                   1685                1690                1695
   Tyr Val Glu Lys Leu Ala Val Lys
               1700